(19) Europäisches Patentamt
European Patent Office
Office européen des brevets

(11) EP 4 008 730 A1

(12) # EUROPEAN PATENT APPLICATION

published in accordance with Art. 153(4) EPC

(43) Date of publication:
**08.06.2022 Bulletin 2022/23**

(21) Application number: **20850855.6**

(22) Date of filing: **31.07.2020**

(51) International Patent Classification (IPC):
***C07K 16/46*** (2006.01) ***C07K 16/28*** (2006.01)
***C12N 15/13*** (2006.01) ***C12N 15/62*** (2006.01)
***C12N 15/63*** (2006.01) ***A61K 39/395*** (2006.01)
***G01N 33/577*** (2006.01) ***G01N 33/68*** (2006.01)
***A61P 7/06*** (2006.01) ***A61P 35/00*** (2006.01)
***A61P 35/02*** (2006.01)

(52) Cooperative Patent Classification (CPC):
**A61K 39/395; A61K 45/00; A61P 7/06; A61P 35/00; A61P 35/02; C07K 16/28; C07K 16/46; C12N 15/62; C12N 15/63; G01N 33/574; G01N 33/577; G01N 33/68**

(86) International application number:
**PCT/CN2020/106309**

(87) International publication number:
**WO 2021/023117 (11.02.2021 Gazette 2021/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.08.2019 CN 201910711122**
**02.12.2019 CN 201911224135**

(71) Applicant: **Akeso Pharmaceuticals, Inc.**
**Guangzhou, Guangdong 510530 (CN)**

(72) Inventors:
- **XIA, Yu**
  **Zhongshan, Guangdong 528437 (CN)**
- **WANG, Zhongmin**
  **Zhongshan, Guangdong 528437 (CN)**
- **ZHANG, Peng**
  **Zhongshan, Guangdong 528437 (CN)**
- **LI, Baiyong**
  **Zhongshan, Guangdong 528437 (CN)**

(74) Representative: **Cooley (UK) LLP**
**22 Bishopsgate**
**London EC2N 4BQ (GB)**

(54) **ANTI-CTLA4-ANTI-PD-1 BISPECIFIC ANTIBODY AND USES THEREOF**

(57) The present invention relates to the field of tumor treatment and molecular immunology, and particularly, to an anti-CTLA4/anti-PD-1 bispecific antibody and use thereof. Specifically, the anti-CTLA4/anti-PD-1 bifunctional antibody comprises a first protein functional region targeting PD-1 and a second protein functional region targeting CTLA4, wherein, according to the EU numbering system, the heavy chain constant region of the immunoglobulin comprised in the bispecific antibody has mutations at any 2 or 3 of positions 234, 235 and 237, and the affinity constant of the bispecific antibody to FcyRIIIa and/or C1q is reduced after the mutation as compared to that before the mutation. The bifunctional antibody of the present invention can well and specifically bind to CTLA4 and PD-1, specifically relieve immunosuppression of CTLA4 and PD-1 in an organism, and activate T lymphocytes, thus having good application prospect.

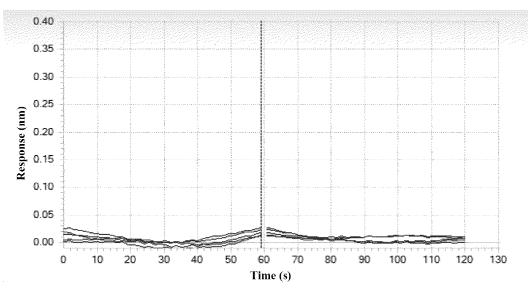


FIG. 13

EP 4 008 730 A1

## Description

TECHNICAL FIELD

The present invention relates to the field of tumor treatment and molecular immunology, and particularly, to an anti-CTLA4/anti-PD-1 bispecific antibody and use thereof. More particularly, the present invention relates to mutant anti-CTLA4/anti-PD-1 bispecific antibodies.

BACKGROUND

The transmembrane receptor PD-1 (programmed cell death protein 1) is a member of the CD28 family, and is expressed in activated T cells, B cells and myeloid cells. Both ligands of PD-1, PDL1 (programmed cell death 1 ligand 1, or PDL-1) and PDL2 (programmed cell death 1 ligand 2, or PDL-2), are members of the B7 superfamily. PDL1 is expressed in a variety of cells including T cells, B cells, endothelial cells and epithelial cells, and PDL2 is expressed only in antigen presenting cells such as dendritic cells and macrophages.

The PD-1/PDL1 signaling pathway plays an important role in regulating immune tolerance, microbial infection and tumor immune escape. PD-1 is mainly expressed in immune cells such as T cells, and the ligand PDL1 of PD-1 is highly expressed in a plurality of human tumor tissues. Blocking the PD-1/PDL1 signaling pathway may activate inhibited T cells, which thus attack cancer cells. Blocking the PD-1/PDL1 signaling can promote the proliferation of tumor antigen-specific T cells, activate tumor cell killing process and further inhibit local tumor growth (Julie R et al., 2012, N Engl J Med., 366:2455-2465). In addition, tumors with high PDL1 expression are associated with cancers that are difficult to detect (Hamanishi et al., 2007, Proc. Natl. Acad. Sci. USA, 104:3360-5). An effective method is administering an anti-PD-1 antibody to modulate the expression of PD-1. Due to the broad anti-tumor prospects and surprising efficacy of PD-1 antibodies, it is widely accepted in the industry that antibodies targeting the PD-1 pathway will bring about breakthroughs in the treatment of various tumors, for example, non-small cell lung cancer, renal cell carcinoma, ovarian cancer, melanoma (Homet M. B., Parisi G., et al., 2015, Semin Oncol., 42(3):466-473), leukemia and anemia (Held SA, Heine A, et al., 2013, Curr Cancer Drug Targets., 13(7):768-74).

Cytotoxic T lymphocyte-associated antigen 4 (CTLA4) and CD28 molecules are very similar in aspects of gene structure, chromosome location, sequence homology and gene expression. Both molecules are receptors of co-stimulatory molecule B7, and mainly expressed on the surface of activated T cells. Binding of CTLA4 to B7 inhibits the activation of mouse and human T cells, and plays a negative regulatory role in T cell activation.

CTLA4 antibodies (or anti-CTLA4 monoclonal antibodies) or CTLA4 ligands can prevent CTLA4 from binding to its natural ligands, thereby blocking the transmission of negative regulatory signals by CTLA4 to T cells and enhancing the reactivity of T cells to various antigens. In this respect, *in vivo* and *in vitro* studies are essentially consistent. Currently, there are CTLA4 monoclonal antibodies in clinical trials or approved for treating prostate cancer, bladder cancer, colorectal cancer, gastrointestinal cancer, liver cancer, malignant melanoma, etc. (Grosso JF., Jure-Kunkel MN., 2013, Cancer Immun., 13:5*)*.

Interleukin 2 (IL-2) is produced by T cells. It is a growth factor that regulates T cell subgroups, and an important factor in regulating immune responses. It promotes the proliferation of activated B cells, and participates in antibody responses, hematopoiesis and tumor surveillance. Recombinant human IL-2 has been approved by the U.S. FDA for treating malignancies, including melanoma, renal tumor, etc., while a clinical study is currently ongoing for treating chronic viral infections (Chavez, A.R., et al., 2009, Ann. N. Y. Acad. Sci., 1182:p.14-27). CTLA4 and CTLA4 antibodies are important influencing factors of T cell functions and interfere with the immune microenvironment in the body. *In-vitro* and *in-vivo* studies demonstrated that CTLA4 antibodies can specifically relieve the immunosuppression of CTLA4, activate T cells, and induce IL-2 generation, and is promising in wide applications in gene therapy against diseases such as tumors and parasite infections.

CTLA4 antibodies can produce specific therapeutic effect on diseases and remarkable efficacy, and may be used for supplementing traditional medicines and for exploring new means of gene therapy.

Bispecific antibodies, also known as bifunctional antibodies, are specific drugs that target two different antigens simultaneously, and can be produced by immunomagnetic separation. Alternatively, they can be obtained by genetic engineering. The genetic engineering has flexibility in aspects of binding site optimization, synthetic form, yield and the like, thus having certain advantages. Currently, over 45 forms have been demonstrated (Dafne Muller, Kontermann R E., 2010, BioDrugs, 24(2):89-98). A number of developed bispecific antibodies are in the form of IgG-scFv, i.e., the Morrison format (Coloma MJ, Morrison SL., 1997, Nat Biotechnol., 15:159-163), which has been demonstrated to be one of the ideal forms for the bispecific antibodies because of its similarity to the natually existing IgG forms and advantages in antibody engineering, expression and purification (Miller BR, Demarest SJ, et al., 2010, Protein Eng Des Sel, 23:549-57; Fitzgerald J, Lugovskoy A., 2011, MAbs, 3:299-309).

ADCC (antibody-dependent cell-mediated cytotoxicity) refers to killing of a target cell by a killer cell (NK cell,

macrophage, etc.) that is mediated by binding of the Fab fragment of an antibody to an epitope of a virus-infected cell or a tumor cell and binding of the Fc fragment of the antibody to an Fc receptor (FcR) on the surface of the killer cell.

CDC (complement dependent cytotoxicity) refers to that the specific binding of an antibody to a corresponding antigen on a cell membrane surface forms a complex and activates the complement system, which further forms an MAC on the surface of the target cell resulting in subsequent target cell lysis. Complements may cause lysis of various bacteria and other pathogenic organisms, and are an important defense mechanism against pathogenic organism infections.

Fc receptors belong to an immunoglobulin family that are expressed on the surface of specific immune cells to recognize antibody Fc regions and mediate immune responses. After the Fab region recognizes an antigen, the Fc region of the antibody binds to the Fc receptor on the immune cell (e.g., a killer cell) to initiate the response function of the immune cell, such as phagocytosis and ADCC.

According to the type of antibody recognized by the Fc receptor and the type of expression cells, Fc receptors are mainly classified into three types, FcyR, FcaR and FcεR. FcyR can be further classified into four subtypes, FcyRI (CD64), FcyRII (CD32), FcyRIII (CD16) and FcRn (neonatal Fc receptor). Among these, FcyRI, FcyRII and FcyRIII are closely associated with ADCC effect. FcyRIII is the most predominant molecule mediating ADCC, with two highly homologous subtypes, FcyRIIIa and FcyRIIIb, in different cell types. In FcγRIIIa populations, two subtypes distinguished by sites of single nucleotide polymorphism (SNP), FcγRIIIa_V158 with high affinity and FcγRIIIa_F158 with low affinity, are present. FcyRI has higher affinity for the Fc region of IgG and participates in ADCC process; FcyRII comprises three subtypes, FcyRIIa, FcyRIIb and FcyRIIc (also referred to as CD32a, CD32b and CD32c, respectively), among which FcyRIIa has ADCC activity; for FcyRIIa, two subtypes, FcγRIIa_H131 and FcγRIIa_R131, are present in humans due to single nucleotide mutation; FcyRIIb is an inhibitory receptor, and is a typical inhibitory FcyR that inhibits nearby ITAM pathways. For example, after the binding of the immune complex to BCR, the Fc fragment binds to FcyRIIb on the same cell, negatively regulating B cell activation and decreasing secretion of antibodies and cytokines (Hogarth PM, Pietersz GA., 2012, NATURE REVIEWS DRUG DISCOVERY, 11(4):311-331).

The IgG family comprises four members, IgG1, IgG2, IgG3 and IgG4, which differ in amino acids in the fragment crystallizable (Fc) region of the heavy chain constant region, resulting in their varying affinities for FcyRs. IgG1 is the most abundant subtype in humans and is also the most common subtype used in monoclonal antibody medication. IgG1 is capable of binding various FcyRs and is able to induce ADCC and CDC effects. IgG2 has the lowest affinity for FcyRs, but is still able to induce monocyte-mediated ADCC by binding to FcyRIIa. IgG3 features the highest binding capacity to FcyRs, and can induce ADCC and a greater CDC effect than IgG1. IgG4 molecules demonstrate a weak binding to FcyRs other than FcyRI, having a lower probability of causing CDC and NK cell-mediated ADCC. However, antibodies of the IgG4 subtype can mediate ADCP effects through binding to FcyRI, and the ADCP effects, present in antibody therapies targeting immune cells, may cause damage to immune cells, posing pharmacological adverse effects. At present, there is still a need for developing a novel anti-CTLA4/anti-PD-1 bispecific antibody to reduce or eliminate the damage caused by antibody-mediated ADCC, ADCP and/or CDC activity on immune cells to which the anti-CTLA4/anti-PD-1 bispecific antibody binds, and to improve the efficacy of the antibody therapy.

Chemotherapies are currently mainly classified into the following nine classes (He Jie, et al., Clinical Oncology, Beijing, People's Medical Publishing House, 2016:230-237). The first class are drugs that directly bind to DNA and prevent DNA replication, including various alkylating agents, mitomycin, bleomycin, dacarbazine, platinum-based drugs (e.g., cisplatin and carboplatin), camptothecins, and derivatives thereof. The second class are drugs for preventing nucleic acid biosynthesis, which mainly affect the enzyme system of tumor cells and block the synthesis of precursors of DNA and RNA, thereby inhibiting the formation of DNA or RNA, including methotrexate, fluorouracil, 6-mercaptopurine, hydroxyurea and cytarabine; such drugs mainly act on cells in S phase, and are antimetabolite chemotherapeutic drugs and cell cycle-specific anticancer drugs. The third class are chemotherapeutic drugs which affect transcription through the pharmacological mechanism that the drugs are inserted into the DNA double helix to form non-covalent binding with the DNA double helix, interfering with the transcription of genetic information on DNA to the DNA-dependent mRNA and causing compromised template function and hindered transcription. The fourth class are those affecting tubulin and mitosis, including vinca alkaloids, podophyllotoxins and taxanes. The fifth class are drugs affecting the function of ribosomes and blocking protein synthesis; representatives of such drugs are harringtonines, which inhibit the initiation of protein synthesis, decompose the ribosome and release new peptide chain, but do not block the binding of mRNA and tRNA to ribosomes; such drugs cause the reduction of nuclear DNA and cytoplasmic RNA and depolymerization of polysomes, and inhibit mitosis. The sixth class are drugs that affect the tumor cell membrane such as concanavalin (Con-A) and phytohemagglutinin (PHA); they can bind to glycoprotein receptors on the cell membrane, thereby affecting DNA synthesis in tumor cells and preventing tumor cell from dividing. The seventh class are drugs that induce apoptosis, such as arsenic trioxide. The eighth class are hormones that treat tumors by regulating the endocrine system, including estrogens, antiestrogens, progestogens, androgens, antiandrogens, corticosteroids, and anticorticosteroids (including dichlorodiphenyldichloroethane and aminoglutethimide). The ninth class is anticancer targeted therapy, including monoclonal antibodies, epidermal growth factor signaling inhibitors (e.g., targeted drugs against receptor tyrosine kinase

pathway), ubiquitin-proteasome inhibitors, and angiogenesis inhibitors.

Anlotinib is a quinoline derivative tyrosine kinase inhibitor. As a multi-target tyrosine kinase inhibitor (TKI), it affects tumor angiogenesis and proliferation signal transduction. The major targets include: receptor tyrosine kinases vascular endothelial growth factor receptors (VEGFRs) 1 to 3, epidermal growth factor receptor (EGFR), fibroblast growth factor receptors (FGFRs) 1 to 4, platelet-derived growth factor receptors (PDGFRs) $\alpha$ and $\beta$, and stem cell factor receptors (SCFRs) 7, 8 and 9. A phase 2 trial showed that anlotinib improved progression-free survival with the potential benefit for overall survival (Han B, et al., Br J Cancer, 2018; 118(5):654-661). A multicenter, double-blind, randomized phase 3 clinical trial showed that anlotinib resulted in extended overall and progression-free survivals in Chinese patients. The finding suggested that anlotinib is well tolerated and is a potential three-line or further treatment for patients with advanced NSCLC (Han B, et al., JAMA Oncol., 2018 Nov.; 4(11):1569-1575).

Example 24 of Patent No. WO2008112407 discloses a quinoline-derived tyrosine kinase inhibitor 1-[[[4-(4-fluoro-2-methyl-1*H*-indol-5-yl)oxy-6-methoxyquinolin-7-yl]oxy]methyl] cyclopropylamine and a method for preparing the same. The structural formula of the quinoline-derived tyrosine kinase inhibitor is shown in formula I. Anlotinib hydrochloride is the hydrochloride salt of the compound of formula I.

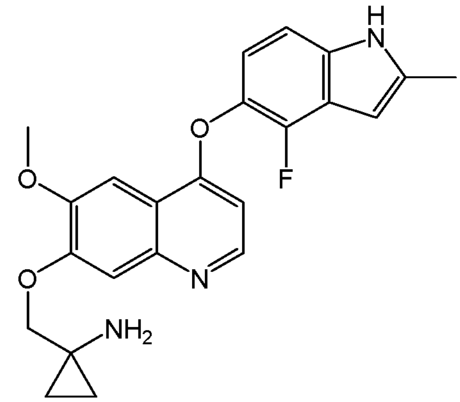

**formula I**

Lenvatinib, an oral multiple tyrosine kinase inhibitor developed by Eisai (Japan), is a multi-target receptor tyrosine kinase inhibitor that inhibits the kinase activity of VEGFR1 (FLT1), VEGFR2 (KDR) and VEGFR3 (FLT4). In addition to normal cellular function, lenvatinib also inhibits other receptor tyrosine kinases involved in pathogenic angiogenesis, tumor growth and cancer progression, including fibroblast growth factor (FGF) receptors FGFR1, FGFR2, FGFR3 and FGFR4, "rearranged during transfection" (RET) receptor, KIT and platelet-derived growth factor receptor $\alpha$ (PDGFR$\alpha$). Lenvatinib also exhibits antiproliferative activity in hepatocellular carcinoma cell lines, which is dependent on activated FGFR signaling and simultaneous inhibition of phosphorylation of FGF receptor substrate 2$\alpha$ (FRS2$\alpha$).

The structure of lenvatinib, 4-(3-chloro-4(cyclopropylaminocarbonyl)aminophenoxy)-7-methoxy-6-quinolinecarboxamide, is disclosed in Example 368 of U.S. Patent No. 7,612,208. U.S. Patent No. 7,253,286 discloses the mesylate salt form of lenvatinib (i.e., lenvatinib mesylate), named 4-[3-chloro-4-(cyclopropylureido)phenoxy]-7-methoxyquinoline-6-carboxamide mesylate, the chemical structure of which is provided below (formula II):

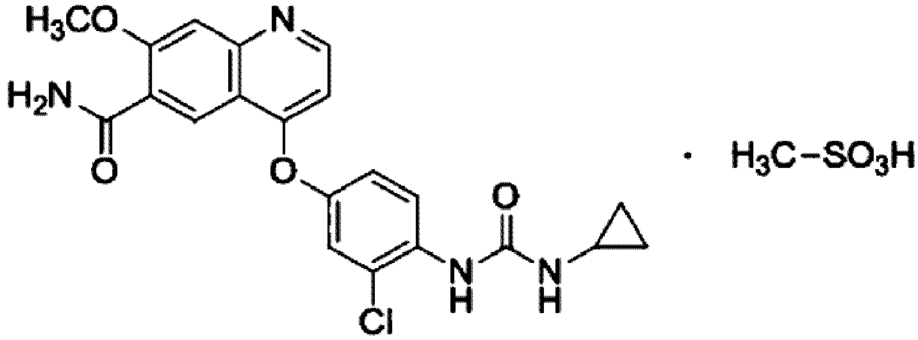

**formula II**

However, for a variety of tumors, the disease is still uncontrollable for a long term after chemotherapy, and the 5-year survival rate is still very low. Therefore, developing a medication or combination therapy with lower toxicity and higher efficacy is of great meaning.

SUMMARY

By intensive research and creative efforts, the inventor correspondingly modified the Fc fragment of the anti-CTLA4/anti-PD-1 antibody structure to reduce the binding capacity of the Fc region to Fc receptors, thereby reducing ADCC, ADCP and/or CDC effects on immune cells and increasing the efficacy of the anti-CTLA4/anti-PD-1 antibody.

The present invention is detailed below.

One aspect of the present invention relates to a bispecific antibody, comprising:

a first protein functional region targeting PD-1, and

a second protein functional region targeting CTLA4;

wherein the first protein functional region is an immunoglobulin, and the second protein functional region is a single chain antibody; or, the first protein functional region is a single chain antibody, and the second protein functional region is an immunoglobulin;

wherein,

for the immunoglobulin, the heavy chain variable region comprises HCDR1-HCDR3 of amino acid sequences set forth in SEQ ID NOs: 27-29 respectively, and the light chain variable region comprises LCDR1-LCDR3 of amino acid sequences set forth in SEQ ID NOs: 30-32 respectively; for the single chain antibody, the heavy chain variable region comprises HCDR1-HCDR3 of amino acid sequences set forth in SEQ ID NOs: 33-35 respectively, and the light chain variable region comprises LCDR1-LCDR3 of amino acid sequences set forth in SEQ ID NOs: 36-38 respectively;

or,

for the immunoglobulin, the heavy chain variable region comprises HCDR1-HCDR3 of amino acid sequences set forth in SEQ ID NOs: 33-35 respectively, and the light chain variable region comprises LCDR1-LCDR3 of amino acid sequences set forth in SEQ ID NOs: 36-38 respectively; for the single chain antibody, the heavy chain variable region comprises HCDR1-HCDR3 of amino acid sequences set forth in SEQ ID NOs: 27-29 respectively, and the light chain variable region comprises LCDR1-LCDR3 of amino acid sequences set forth in SEQ ID NOs: 30-32 respectively;

the immunoglobulin is of human IgG1 subtype;

wherein, according to the EU numbering system, the heavy chain constant region of the immunoglobulin has mutations at any 2 or 3 of positions 234, 235 and 237, and the affinity constant of the bispecific antibody to FcyRIIIa and/or C1q is reduced after the mutation as compared to that before the mutation; preferably, the affinity constant is measured by a Fortebio Octet system.

In one or more embodiments of the present invention, for the bispecific antibody, after the mutation described above, the affinity constant of the bispecific antibody to FcyRIIIa, FcyRI, FcγRIIa_H131, FcγRIIIa_V158 and/or FcyRIIb is reduced as compared to that before the mutation; preferably, the affinity constant is measured by a Fortebio Octet system.

In one or more embodiments of the present invention, for the bispecific antibody, according to the EU numbering system, the heavy chain constant region of the immunoglobulin has the following mutations at positions 234, 235 and/or 237:

L234A and L235A;

L234A and G237A;

L235A and G237A;

or

L234A, L235A and G237A.

In the present invention, letters before the position number represent amino acids before mutation, and letters after the position number represent amino acids after mutation, unless otherwise specified.
The present invention further relates to a bispecific antibody, comprising:

a first protein functional region targeting PD-1, and

a second protein functional region targeting CTLA4;

wherein the first protein functional region is an immunoglobulin, and the second protein functional region is a single chain antibody; or, the first protein functional region is a single chain antibody, and the second protein functional region is an immunoglobulin;

wherein,

for the immunoglobulin, the heavy chain variable region comprises HCDR1-HCDR3 of amino acid sequences set forth in SEQ ID NOs: 27-29 respectively, and the light chain variable region comprises LCDR1-LCDR3 of amino acid sequences set forth in SEQ ID NOs: 30-32 respectively; for the single chain antibody, the heavy chain variable region comprises HCDR1-HCDR3 of amino acid sequences set forth in SEQ ID NOs: 33-35 respectively, and the light chain variable region comprises LCDR1-LCDR3 of amino acid sequences set forth in SEQ ID NOs: 36-38 respectively;

or,

for the immunoglobulin, the heavy chain variable region comprises HCDR1-HCDR3 of amino acid sequences set forth in SEQ ID NOs: 33-35 respectively, and the light chain variable region comprises LCDR1-LCDR3 of amino acid sequences set forth in SEQ ID NOs: 36-38 respectively; for the single chain antibody, the heavy chain variable region comprises HCDR1-HCDR3 of amino acid sequences set forth in SEQ ID NOs: 27-29 respectively, and the light chain variable region comprises LCDR1-LCDR3 of amino acid sequences set forth in SEQ ID NOs: 30-32 respectively;

the immunoglobulin is of human IgG1 subtype;

wherein, according to the EU numbering system, the heavy chain constant region of the immunoglobulin has the following mutations at positions 234, 235 and/or 237:

L234A and L235A;

L234A and G237A;

L235A and G237A; or

L234A, L235A and G237A.

In one or more embodiments of the present invention, for the bispecific antibody, according to the EU numbering system, the heavy chain constant region of the immunoglobulin has one or more mutations selected from: N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.
In one or more embodiments of the present invention, the bispecific antibody is in the form of IgG-scFv, i.e., the Morrison format.
In one or more embodiments of the present invention, for the bispecific antibody,

the amino acid sequence of the heavy chain variable region of the immunoglobulin is selected from SEQ ID NO: 14 and SEQ ID NO: 18; the amino acid sequence of the light

chain variable region of the immunoglobulin is selected from SEQ ID NO: 16 and SEQ ID NO: 20; the amino acid sequence of the heavy chain variable region of the single chain antibody is selected from SEQ ID NO: 2, SEQ ID NO: 6, SEQ ID NO: 10, SEQ ID NO: 41 and SEQ ID NO: 43; the amino acid sequence of the light chain variable region of the single chain antibody is selected from SEQ ID NO: 4, SEQ ID NO: 8, SEQ ID NO: 12, SEQ ID NO: 42 and SEQ ID NO: 44;

or,

the amino acid sequence of the heavy chain variable region of the immunoglobulin is selected from SEQ ID NO: 2, SEQ ID NO: 6, SEQ ID NO: 10, SEQ ID NO: 41 and SEQ ID NO: 43; the amino acid sequence of the light chain variable region of the immunoglobulin is selected from SEQ ID NO: 4, SEQ ID NO: 8, SEQ ID NO: 12, SEQ ID NO: 42 and SEQ ID NO: 44; the amino acid sequence of the heavy chain variable region of the single chain antibody is selected from SEQ ID NO: 14 and SEQ ID NO: 18; the amino acid sequence of the light chain variable region of the single chain antibody is selected from SEQ ID NO: 16 and SEQ ID NO: 20.

In one or more embodiments of the present invention, the bispecific antibody is selected from any one of the following (1)-(20):

(1)
the amino acid sequence of the heavy chain variable region of the immunoglobulin is set forth in SEQ ID NO: 14, and the amino acid sequence of the light chain variable region of the immunoglobulin is set forth in SEQ ID NO: 16; the amino acid sequence of the heavy chain variable region of the single chain antibody is set forth in SEQ ID NO: 2, and the amino acid sequence of the light chain variable region of the single chain antibody is set forth in SEQ ID NO: 4;

(2)
the amino acid sequence of the heavy chain variable region of the immunoglobulin is set forth in SEQ ID NO: 14, and the amino acid sequence of the light chain variable region of the immunoglobulin is set forth in SEQ ID NO: 16; the amino acid sequence of the heavy chain variable region of the single chain antibody is set forth in SEQ ID NO: 6, and the amino acid sequence of the light chain variable region of the single chain antibody is set forth in SEQ ID NO: 8;

(3)
the amino acid sequence of the heavy chain variable region of the immunoglobulin is set forth in SEQ ID NO: 14, and the amino acid sequence of the light chain variable region of the immunoglobulin is set forth in SEQ ID NO: 16; the amino acid sequence of the heavy chain variable region of the single chain antibody is set forth in SEQ ID NO: 10, and the amino acid sequence of the light chain variable region of the single chain antibody is set forth in SEQ ID NO: 12;

(4)
the amino acid sequence of the heavy chain variable region of the immunoglobulin is set forth in SEQ ID NO: 18, and the amino acid sequence of the light chain variable region of the immunoglobulin is set forth in SEQ ID NO: 20; the amino acid sequence of the heavy chain variable region of the single chain antibody is set forth in SEQ ID NO: 2, and the amino acid sequence of the light chain variable region of the single chain antibody is set forth in SEQ ID NO: 4;

(5)
the amino acid sequence of the heavy chain variable region of the immunoglobulin is set forth in SEQ ID NO: 18, and the amino acid sequence of the light chain variable region of the immunoglobulin is set forth in SEQ ID NO: 20; the amino acid sequence of the heavy chain variable region of the single chain antibody is set forth in SEQ ID NO: 6, and the amino acid sequence of the light chain variable region of the single chain antibody is set forth in SEQ ID NO: 8;

(6)
the amino acid sequence of the heavy chain variable region of the immunoglobulin is set forth in SEQ ID NO: 18, and the amino acid sequence of the light chain variable region of the immunoglobulin is set forth in SEQ ID NO: 20; the amino acid sequence of the heavy chain variable region of the single chain antibody is set forth in SEQ ID NO: 10, and the amino acid sequence of the light chain variable region of the single chain antibody is set forth in SEQ

ID NO: 12;

(7)
the amino acid sequence of the heavy chain variable region of the immunoglobulin is set forth in SEQ ID NO: 2, and the amino acid sequence of the light chain variable region of the immunoglobulin is set forth in SEQ ID NO: 4; the amino acid sequence of the heavy chain variable region of the single chain antibody is set forth in SEQ ID NO: 14, and the amino acid sequence of the light chain variable region of the single chain antibody is set forth in SEQ ID NO: 16;

(8)
the amino acid sequence of the heavy chain variable region of the immunoglobulin is set forth in SEQ ID NO: 2, and the amino acid sequence of the light chain variable region of the immunoglobulin is set forth in SEQ ID NO: 4; the amino acid sequence of the heavy chain variable region of the single chain antibody is set forth in SEQ ID NO: 18, and the amino acid sequence of the light chain variable region of the single chain antibody is set forth in SEQ ID NO: 20;

(9)
the amino acid sequence of the heavy chain variable region of the immunoglobulin is set forth in SEQ ID NO: 6, and the amino acid sequence of the light chain variable region of the immunoglobulin is set forth in SEQ ID NO: 8; the amino acid sequence of the heavy chain variable region of the single chain antibody is set forth in SEQ ID NO: 14, and the amino acid sequence of the light chain variable region of the single chain antibody is set forth in SEQ ID NO: 16;

(10)
the amino acid sequence of the heavy chain variable region of the immunoglobulin is set forth in SEQ ID NO: 6, and the amino acid sequence of the light chain variable region of the immunoglobulin is set forth in SEQ ID NO: 8; the amino acid sequence of the heavy chain variable region of the single chain antibody is set forth in SEQ ID NO: 18, and the amino acid sequence of the light chain variable region of the single chain antibody is set forth in SEQ ID NO: 20;

(11)
the amino acid sequence of the heavy chain variable region of the immunoglobulin is set forth in SEQ ID NO: 10, and the amino acid sequence of the light chain variable region of the immunoglobulin is set forth in SEQ ID NO: 12; the amino acid sequence of the heavy chain variable region of the single chain antibody is set forth in SEQ ID NO: 14, and the amino acid sequence of the light chain variable region of the single chain antibody is set forth in SEQ ID NO: 16;

(12)
the amino acid sequence of the heavy chain variable region of the immunoglobulin is set forth in SEQ ID NO: 10, and the amino acid sequence of the light chain variable region of the immunoglobulin is set forth in SEQ ID NO: 12; the amino acid sequence of the heavy chain variable region of the single chain antibody is set forth in SEQ ID NO: 18, and the amino acid sequence of the light chain variable region of the single chain antibody is set forth in SEQ ID NO: 20;

(13)
the amino acid sequence of the heavy chain variable region of the immunoglobulin is set forth in SEQ ID NO: 14, and the amino acid sequence of the light chain variable region of the immunoglobulin is set forth in SEQ ID NO: 16; the amino acid sequence of the heavy chain variable region of the single chain antibody is set forth in SEQ ID NO: 41, and the amino acid sequence of the light chain variable region of the single chain antibody is set forth in SEQ ID NO: 42;

(14)
the amino acid sequence of the heavy chain variable region of the immunoglobulin is set forth in SEQ ID NO: 14, and the amino acid sequence of the light chain variable region of the immunoglobulin is set forth in SEQ ID NO: 16; the amino acid sequence of the heavy chain variable region of the single chain antibody is set forth in SEQ ID NO: 43, and the amino acid sequence of the light chain variable region of the single chain antibody is set forth in SEQ ID NO: 44;

(15)
the amino acid sequence of the heavy chain variable region of the immunoglobulin is set forth in SEQ ID NO: 18, and the amino acid sequence of the light chain variable region of the immunoglobulin is set forth in SEQ ID NO: 20; the amino acid sequence of the heavy chain variable region of the single chain antibody is set forth in SEQ ID NO: 41, and the amino acid sequence of the light chain variable region of the single chain antibody is set forth in SEQ ID NO: 42;

(16)
the amino acid sequence of the heavy chain variable region of the immunoglobulin is set forth in SEQ ID NO: 18, and the amino acid sequence of the light chain variable region of the immunoglobulin is set forth in SEQ ID NO: 20; the amino acid sequence of the heavy chain variable region of the single chain antibody is set forth in SEQ ID NO: 43, and the amino acid sequence of the light chain variable region of the single chain antibody is set forth in SEQ ID NO: 44;

(17)
the amino acid sequence of the heavy chain variable region of the immunoglobulin is set forth in SEQ ID NO: 41, and the amino acid sequence of the light chain variable region of the immunoglobulin is set forth in SEQ ID NO: 42; the amino acid sequence of the heavy chain variable region of the single chain antibody is set forth in SEQ ID NO: 14, and the amino acid sequence of the light chain variable region of the single chain antibody is set forth in SEQ ID NO: 16;

(18)
the amino acid sequence of the heavy chain variable region of the immunoglobulin is set forth in SEQ ID NO: 43, and the amino acid sequence of the light chain variable region of the immunoglobulin is set forth in SEQ ID NO: 44; the amino acid sequence of the heavy chain variable region of the single chain antibody is set forth in SEQ ID NO: 14, and the amino acid sequence of the light chain variable region of the single chain antibody is set forth in SEQ ID NO: 16;

(19)
the amino acid sequence of the heavy chain variable region of the immunoglobulin is set forth in SEQ ID NO: 41, and the amino acid sequence of the light chain variable region of
the immunoglobulin is set forth in SEQ ID NO: 42; the amino acid sequence of the heavy chain variable region of the single chain antibody is set forth in SEQ ID NO: 18, and the amino acid sequence of the light chain variable region of the single chain antibody is set forth in SEQ ID NO: 20;
and,

(20)
the amino acid sequence of the heavy chain variable region of the immunoglobulin is set forth in SEQ ID NO: 43, and the amino acid sequence of the light chain variable region of the immunoglobulin is set forth in SEQ ID NO: 44; the amino acid sequence of the heavy chain variable region of the single chain antibody is set forth in SEQ ID NO: 18, and the amino acid sequence of the light chain variable region of the single chain antibody is set forth in SEQ ID NO: 20.

In one or more embodiments of the present invention, for the bispecific antibody,
the amino acid sequence of the heavy chain of the immunoglobulin is set forth in SEQ ID NO: 40, and the amino acid sequence of the light chain of the immunoglobulin is set forth in SEQ ID NO: 24.

In one or more embodiments of the present invention, for the bispecific antibody, the immunoglobulin or the antigen-binding fragment thereof binds to FcγRIIIa_F158, FcyRI, FcγRIIa_H131, FcγRIIIa_V158 and/or FcyRIIb with an affinity constant greater than about $10^{-7}$ M, for example, greater than about $10^{-6}$ M, $10^{-5}$ M, $10^{-4}$ M, or $10^{-3}$ M or greater; preferably, the affinity constant is measured by a Fortebio Octet system;
preferably, the immunoglobulin or the antigen binding fragment thereof has no binding signal or a binding signal of less than 0.1 nm to FcγRIIIa_F158, FcyRI, FcγRIIa_H131, FcγRIIIa_V158 and/or FcyRIIb; preferably, the binding signal refers to a response measured by a Fortebio Octet system.

In one or more embodiments of the present invention, for the bispecific antibody, the immunoglobulin or the antigen-binding fragment thereof binds to C1q with an affinity constant greater than about $10^{-9}$ M, for example, greater than about $10^{-8}$ M, $10^{-7}$ M, $10^{-6}$ M, or $10^{-5}$ M or greater; preferably, the affinity constant is measured by a Fortebio Octet system;
preferably, the immunoglobulin or the antigen binding fragment thereof has no binding signal or a binding signal of less

than 0.1 nm to C1q; preferably, the binding signal refers to a response measured by a Fortebio Octet system.

In one or more embodiments of the present invention, for the bispecific antibody, the first protein functional region is linked to the second protein functional region either directly or via a linker fragment; and/or the heavy chain variable region of the single chain antibody is linked to the light chain variable region of the single chain antibody either directly or via a linker fragment.

In one or more embodiments of the present invention, for the bispecific antibody, the linker fragment is (GGGGS)n, n being a positive integer; preferably, n is 1, 2, 3, 4, 5 or 6.

In one or more embodiments of the present invention, for the bispecific antibody, the numbers of the first protein functional region and the second protein functional region are each independently 1, 2 or more.

In one or more embodiments of the present invention, for the bispecific antibody, the number of the first protein functional region is 1 and the number of the second protein functional region is 2.

In one or more embodiments of the present invention, for the bispecific antibody, the single chain antibody is linked to the C terminus of the heavy chain of the immunoglobulin. Since an immunoglobulin has two heavy chains, two single chain antibody molecules are linked to one immunoglobulin molecule. Preferably, the two single chain antibody molecules are identical. Preferably, the single chain antibody is linked to the C terminus of the heavy chain of the immunoglobulin by forming an amide bond via the aforementioned linker fragment.

In one or more embodiments of the present invention, the constant regions of the immunoglobulin are humanized. For example, the heavy chain constant region is Ig gamma-1 chain C region, ACCESSION: P01857, and the light chain constant region is Ig kappa chain C region, ACCESSION: P01834.

In one or more embodiments of the present invention, the bispecific antibody binds to CTLA4 protein and/or PD-1 protein with a $K_D$ less than about $10^{-5}$ M, e.g., less than about $10^{-6}$ M, $10^{-7}$ M, $10^{-8}$ M, $10^{-9}$ M or $10^{-10}$ M or less.

In one or more embodiments of the present invention, the bispecific antibody is a monoclonal antibody.

In one or more embodiments of the present invention, the bispecific antibody is a humanized antibody.

In one or more embodiments of the present invention, the bispecific antibody comprises:

a first protein functional region targeting PD-1, and

a second protein functional region targeting CTLA4;

the number of the first protein functional region is 1, and the number of the second protein functional region is 2;

wherein the first protein functional region is an immunoglobulin, and the second protein functional region is a single chain antibody;

the amino acid sequence of the heavy chain of the immunoglobulin is set forth in SEQ ID NO: 40, and the amino acid sequence of the light chain of the immunoglobulin is set forth in SEQ ID NO: 24;

the amino acid sequence of the heavy chain variable region of the single chain antibody is set forth in SEQ ID NO: 43, and the amino acid sequence of the light chain variable region of the single chain antibody is set forth in SEQ ID NO: 44;

the single chain antibody is linked to the C terminus of the heavy chain of the immunoglobulin;

the first protein functional region is linked to the second protein functional region via a first linker fragment; and the heavy chain variable region of the single chain antibody is linked to the light chain variable region of the single chain antibody via a second linker fragment; the first linker fragment and the second linker fragment are the same or different;

preferably, the amino acid sequences of the first linker fragment and second linker fragment are independently selected from SEQ ID NO: 25 and SEQ ID NO: 26;

preferably, the amino acid sequences of the first linker fragment and second linker fragments are set forth in SEQ ID NO: 26.

Another aspect of the present invention relates to an isolated nucleic acid molecule encoding the bispecific antibody according to any embodiment of the present invention.

The present invention also relates to a vector comprising the isolated nucleic acid molecule of the present invention.

The present invention also relates to a host cell comprising the isolated nucleic acid molecule of the present

invention or the vector of the present invention.

Another aspect of the present invention relates to a conjugate comprising an antibody or antigen-binding fragment thereof, and a conjugated moiety, wherein the immunoglobulin is the bispecific antibody according to any embodiment of the present invention, and the conjugated moiety is a detectable label; preferably, the conjugated moiety is a radioisotope, a fluorescent substance, a luminescent substance, a colored substance, or an enzyme.

Another aspect of the present invention relates to a kit comprising the bispecific antibody according to any embodiment of the present invention or comprising the conjugate of the present invention;
wherein preferably, the kit further comprises a second antibody capable of specifically recognizing the immunoglobulin or the antigen binding fragment thereof; optionally, the second antibody further comprises a detectable label, for example, a radioisotope, a fluorescent substance, a luminescent substance, a colored substance, or an enzyme.

Another aspect of the present invention relates to use of the bispecific antibody or the conjugate according to any embodiment of the present invention in preparing a kit for detecting the presence or level of PD-1 and/or CTLA4 in a sample.

Another aspect of the present invention relates to a pharmaceutical composition comprising the bispecific antibody or the conjugate according to any embodiment of the present invention; optionally, the pharmaceutical composition further comprises a pharmaceutically acceptable carrier and/or excipient.

In one or more embodiments of the present invention, the pharmaceutical composition further comprises one or more anti-tumor chemotherapeutics;
preferably, the anti-tumor chemotherapeutic is a tyrosine kinase inhibitor; more preferably, the anti-tumor chemotherapeutic is anlotinib or a pharmaceutically acceptable salt thereof (e.g., hydrochloride salt), or lenvatinib or a pharmaceutically acceptable salt thereof (e.g., mesylate salt).

In one or more embodiments of the present invention, the unit dose of the pharmaceutical composition is 100-1000 mg, 200-800 mg, 200-500 mg, 300-600 mg, 400-500 mg, or 450 mg, based on the mass of the bispecific antibody.

Another aspect of the present invention relates to a combination product comprising a first product and a second product in separate packages,

wherein,

the first product comprises the bispecific antibody, the conjugate or the pharmaceutical composition according to any embodiment of the present invention;

the second product comprises one or more anti-tumor chemotherapeutics; preferably, the anti-tumor chemotherapeutic is a tyrosine kinase inhibitor; more preferably, the anti-tumor chemotherapeutic is anlotinib or a pharmaceutically acceptable salt thereof (e.g., hydrochloride salt), or lenvatinib or a pharmaceutically acceptable salt thereof (e.g., mesylate salt);

preferably, the first product and the second product further independently comprise one or more pharmaceutically acceptable excipients;

preferably, the combination product further comprises a package insert.

In one or more embodiments of the present invention, for the combination product, the unit dose of the first product is 100-1000 mg, 200-800 mg, 200-500 mg, 300-600 mg, 400-500 mg, or 450 mg, based on the mass of the bispecific antibody.

In one or more embodiments of the present invention, for the combination product, the unit dose of the second product is 0.1-100 mg, 0.5-50 mg, 0.5-10 mg, 1-10 mg, 2-8 mg, or 1-5 mg, based on the mass of the active ingredient.

In one or more embodiments of the present invention, for the combination product, the unit dose of the second product is 1-20 mg, 2-15 mg, 4-12 mg, or 8-12 mg, based on the mass of the active ingredient.

Another aspect of the present invention relates to use of the bispecific antibody, the conjugate, the pharmaceutical composition or the combination product according to any embodiment of the present invention in preparing a medicament for treating and/or preventing a tumor or anemia, or in preparing a medicament for diagnosing a tumor or anemia; preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;

preferably, the tumor is a solid tumor of MSI-H/dMMR phenotype; preferably, the tumor is colon cancer, rectal cancer, endometrial cancer, gastric cancer, mesothelioma, sarcoma, adrenocortical carcinoma, malignant melanoma or

ovarian germ cell neoplasm of MSI-H/dMMR phenotype;

preferably, the lung cancer is selected from one or more of non-small cell lung cancer, small cell lung cancer and squamous cell lung cancer;

preferably, the gastric cancer is gastric adenocarcinoma or gastroesophageal junction adenocarcinoma.

MSI refers to microsatellite instability. Microsatellites are short tandem repeats throughout the human genome, including 10-50 repeats of one, two or more nucleotides. Microsatellites in certain abnormal cells, such as tumors, are altered in length by insertion or deletion of repeat units as compared to normal cells. Such alteration is referred to as MSI. Based on instability and extent, MSI can be classified as microsatellite instability-high (MSI-H), microsatellite instability-low (MSI-L) and microsatellite stable (MSS). The major cause of MSI is DNA mismatch repair (MMR). Human mismatch repair genes (MMR genes) can express corresponding mismatch repair proteins through transcription and translation. Absence of any MMR protein may lead to mismatch repair deficiency, and basepair mismatch will accumulate in the process of DNA replication due to such deficiency, ultimately resulting in MSI. About 15% of colorectal cancers are attributed to the MSI pathway. This was first reported in colorectal cancer, and may also occur in gastric cancer, endometrial cancer, adrenocortical carcinoma and the like (Baretti M et al., Pharmacol Ther., 2018; 189:45-62). MSI-H/dMMR characteristics were also found in mesothelioma, sarcoma, adrenocortical carcinoma, malignant melanoma and ovarian germ cell neoplasm in subsequent studies.

MSI-H and dMMR represent the results of two different assays and are biologically consistent, called MSI-H/dMMR or MSI-high/dMMR, while MSI-L and MSS are phenotypes of proficient MMR (pMMR). The detection of dMMR is to carry out immunohistochemistry of protein expression for four mismatch genes of MSH2, MLH1, MSH6 and PMS2 based on tumor specimens (including surgical specimens and aspiration specimens). Absence of any of the four proteins confirms the dMMR; positive results of all the four proteins indicate pMMR, i.e., a complete mismatch repair function. The detection of MSI is to match the length of the repeated DNA sequences (microsatellite sequences) in tumor cells and somatic cells, and to compare the lengths. When 5 standard loci are detected using PCR based on the American NCI standard, inconsistencies in two or more loci indicate instability, defined as MSI-H, one inconsistent locus indicates MSI-L, and 5 consistent loci indicate MSS. High-throughput sequencing (also referred to as next-generation sequencing, or NGS) can also be used as a method for detecting microsatellite instability. When more microsatellite loci are selected, such as more than 5 loci or other microsatellite loci, for PCR assay, inconsistency in ≥30% loci is defined as MSI-H, consistency in all loci is defined as MSS, and inconsistency between 0 and 30% is defined as MSI-L.

Another aspect of the present invention relates to use of the bispecific antibody or the conjugate according to any embodiment of the present invention in preparing:

a medicament for blocking the binding of PD-1 to PD-L1,

a medicament for down-regulating the activity or level of PD-1,

a medicament for relieving the immunosuppression of PD-1 in an organism, or

a medicament for elevating IFN-γ and/or IL-2 expression in T lymphocytes;

and/or

a medicament for blocking the binding of CTLA4 to B7,

a medicament for down-regulating the activity or level of CTLA4,

a medicament for relieving the immunosuppression of CTLA4 in an organism, or

a medicament for elevating IL-2 expression in T lymphocytes.

Another aspect of the present invention relates to a method for the prevention and/or

treatment and/or adjuvant treatment and/or diagnosis of a tumor, comprising:

administering to a subject in need an effective amount of the bispecific antibody, the conjugate, the pharmaceutical composition or the combination product according to any embodiment of the present invention; preferably, the tumor

is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;

preferably, the lung cancer is selected from one or more of non-small cell lung cancer, small cell lung cancer and squamous cell lung cancer;

preferably, the gastric cancer is gastric adenocarcinoma or gastroesophageal junction adenocarcinoma;

preferably, the tumor is a solid tumor of MSI-H/dMMR phenotype; preferably, the tumor is selected from one or more of the following tumors of MSI-H/dMMR phenotype:

colon cancer, rectal cancer, endometrial cancer, gastric cancer, mesothelioma, sarcoma, adrenocortical carcinoma, malignant melanoma and ovarian germ cell neoplasm.

In one or more embodiments of the present invention, for the method, the administration is before or after a surgical treatment and/or before or after a radiotherapy.

In one or more embodiments of the present invention, the method, wherein,

the unit dose of anti-CTLA4/anti-PD-1 bispecific antibody is 0.1-100 mg, preferably 1-10 mg (e.g., 1 mg, 2 mg, 3 mg, 4 mg, 5 mg, 6 mg, 7 mg, 8 mg, 9 mg or 10 mg) per kg body weight; alternatively, the unit dose of the anti-CTLA4/anti-PD-1 bispecific antibody is 10-1000 mg (e.g., about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg or about 1000 mg), preferably 50-500 mg, 100-400 mg, 150-300 mg, 150-250 mg or 200 mg in each subject;

preferably, the dose is given once every 3 days, 4 days, 5 days, 6 days, 10 days, 1 week, 2 weeks or 3 weeks;

preferably, the route of administration is intravenous drip infusion or intravenous injection.

In some embodiments, the administration of the anti-CTLA4/anti-PD-1 bispecific antibody is performed in cycles of 2 weeks (14 days) or 3 weeks (21 days), and preferably, the anti-CTLA4/anti-PD-1 bispecific antibody is administered intravenously on the first day (D1) of each cycle. For example, the anti-CTLA4/anti-PD-1 bispecific antibody is administered once every two weeks (q2w) or three weeks (q3w).

Also provided is the bispecific antibody, the conjugate, the pharmaceutical composition or the combination product according to any embodiment of the present invention for use in the prevention and/or treatment and/or adjuvant treatment and/or diagnosis of a tumor; preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;

preferably, the lung cancer is selected from one or more of non-small cell lung cancer, small cell lung cancer and squamous cell lung cancer;

preferably, the gastric cancer is gastric adenocarcinoma or gastroesophageal junction adenocarcinoma;

preferably, the tumor is a solid tumor of MSI-H/dMMR phenotype; preferably, the tumor is selected from one or more of the following tumors of MSI-H/dMMR phenotype:

colon cancer, rectal cancer, endometrial cancer, gastric cancer, mesothelioma, sarcoma, adrenocortical carcinoma, malignant melanoma and ovarian germ cell neoplasm.

Provided is the bispecific antibody or conjugate according to any embodiment of the present invention for use in:

blocking the binding of PD-1 to PD-L1,

down-regulating the activity or level of PD-1,

relieving the immunosuppression of PD-1 in an organism, or

elevating IFN-γ expression in T lymphocytes;

and/or

blocking the binding of CTLA4 to B7,

down-regulating the activity or level of CTLA4,

relieving the immunosuppression of CTLA4 in an organism, or

elevating IL-2 expression in T lymphocytes.

Antibody drugs, especially monoclonal antibodies (mAbs), have achieved good efficacy in the treatment of various diseases. Traditional experimental methods for acquiring these therapeutic antibodies are to immunize animals with the antigen and acquire antibodies targeting the antigen in the immunized animals, or to improve those antibodies with lower affinity for the antigen by affinity maturation.
The variable regions of the light chain and the heavy chain determine the binding of the antigen; the variable region of each chain comprises three hypervariable regions, i.e., complementarity determining regions (CDRs) (the CDRs of the heavy chain (H) include HCDR1, HCDR2, HCDR3, and the CDRs of the light chain (L) include LCDR1, LCDR2, LCDR3; defined by Kabat et al., see Sequences of Proteins of Immunological Interest, Fifth Edition (1991), Volumes 1-3, NIH Publication 91-3242, Bethesda Md).
The amino acid sequences of the CDR regions of the monoclonal antibody in (1) to (13) below are analyzed by technical means well known to those skilled in the art, for example, by a VBASE2 database, and the results are as follows:

(1) 14C12

The amino acid sequence of the heavy chain variable region is set forth in SEQ ID NO: 14, and the amino acid sequence of the light chain variable region is set forth in SEQ ID NO: 16.
The amino acid sequences of the 3 CDR regions of the heavy chain variable region are as follows:

HCDR1: GFAFSSYD (SEQ ID NO: 27)

HCDR2: ISGGGRYT (SEQ ID NO: 28)

HCDR3: ANRYGEAWFAY (SEQ ID NO: 29)

The amino acid sequences of the 3 CDR regions of the light chain variable region are as follows:

LCDR1: QDINTY (SEQ ID NO: 30)

LCDR2: RAN (SEQ ID NO: 31)

LCDR3: LQYDEFPLT (SEQ ID NO: 32)

(2) 14C12H1L1

The amino acid sequence of the heavy chain variable region is set forth in SEQ ID NO: 18, and the amino acid sequence of the light chain variable region is set forth in SEQ ID NO: 20.
The amino acid sequences of the 3 CDR regions of the heavy chain variable region are the same as 14C12.
The amino acid sequences of the 3 CDR regions of the light chain variable region are the same as 14C12.

(3) 4G10

The amino acid sequence of the heavy chain variable region is set forth in SEQ ID NO: 2, and the amino acid sequence of the light chain variable region is set forth in SEQ ID NO: 4.
The amino acid sequences of the 3 CDR regions of the heavy chain variable region are as follows:

HCDR1: GYSFTGYT (SEQ ID NO: 33)

HCDR2: INPYNNIT (SEQ ID NO: 34)

HCDR3: ARLDYRSY (SEQ ID NO: 35)

The amino acid sequences of the 3 CDR regions of the light chain variable region are as follows:

LCDR1: TGAVTTSNF (SEQ ID NO: 36)

LCDR2: GTN (SEQ ID NO: 37)

LCDR3: ALWYSNHWV (SEQ ID NO: 38)

(4) 4G10H1L1

The amino acid sequence of the heavy chain variable region is set forth in SEQ ID NO: 6, and the amino acid sequence of the light chain variable region is set forth in SEQ ID NO: 8;

The amino acid sequences of the 3 CDR regions of the heavy chain variable region are the same as 4G10.

The amino acid sequences of the 3 CDR regions of the light chain variable region are the same as 4G10.

(5) 4G10H3L3

The amino acid sequence of the heavy chain variable region is set forth in SEQ ID NO: 10, and the amino acid sequence of the light chain variable region is set forth in SEQ ID NO: 12. The amino acid sequences of the 3 CDR regions of the heavy chain variable region are the same as 4G10.

The amino acid sequences of the 3 CDR regions of the light chain variable region are the same as 4G10.

(6) BiAb001(M)

The amino acid sequences of the 9 CDR regions associated with the heavy chain variable region are as follows:

HCDR1: GFAFSSYD (SEQ ID NO: 27)

HCDR2: ISGGGRYT (SEQ ID NO: 28)

HCDR3: ANRYGEAWFAY (SEQ ID NO: 29)

HCDR4: GYSFTGYT (SEQ ID NO: 33)

HCDR5: INPYNNIT (SEQ ID NO: 34)

HCDR6: ARLDYRSY (SEQ ID NO: 35)

HCDR7: TGAVTTSNF (SEQ ID NO: 36)

HCDR8: GTN (SEQ ID NO: 37)

HCDR9: ALWYSNHWV (SEQ ID NO: 38)

The amino acid sequences of the 3 CDR regions associated with the light chain variable region are as follows:

LCDR1: QDINTY (SEQ ID NO: 30)

LCDR2: RAN (SEQ ID NO: 31)

LCDR3: LQYDEFPLT (SEQ ID NO: 32)

(7) BiAb002(M)

The amino acid sequences of the 9 CDR regions associated with the heavy chain variable region are the same as BiAb001(M).
The amino acid sequences of the 3 CDR regions associated with the light chain variable region are the same as BiAb001(M).

(8) BiAb003(M)

The amino acid sequences of the 9 CDR regions associated with the heavy chain variable region are the same as BiAb001(M).
The amino acid sequences of the 3 CDR regions associated with the light chain variable region are the same as BiAb001(M).

(9) BiAb004(M)

The amino acid sequences of the 9 CDR regions associated with the heavy chain variable region are the same as BiAb001(M).
The amino acid sequences of the 3 CDR regions associated with the light chain variable region are the same as BiAb001(M).
For the antibody BiAb004(hG1TM) of the present invention, amino acid mutations are introduced into the non-variable region of BiAb004(M). According to the EU numbering system, amino acid mutations are introduced at positions 234, 235 and 237:
BiAb004(hG1TM) is obtained by introducing a leucine-to-alanine point mutation at position 234 (L234A), a leucine-to-alanine point mutation at position 235 (L235A), and a glycine-to-alanine point mutation at position 237 (G237A) in the hinge region of the heavy chain.
In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.
As used herein, when referring to the amino acid sequence of CTLA4 protein (cytotoxic T-lymphocyte antigen 4), it includes the full length of CTLA4 protein, or the extracellular fragment CTLA4ECD of CTLA4 or a fragment comprising CTLA4ECD; also included are fusion proteins of CTLA4ECD, such as a fragment fused to an Fc protein fragment of a mouse or human IgG (mFc or hFc). However, those skilled in the art will appreciate that in the amino acid sequence of the CTLA4 protein, mutations or variations (including but not limited to, substitutions, deletions and/or additions) can be naturally produced or artificially introduced without affecting biological functions thereof. Therefore, in the present invention, the term "CTLA4 protein" should include all such sequences, including their natural or artificial variants. In addition, when describing the sequence fragment of the CTLA4 protein, it also includes the corresponding sequence fragments in its natural or artificial variants.
As used herein, when referring to the amino acid sequence of PD-1 protein (NCBI GenBank: NM_005018), it includes the full length of the PD-1 protein, or the extracellular fragment PD-1ECD of PD-1 or a fragment comprising PD-1ECD; also included are fusion proteins of PD-1ECD, such as a fragment fused to an Fc protein fragment of a mouse or human IgG (mFc or hFc). However, those skilled in the art will appreciate that in the amino acid sequence of the PD-1 protein, mutations or variations (including but not limited to, substitutions, deletions and/or additions) can be naturally produced or artificially introduced without affecting biological functions thereof. Therefore, in the present invention, the term "PD-1 protein" should include all such sequences, including their natural or artificial variants. In addition, when describing the sequence fragment of the PD-1 protein, it also includes the corresponding sequence fragments in its natural or artificial variants.
As used herein, unless otherwise specified, the B7 is B7-1 and/or B7-2; specific sequences thereof are those known in the prior art, and reference may be made to sequences disclosed in the existing literature or GenBank. For example, B7-1 (CD80, NCBI Gene ID: 941); B7-2 (CD86, NCBI Gene ID: 942).
As used herein, the term $EC_{50}$ refers to the concentration for 50% of maximal effect, i.e., the concentration that can cause 50% of the maximal effect.
As used herein, the term "antibody" refers to an immunoglobulin molecule that generally consists of two pairs of polypeptide chains (each pair with one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains are classified as κ and λ light chains.

Heavy chains are generally classified as μ, δ, γ, α, or ε, and isotypes of antibodies are defined as IgM, IgD, IgG, IgA, and IgE, respectively. In light chains and heavy chains, the variable region and constant region are linked by a "J" region of about 12 or more amino acids, and the heavy chain also comprises a "D" region of about 3 or more amino acids. Each heavy chain consists of a heavy chain variable region ($V_H$) and a heavy chain constant region ($C_H$). The heavy chain constant region consists of 3 domains ($C_{H1}$, $C_{H2}$, and $C_{H3}$). Each light chain consists of a light chain variable region ($V_L$) and a light chain constant region ($C_L$). The light chain constant region consists of one domain $C_L$. The constant region of the antibody can mediate the binding of immunoglobulins to host tissues or factors, including the binding of various cells of the immune system (e.g., effector cells) to the first component (C1q) of classical complement system. The $V_H$ and $V_L$ regions can be further subdivided into highly variable regions (called Complementarity Determining Regions (CDRs)), between which conservative regions called framework regions (FRs) are distributed. Each $V_H$ and $V_L$ consists of 3 CDRs and 4 FRs arranged from amino terminus to carboxyl terminus in the following order: FR1, CDR1, FR2, CDR2, FR3, CDR3, FR4. The variable regions ($V_H$ and $V_L$) of each heavy chain/light chain pair form antibody binding sites, respectively. The assignment of amino acids to each region or domain follows the definition of Kabat Sequences of Proteins of Immunological Interest (National Institutes of Health, Bethesda, MD. (1987 and 1991)), Chothia & Lesk, (1987) J. Mol. Biol., 196:901-917, or Chothia et al. (1989) Nature, 342:878-883. In particular, the heavy chain may also comprise more than 3 CDRs, such as 6, 9, or 12. For example, in the bispecific antibody of the present invention, the heavy chain may be an scFv with the C terminus of the heavy chain of IgG antibody linked to another antibody, and in this case, the heavy chain comprises 9 CDRs. The term "antibody" is not limited by any specific method for producing antibody. For example, the antibody includes, in particular, a recombinant antibody, a monoclonal antibody, and a polyclonal antibody. Antibodies can be different isotypes, such as IgG (e.g., subtype IgG1, IgG2, IgG3 or IgG4), IgA1, IgA2, IgD, IgE or IgM.

Antigen binding fragments (e.g., the above mentioned antibody fragments) of antibodies can be obtained from given antibodies by using conventional techniques known to those skilled in the art (e.g., DNA recombination, or enzymatic or chemical cleavage), and the antigen binding fragments of the antibodies are screened for specificity in the same way as for intact antibodies.

As used herein, unless otherwise clearly defined in the context, when referring to the term "antibody", it includes not only intact antibodies but also antigen binding fragments of antibodies.

As used herein, the terms "mAb" and "monoclonal antibody" refer to an antibody or a fragment thereof that is derived from a group of highly homologous antibodies, i.e. from a group of identical antibody molecules, except for natural mutations that may occur spontaneously. The monoclonal antibody is highly specific for a single epitope on an antigen. The polyclonal antibody, relative to the monoclonal antibody, generally comprises at least two or more different antibodies which generally identify different epitopes on an antigen. Monoclonal antibodies can generally be obtained using hybridoma technique first reported by Kohler et al. (Kohler et al., Nature, 256:495, 1975), but can also be obtained using DNA recombination (see, e.g., U.S. Patent No. 4,816,567).

As used herein, the term "humanized antibody" refers to an antibody or antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, for example, Jones et al., Nature, 321:522-525 (1986); Reichmann et al., Nature, 332:323-329 (1988); Presta, Curr. Op. Struct. Biol., 2:593-596 (1992); and Clark, Immunol. Today 21: 397-402 (2000).

As used herein, the term "epitope" refers to a site on the antigen that an immunoglobulin or antibody specifically binds to. "Epitope" is also called in the field as an "antigenic determinant". The epitope or antigenic determinant generally consists of chemically active surface groups of molecules such as amino acids, carbohydrates or sugar side chains, and usually has specific three-dimensional structural characteristics and specific charge characteristics. For example, the epitope generally comprises at least 3, 4, 5, 6, 7, 8, 9, 10, 11, 12, 13, 14, or 15 consecutive or non-consecutive amino acids in a unique spatial conformation, which can be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

As used herein, the term "isolated" refers to obtained by artificial means from natural state. If a certain "isolated" substance or component appears in nature, it may be the case that change occurs in its natural environment, or that it is isolated from the natural environment, or both. For example, a certain non-isolated polynucleotide or polypeptide naturally exists in a certain living animal, and the same polynucleotide or polypeptide with a high purity isolated from such a natural state is called isolated polynucleotide or polypeptide. The term "isolated" does not exclude the existence of artificial or synthetic substances or other impurities that do not affect the activity of the substance.

As used herein, the term "E. coli expression system" refers to an expression system consisting of E. coli (strain) and a vector, wherein the E. coli (strain) is derived from a commercially available strain, such as but not limited to GI698, ER2566, BL21 (DE3), B834 (DE3), and BLR (DE3).

As used herein, the term "vector" refers to a nucleic acid vehicle into which a polynucleotide can be inserted. When a vector allows for the expression of the protein encoded by the inserted polynucleotide, the vector is called an expression vector. A vector can be introduced into a host cell by transformation, transduction, or transfection so that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis,* fungal cells such as yeast cells or *aspergillus,* insect cells such as S2 drosophila cells or Sf9, or animal cells such as fibroblasts, CHO cells, COS cells, NSO cells, HeLa cells, BHK cells, HEK 293 cells, or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) refers to that the antibody binds to the antigen with an affinity ($K_D$) of less than about $10^{-5}$ M, such as less than about $10^{-6}$ M, $10^{-7}$ M, $10^{-8}$ M, $10^{-9}$ M or $10^{-10}$ M or less. In some embodiments of the present invention, the term "target" refers to specific binding.

As used herein, the term "$K_D$" refers to a dissociation equilibrium constant for a specific antibody-antigen interaction, which is used to describe the binding affinity between the antibody and the antigen. The smaller the equilibrium dissociation constant, the tighter the antibody-antigen binding, and the higher the affinity between the antibody and the antigen. Generally, an antibody binds to an antigen with a dissociation equilibrium constant ($K_D$) of less than about $10^{-5}$ M, such as less than about $10^{-6}$ M, $10^{-7}$ M, $10^{-8}$ M, $10^{-9}$ M or $10^{-10}$ M or less, for example, as measured by a BIACORE surface plasmon resonance (SPR) instrument or a Fortebio Octet system.

As used herein, the terms "monoclonal antibody" and "mAb" have the same meaning and can be used interchangeably; the terms "polyclonal antibody" and "pAb" have the same meaning and can be used interchangeably; the terms "polypeptide" and "protein" have the same meaning and can be used interchangeably. Besides, herein, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, e.g., Remington's Pharmaceutical Sciences, edited by Gennaro AR, 19th ed., Pennsylvania, Mack Publishing Company, 1995), including but not limited to: pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "adjuvant" refers to a non-specific immune enhancer, which can enhance the immune response of an organism to antigens or change the type of immune response when delivered into the organism together with the antigens or delivered into the organism in advance. There are various adjuvants, including, but not limited to, aluminum adjuvant (e.g., aluminum hydroxide), Freund's adjuvant (e.g., complete Freund's adjuvant and incomplete Freund's adjuvant), *Corynebacterium parvum,* lipopolysaccharide, cytokine, etc. The Freund's adjuvant is the most commonly used adjuvant in animal experiments. The aluminum hydroxide adjuvant is used more in clinical trials.

As used herein, the term "effective amount" refers to an amount sufficient to obtain or at least partially obtain desired effect. For example, a prophylactically effective amount (e.g., for a disease associated with binding of CTLA4 to B7 or CTLA4 overactivity, such as a tumor) is an amount sufficient to prevent, stop, or delay the onset of the disease (e.g., a disease associated with binding of CTLA4 to B7 or CTLA4 overactivity, such as a tumor); a therapeutically effective amount is an amount sufficient to cure or at least partially stop a disease and its complications in a patient suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, weight and gender, the route of administration, and other treatments given concurrently, etc.

A "recurrent" cancer is one that regenerates at the original site or a distant site after response to a previous

treatment (e.g., surgery). A "locally recurrent" cancer is one that occurs at the same site after treatment as the previously treated cancer.

A "metastatic" cancer refers to one that spreads from one part of the body (e.g., the lungs) to another.

As used herein, the term "completely eliminated" refers to the absence of binding signal or an extremely weak binding signal as detected by existing instrumentation (e.g., a Fortebio Octet system). In one embodiment of the present invention, the absence of binding signal or the extremely weak binding signal refers to a binding signal (i.e., response) below 0.1 nm.

In the present invention, the terms "first" (e.g., first protein functional region, first linker fragment or first product) and "second" (e.g., second protein functional region, second linker fragment or second product) are used for distinguishing or clarity in expression and do not carry typical sequential meanings, unless otherwise specified.

In the present invention, "about" or "approximately" refers to that the value or physical quantity defined fluctuates within a range of 10%, 20% or 30%, unless otherwise specified. For example, about 100 minutes or approximately 100 minutes may be 90 minutes to 110 minutes, 80 minutes to 120 minutes or 70 minutes to 130 minutes.

Advantages of the present invention:
The present invention achieves one or more of the following technical effects (1) to (3):

(1) The modification of the Fc fragment of the antibody of the present invention by the inventor completely eliminates the binding activity of BiAb004(hG1TM) to FcyRI, FcγRIIa_H131, FcγRIIIa_V158 and/or FcγRIIIa_F158, thereby eliminating the ADCC activity and/or ADCP activity.

(2) The modification of the Fc fragment of the antibody of the present invention by the inventor completely eliminates the binding activity to complement C1q, thereby eliminating the CDC activity.

(3) The antibody of the present invention has potential for use in preparing medicaments for preventing and treating tumors.

BRIEF DESCRIPTION OF THE DRAWINGS

FIG. 1: Affinity constant assay of BiAb004(hG1TM) to FcyRI. The antibody concentrations for the curve pairs from top to bottom are 50 nM, 25 nM, 12.5 nM, 6.25 nM and 3.12 nM, respectively.

FIG. 2: Affinity constant assay of BiAb004(hG1WT) to FcyRI. The antibody concentrations for the curve pairs from top to bottom are 50 nM, 25 nM, 12.5 nM, 6.25 nM and 3.12 nM, respectively.

FIG. 3: Affinity constant assay of BiAb004(hG1TM) to FcγRIIIa_V158. The antibody concentrations for the curve pairs from top to bottom are 500 nM, 250 nM, 125 nM, 62.5 nM and 31.25 nM, respectively.

FIG. 4: Affinity constant assay of BiAb004(hG1WT) to FcγRIIIa_V158. The antibody concentrations for the curve pairs from top to bottom are 500 nM, 250 nM, 125 nM, 62.5 nM and 31.25 nM, respectively.

FIG. 5: Affinity constant assay of BiAb004(hG1TM) to FcγRIIIa_F158. The antibody concentrations for the curve pairs from top to bottom are 500 nM, 250 nM, 125 nM, 62.5 nM and 31.25 nM, respectively.

FIG. 6: Affinity constant assay of BiAb004(hG1WT) to FcγRIIIa_F158. The antibody concentrations for the curve pairs from top to bottom are 500 nM, 250 nM, 125 nM, 62.5 nM and 31.25 nM, respectively.

FIG. 7: Affinity constant assay of BiAb004(hG1TM) to FcγRIIa_H131. The antibody concentrations for the curve pairs from top to bottom are 200 nM, 100 nM, 50 nM, 25 nM and 12.5 nM, respectively.

FIG. 8: Affinity constant assay of BiAb004(hG1WT) to FcγRIIa_H131. The antibody concentrations for the curve pairs from top to bottom are 200 nM, 100 nM, 50 nM, 25 nM and 12.5 nM, respectively.

FIG. 9: Affinity constant assay of BiAb004(hG1TM) to FcγRIIa_R131. The antibody concentrations for the curve pairs from top to bottom are 200 nM, 100 nM, 50 nM, 25 nM and 12.5 nM, respectively.

FIG. 10: Affinity constant assay of BiAb004(hG1WT) to FcγRIIa_R131. The antibody concentrations for the curve

pairs from top to bottom are 200 nM, 100 nM, 50 nM, 25 nM and 12.5 nM, respectively.

FIG. 11: Affinity constant assay of BiAb004(hG1TM) to FcyRIIb. The antibody concentrations for the curve pairs from top to bottom are 200 nM, 100 nM, 50 nM, 25 nM and 12.5 nM, respectively.

FIG. 12: Affinity constant assay of BiAb004(hG1WT) to FcγRIIb. The antibody concentrations for the curve pairs from top to bottom are 200 nM, 100 nM, 50 nM, 25 nM and 12.5 nM, respectively.

FIG. 13: Affinity constant assay of BiAb004(hG1TM) to C1q. The antibody concentrations for the curve pairs from top to bottom are 20 nM, 10 nM, 5 nM, 2.5 nM and 1.25 nM, respectively.

FIG. 14: Affinity constant assay of BiAb004(hG1WT) to C1q. The antigen concentrations for the curve pairs from top to bottom are 20 nM, 10 nM, 5 nM, 2.5 nM and 1.25 nM, respectively.

FIG. 15: Affinity constant assay of 5C10H2L2-IgG1mt to C1q. The antigen concentrations for the curve pairs from top to bottom are 20 nM, 10 nM, 5 nM, 2.5 nM and 1.25 nM, respectively.

FIG. 16: ADCC activity assay of BiAb004(hG1WT) and BiAb004(hG1TM) on 293T-CTLA4-PD1 cells expressing CTLA-4 and PD-1 antigens.

FIG. 17: Inhibition of subcutaneous human lung cancer HCC827 cell tumor xenograft by BiAb004(hG1TM) in combination with lenvatinib.

FIG. 18: Inhibition of subcutaneous colon cancer MC38-hPDL1/hCD73 tumor graft by BiAb004(hG1TM) in C57BL/6-hPD1/hPDL1/hCD73 mice.

FIG. 19: Antibody-mediated phagocytic activity of BiAb004(hG1TM) on CHO-K1-PD1.

FIG. 20: Significantly enhanced immune response of immune cells to human gastric cancer KATO III cells by BiAb004(hG1TM).

FIG. 21: Significantly enhanced immune response of immune cells to human cervical cancer Hela cells by BiAb004(hG1TM).

FIG. 22: Significantly enhanced immune response of immune cells to human T cell lymphomas Jurkat cells by BiAb004(hG1TM).

FIG. 23: Significantly enhanced immune response of immune cells to human nasopharyngeal cancer CNE-2Z cells by BiAb004(hG1TM).

FIG. 24: Significantly enhanced immune response of immune cells to human breast cancer MDA-MB-231 cells by BiAb004(hG1TM).

FIG. 25: Significantly enhanced immune response of immune cells to human mesothelioma NCI-H2452 cells by BiAb004(hG1TM).

FIG. 26: Significantly enhanced immune response of immune cells to human non-small cell lung cancer (human lung adenocarcinoma) A549 cells by BiAb004(hG1TM) in combination with anlotinib.

FIG. 27: Significantly enhanced immune response of immune cells to human small cell lung cancer NCI-H446 cells by BiAb004(hG1TM) in combination with anlotinib.

FIG. 28: Significantly enhanced immune response of immune cells to human squamous cell lung cancer NCI-H226 cells by BiAb004(hG1TM) in combination with anlotinib.

FIG. 29: Significantly enhanced immune response of immune cells to human colorectal cancer SW48 cells of MSI-H/dMMR phenotype by BiAb004(hG1TM).

FIG. 30: Significantly enhanced immune response of immune cells to human colorectal cancer SW837 cells of non-MSI-H/dMMR phenotype by BiAb004(hG1TM).

DETAILED DESCRIPTION

The embodiments of the present invention will be described in detail below with reference to the examples. Those skilled in the art will understand that the following examples are only used to illustrate the present invention, and should not be regarded as limiting the scope of the present invention. In the cases where the techniques or conditions are not specified, the examples were carried out according to the techniques or conditions described in the literature in the art (e.g., see, Molecular Cloning: A Laboratory Manual, authored by J. Sambrook et al., and translated by Huang Peitang et al., Third Edition, Science Press) or according to the product manual. Reagents or instruments used are commercially available conventional products if the manufacturers thereof are not specified.

In the following examples of the present invention:

BALB/c mice were purchased from Guangdong Medical Laboratory Animal Center.

Human peripheral blood mononuclear cells were isolated and prepared in Akeso Biopharma, Inc., with informed consent of the donor.

Raji-PDL1 is a cell expressing human PD-L1 constructed by Akeso Biopharma on the basis of human B cells Raji via transfection.

Ficoll-Paque™ PLUS (or Ficoll-Paque PLUS) was purchased from GE Healthcare.

Human IL-2 ELISA kit was purchased from Dakewe Biotech Co., Ltd.

RPMI 1640 medium, DMEM medium, Trypsin-EDTA (0.25%) phenol red and Blastidin were all purchased from Gibco.

*Staphylococcus aureus* enterotoxin B (SEB) was purchased from Dianotech.

FBS was purchased from Excell bio.

Mitomycin C (MMC) was purchased from Stressmarq.

The sequence of the isotype control, human anti-hen egg lysozyme IgG (anti-HEL antibody, or human IgG, abbreviated as hIgG) is derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1):130-146).

Anlotinib used in the examples is hydrochloride salt of anlotinib under the brand name Fukewei® and generic name anlodinib hydrochloride, and was purchased from CTTQ Pharma.

Lenvatinib used in the examples is lenvatinib mesylate under the brand name Lenvima®, and was purchased from Eisai (China).

Preparation Example 1: Sequence Design of Anti-CTLA4 Antibodies

The amino acid sequences and encoding nucleotide sequences of the heavy and light chains of the anti-CTLA4 antibody 4G10 and its humanized antibodies 4G10H1L1 and 4G10H3L3 are identical to those of 4G10, 4G10H1L1 and 4G10H3L3 in Chinese Patent Publication No. CN106967172A, respectively.

(1) Heavy and light chain variable region sequences of 4G10

Nucleotide sequence of the heavy chain variable region: (372 bp)

**CAGGTCAAGCTGCAGGAGTCTGGACCTGAGCTGGTGAAGCCTGGAGCTTCAAT GAAGATATCCTGCAAGGCTTCTGGTTACTCATTCACTGGCTACACCATGAACTGG GTGAAGCAGAGCCATGGAAAGAACCTTGAATGGATTGGACTTATTAATCCTTACA ATAATATTACTAACTACAACCAGAAGTTCATGGGCAAGGCCACATTTACTGTAGA CAAGTCATCCAGCACAGCCTACATGGAACTCCTCAGACTGACATCTGAAGACTC TGGAGTCTATTTCTGTGCAAGACTCGACTATAGGTCTTATTGGGGCCAAGGGACT CTGGTCACTGTCTCTGCAGCCAAAACGACACCCCCATCTGTCTAT (SEQ ID NO: 1)**

Encoded amino acid sequence: (124 aa)

**QVKLQESGPELVKPGASMKISCKASGYSFTGYTMNWVKQSHGKNLEWIGLINPYN NITNYNQKFMGKATFTVDKSSSTAYMELLRLTSEDSGVYFCARLDYRSYWGQGTLV TVSAAKTTPPSVY (SEQ ID NO: 2)**

Nucleotide sequence of the light chain variable region: (378 bp)

**CAGGCTGTTGTGACTCAGGAATCTGCACTCACCACATCACCTGGTGAAACAGTC ACACTCACTTGTCGCTCAAGTACTGGGGCTGTTACAACTAGTAACTTTGCCAACT GGGTCCAAGAAAAACCAGATCATTTATTCACTAGTCTAATAGGTGGTACCAACAA CCGAGCTCCAGGTGTTCCTGCCAGATTCTCAGGCTCCCTGATTGGAGACAAGGC TGCCCTCACCATCACAGGGGCACAGACTGAGGATGAGGCAATATATTTCTGTGC TCTATGGTACAGCAACCATTGGGTGTTCGGTGGAGGAACCAAACTGACTGTCCT AGGCCAGCCCAAGTCTTCGCCATCAGTCACCCTGTTTCAAGGGCAATTCTGC (SEQ ID NO: 3)**

Encoded amino acid sequence: (126 aa)

**QAVVTQESALTTSPGETVTLTCRSSTGAVTTSNFANWVQEKPDHLFTSLIGGTNNRA PGVPARFSGSLIGDKAALTITGAQTEDEAIYFCALWYSNHWVFGGGTKLTVLGQPKS SPSVTLFQGQFC (SEQ ID NO: 4)**

(2) Heavy and light chain variable region sequences of humanized monoclonal antibody 4G10H1L1

Nucleotide sequence of the heavy chain variable region (4G10H1V): (345 bp)

**CAGGTGCAGCTGGTGGAGTCTGGGGCCGAGCTGGTGAAGCCCGGCGCCTCCAT GAAGATCTCTTGCAAGGCCAGCGGATACAGTTTCACTGGCTATACCATGAACTG GGTCAAACAGGCTCCAGGACAGGGACTGGAGTGGATCGGGCTGATTAATCCTTA CAACAACATCACCAACTACAACCAGAAGTTCATGGGAAAAGCAACCTTTACAGT GGACAAGAGCATTTCCACAGCCTACATGGAACTGAGCCGGCTGACTTCAGACGA TAGCGGGGTCTATTTTTGTGCAAGGCTGGATTATCGCTCTTACTGGGGGCAGGG AACTCTGGTCACTGTCTCCGCT (SEQ ID NO: 5)**

Encoded amino acid sequence: (115 aa)

**QVQLVESGAELVKPGASMKISCKASGYSFTGYTMNWVKQAPGQGLEWIGLINPYN NITNYNQKFMGKATFTVDKSISTAYMELSRLTSDDSGVYFCARLDYRSYWGQGTLV TVSA (SEQ ID NO: 6)**

Nucleotide sequence of the light chain variable region (4G10L1V): (327 bp)

**CAGGCTGTCGTCACTCAGGAACCTTCACTGACTGTGAGCCCAGGAGGAACTGT CACCCTGACATGCGGAAGCTCCACCGGAGCAGTGACCACATCCAACTTCGCCAA TTGGGTCCAGGAAAAGCCAGGCCAGGCATTTCGATCCCTGATCGGAGGCACAAA CAATCGGGCTTCTTGGGTGCCCGCAAGATTCTCAGGAAGCCTGCTGGGGGGAA AAGCCGCTCTGACCATTAGTGGCGCTCAGCCTGAGGACGAAGCCGAGTACTTCT GCGCTCTGTGGTATAGCAACCACTGGGTGTTTGGCGGGGGAACAAAGCTGACT GTGCTG (SEQ ID NO: 7)**

Encoded amino acid sequence: (109 aa)

**QAVVTQEPSLTVSPGGTVTLTCGSSTGAVTTSNFANWVQEKPGQAFRSLIGGTNNRA SWVPARFSGSLLGGKAALTISGAQPEDEAEYFCALWYSNHWVFGGGTKLTVL (SEQ ID NO: 8)**

(3) Heavy and light chain variable region sequences of humanized monoclonal antibody 4G10H3L3

Nucleotide sequence of the heavy chain variable region (4G10H3V): (345 bp)

**CAGGTGCAGCTGGTCGAGTCTGGGGCCGAAGTGAAGAAACCCGGCGCCTCAGT GAAGGTCAGCTGCAAGGCCAGCGGGTACAGTTTCACTGGATATACCATGAACTG GGTCCGACAGGCCCCTGGCCAGGGGCTGGAGTGGATCGGCCTGATTAACCCTTA CAACAACATCACTAACTACGCACAGAAGTTCCAGGGGAGAGTGACCTTTACAGT GGACACCAGCATTTCCACAGCCTACATGGAACTGTCCCGGCTGAGATCTGACGA TACAGGCGTGTACTTCTGCGCTAGGCTGGATTACCGCAGCTATTGGGGACAGGG CACACTGGTGACTGTCAGCGCA (SEQ ID NO: 9)**

Encoded amino acid sequence (4G10H3V): (115 aa)

**QVQLVESGAEVKKPGASVKVSCKASGYSFTGYTMNWVRQAPGQGLEWIGLINPYN NITNYAQKFQGRVTFTVDTSISTAYMELSRLRSDDTGVYFCARLDYRSYWGQGTLVT VSA (SEQ ID NO: 10)**

Nucleotide sequence of the light chain variable region (4G10L3V): (327 bp)

**CAGGCTGTCGTCACTCAGGAACCTTCACTGACCGTGTCTCCTGGCGGGACTGTC ACCCTGACATGCGGCAGCTCCACAGGGGCCGTGACCACAAGTAACTTCCCAAAT TGGGTCCAGCAGAAGCCAGGACAGGCTCCCCGGAGTCTGATCGGAGGCACCAA CAACAAGGCCAGCTGGACACCCGCACGGTTCAGCGGCAGCCTGCTGGGCGGCA AGGCCGCTCTGACAATTAGCGGAGCCCAGCCTGAGGACGAAGCCGAGTACTATT GCGCTCTGTGGTACTCCAACCACTGGGTGTTCGGCGGCGGCACCAAGCTGACT GTGCTG (SEQ ID NO: 11)**

Encoded amino acid sequence (4G10L3V): (109 aa)

**QAVVTQEPSLTVSPGGTVTLTCGSSTGAVTTSNFPNWVQQKPGQAPRSLIGGTNNKA SWTPARFSGSLLGGKAALTISGAQPEDEAEYYCALWYSNHWVFGGGTKLTVL (SEQ ID NO: 12)**

Preparation Example 2: Sequence Design of Anti-PD-1 Antibody 14C12 and Its Humanized Antibody 14C12H1L1

The amino acid sequences and encoding nucleotide sequences of the heavy and light chains of anti-PD-1 antibody 14C12 and its humanized antibody 14C12H1L1 are identical to those of 14C12 and 14C12H1L1 in Chinese Patent Publication No. CN106967172A, respectively.

(1) Heavy and light chain variable region sequences of 14C12

Nucleotide sequence of the heavy chain variable region: (354 bp)

**GAGGTCAAACTGGTGGAGAGCGGCGGCGGGCTGGTGAAGCCCGGCGGGTCAC TGAAACTGAGCTGCGCCGCTTCCGGCTTCGCCTTTAGCTCCTACGACATGTCAT GGGTGAGGCAGACCCCTGAGAAGCGCCTGGAATGGGTCGCTACTATCAGCGGA GGCGGGCGATACACCTACTATCCTGACTCTGTCAAAGGGAGATTCACAATTAGTC GGGATAACGCCAGAAATACTCTGTATCTGCAGATGTCTAGTCTGCGGTCCGAGG ATACAGCTCTGTACTATTGTGCAAACCGGTACGGCGAAGCATGGTTTGCCTATTG GGGACAGGGCACCCTGGTGACAGTCTCTGCC (SEQ ID NO: 13)**

Encoded amino acid sequence: (118 aa)

**EVKLVESGGGLVKPGGSLKLSCAASGFAFSSYDMSWVRQTPEKRLEWVATISGGGR YTYYPDSVKGRFTISRDNARNTLYLQMSSLRSEDTALYYCANRYGEAWFAYWGQGT LVTVSA (SEQ ID NO: 14)**

Nucleotide sequence of the light chain variable region: (321 bp)

**GACATTAAGATGACACAGTCCCCTTCCTCAATGTACGCTAGCCTGGGCGAGCGA GTGACCTTCACATGCAAAGCATCCCAGGACATCAACACATACCTGTCTTGGTTTC AGCAGAAGCCAGGCAAAAGCCCCAAGACCCTGATCTACCGGGCCAATAGACTGG TGGACGGGGTCCCCAGCAGATTCTCCGGATCTGGCAGTGGGCAGGATTACTCCC TGACCATCAGCTCCCTGGAGTATGAAGACATGGGCATCTACTATTGCCTGCAGTA TGATGAGTTCCCTCTGACCTTTGGAGCAGGCACAAAACTGGAACTGAAG (SEQ ID NO: 15)**

Encoded amino acid sequence: (107 aa)

**DIKMTQSPSSMYASLGERVTFTCKASQDINTYLSWFQQKPGKSPKTLIYRANRLVDG VPSRFSGSGSGQDYSLTISSLEYEDMGIYYCLQYDEFPLTFGAGTKLELK (SEQ ID NO: 16)**

(2) Heavy and light chain variable region and heavy and light chain sequences of humanized monoclonal antibody 14C12H1L1

Nucleotide sequence of the heavy chain variable region: (354 bp)

**GAAGTGCAGCTGGTCGAGTCTGGGGGAGGGCTGGTGCAGCCCGGCGGGTCACTGCGACTGAGCTGCGCAGCTTCCGGATTCGCCTTTAGCTCCTACGACATGTCCTGGGTGCGACAGGCACCAGGAAAGGGACTGGATTGGGTCGCTACTATCTCAGGAGGCGGGAGATACACCTACTATCCTGACAGCGTCAAGGGCCGGTTCACAATCTCTAGAGATAACAGTAAGAACAATCTGTATCTGCAGATGAACAGCCTGAGGGCTGAGGACACCGCACTGTACTATTGTGCCAACCGCTACGGGGAAGCATGGTTTGCCTATTGGGGGCAGGGAACCCTGGTGACAGTCTCTAGT (SEQ ID NO: 17)**

Encoded amino acid sequence: (118 aa)

**EVQLVESGGGLVQPGGSLRLSCAASGFAFSSYDMSWVRQAPGKGLDWVATISGGGRYTYYPDSVKGRFTISRDNSKNNLYLQMNSLRAEDTALYYCANRYGEAWFAYWGQGTLVTVSS (SEQ ID NO: 18)**

Nucleotide sequence of the light chain variable region: (321 bp)

**GACATTCAGATGACTCAGAGCCCCTCCTCCATGTCCGCCTCTGTGGGCGACAGGGTCACCTTCACATGCCGCGCTAGTCAGGATATCAACACCTACCTGAGCTGGTTTCAGCAGAAGCCAGGGAAAGCCCCAAGACACTGATCTACCGGGCTAATAGACTGGTGTCTGGAGTCCCAAGTCGGTTCAGTGGCTCAGGGAGCGGACAGGACTACACTCTGACCATCAGCTCCCTGCAGCCTGAGGACATGGCAACCTACTATTGCCTGCAGTATGATGAGTTCCCACTGACCTTTGGCGCCGGGACAAAACTGGAGCTGAAG (SEQ ID NO: 19)**

Encoded amino acid sequence: (107 aa)

**DIQMTQSPSSMSASVGDRVTFTCRASQDINTYLSWFQQKPGKSPKTLIYRANRLVSGVPSRFSGSGSGQDYTLTISSLQPEDMATYYCLQYDEFPLTFGAGTKLELK (SEQ ID NO: 20)**

DNA sequence of 14C12H1L1 heavy chain (14C12H1): (1344 bp)

**GAAGTGCAGCTGGTCGAGTCTGGGGGAGGGCTGGTGCAGCCCGGCGGGTCACT
GCGACTGAGCTGCGCAGCTTCCGGATTCGCCTTTAGCTCCTACGACATGTCCTGG
GTGCGACAGGCACCAGGAAAGGGACTGGATTGGGTCGCTACTATCTCAGGAGGC
GGGAGATACACCTACTATCCTGACAGCGTCAAGGGCCGGTTCACAATCTCTAGAG
ATAACAGTAAGAACAATCTGTATCTGCAGATGAACAGCCTGAGGGCTGAGGACAC
CGCACTGTACTATTGTGCCAACCGCTACGGGGAAGCATGGTTTGCCTATTGGGGG
CAGGGAACCCTGGTGACAGTCTCTAGTGCCAGCACCAAAGGACCTAGCGTGTTT
CCTCTCGCCCCCTCCTCCAAAAGCACCAGCGGAGGAACCGCTGCTCTCGGATGT
CTGGTGAAGGACTACTTCCCTGAACCCGTCACCGTGAGCTGGAATAGCGGCGCT
CTGACAAGCGGAGTCCATACATTCCCTGCTGTGCTGCAAAGCAGCGGACTCTATT
CCCTGTCCAGCGTCGTCACAGTGCCCAGCAGCAGCCTGGGCACCCAGACCTACA
TCTGTAACGTCAACCACAAGCCCTCCAACACCAAGGTGGACAAGAAAGTGGAGC
CCAAATCCTGCGACAAGACACACACCTGTCCCCCCTGTCCTGCTCCCGAACTCCT
CGGAGGCCCTAGCGTCTTCCTCTTTCCTCCCAAACCCAAGGACACCCTCATGATC
AGCAGAACCCCTGAAGTCACCTGTGTCGTCGTGGATGTCAGCCATGAGGACCCC
GAGGTGAAATTCAACTGGTATGTCGATGGCGTCGAGGTGCACAACGCCAAAACC
AAGCCCAGGGAGGAACAGTACAACTCCACCTACAGGGTGGTGTCCGTGCTGACA
GTCCTCCACCAGGACTGGCTGAACGGCAAGGAGTACAAGTGCAAGGTGTCCAAC
AAGGCTCTCCCTGCCCCCATTGAGAAGACCATCAGCAAGGCCAAAGGCCAACCC
AGGGAGCCCCAGGTCTATACACTGCCTCCCTCCAGGGACGAACTCACCAAGAAC
CAGGTGTCCCTGACCTGCCTGGTCAAGGGCTTTTATCCCAGCGACATCGCCGTCG
AGTGGGAGTCCAACGGACAGCCCGAGAATAACTACAAGACCACCCCTCCTGTCC
TCGACTCCGACGGCTCCTTCTTCCTGTACAGCAAGCTGACCGTGGACAAAAGCA
GGTGGCAGCAGGGAAACGTGTTCTCCTGCAGCGTGATGCACGAAGCCCTCCACA
ACCACTACACCCAGAAAAGCCTGTCCCTGAGCCCCGGCAAA (SEQ ID NO: 21)**

Encoded amino acid sequence: (448 aa)

**EVQLVESGGGLVQPGGSLRLSCAASGFAFSSYDMSWVRQAPGKGLDWVATISGGGRY
TYYPDSVKGRFTISRDNSKNNLYLQMNSLRAEDTALYYCANRYGEAWFAYWGQGTLV
TVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK (SEQ ID NO: 22)**

DNA sequence of 14C12H1L1 light chain (14C12L1): (642 bp)

GACATTCAGATGACTCAGAGCCCCTCCTCCATGTCCGCCTCTGTGGGCGACAGG GTCACCTTCACATGCCGCGCTAGTCAGGATATCAACACCTACCTGAGCTGGTTTC AGCAGAAGCCAGGGAAAAGCCCCAAGACACTGATCTACCGGGCTAATAGACTGG

TGTCTGGAGTCCCAAGTCGGTTCAGTGGCTCAGGGAGCGGACAGGACTACACT CTGACCATCAGCTCCCTGCAGCCTGAGGACATGGCAACCTACTATTGCCTGCAG TATGATGAGTTCCCACTGACCTTTGGCGCCGGGACAAAACTGGAGCTGAAGCGA ACTGTGGCCGCTCCCTCCGTCTTCATTTTTCCCCCTTCTGACGAACAGCTGAAAT CAGGCACAGCCAGCGTGGTCTGTCTGCTGAACAATTTCTACCCTAGAGAGGCAA AAGTGCAGTGGAAGGTCGATAACGCCCTGCAGTCCGGCAACAGCCAGGAGAGT GTGACTGAACAGGACTCAAAAGATAGCACCTATTCCCTGTCTAGTACACTGACTC TGTCCAAGGCTGATTACGAGAAGCACAAAGTGTATGCATGCGAAGTGACACATC AGGGACTGTCAAGCCCCGTGACTAAGTCTTTTAACCGGGGCGAATGT (SEQ ID NO: 23)

Encoded amino acid sequence: (214 aa)

DIQMTQSPSSMSASVGDRVTFTCRASQDINTYLSWFQQKPGKSPKTLIYRANRLVSG VPSRFSGSGSGQDYTLTISSLQPEDMATYYCLQYDEFPLTFGAGTKLELKRTVAAPSV FIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC (SEQ ID NO: 24)

Preparation Example 3: Sequence Design of Bifunctional Antibodies BiAb001(M), BiAb002(M), BiAb003(M) and BiAb004(M)

The structural patterns of the bifunctional antibodies BiAb001(M), BiAb002(M), BiAb003(M) and BiAb004(M) are in the Morrison format (IgG-scFv), i.e., C termini of two heavy chains of one IgG antibody are separately linked to the scFv fragment of another antibody via linker fragments. The components for the heavy and light chain design are shown in Table A below.

Table A: Sequence design of BiAb001(M), BiAb002(M), BiAb003(M) and BiAb004(M)

| Bifunctional antibody | Immunoglobulin moiety | | Linker fragment | scFv part |
|---|---|---|---|---|
| | Heavy chain | Light chain | | |
| BiAb001(M) | 14C12H1 | 14C12L1 | Linker1 | 4G10H1V(M)- Linker2 -4G10L1V (M) |
| BiAb002(M) | 14C12H1 | 14C12L1 | Linker2 | 4G10H1V(M)- Linker2 -4G10L1V (M) |
| BiAb003(M) | 14C12H1 | 14C12L1 | Linker1 | 4G10H3V(M)- Linker2 -4G10L3V (M) |
| BiAb004(M) | 14C12H1 | 14C12L1 | Linker2 | 4G10H3V(M)- Linker2 -4G10L3V (M) |

In Table A above:

the amino acid sequence of Linker 1 is $(GGGGS)_3$ (SEQ ID NO: 25), and

the amino acid sequence of Linker 2 is $(GGGGS)_4$ (SEQ ID NO: 26).

In Table A above, scFv fragments 4G10H1V(M), 4G10L1V(M), 4G10H3V(M) and 4G10L3V(M) of BiAb001(M), BiAb002(M), BiAb003(M) and BiAb004(M) antibodies comprised mutations in individual amino acids of framework regions on the basis of 4G10H1V, 4G10L1V, 4G10H3V and 4G10L3V, respectively, which effectively optimized the structure of the antibodies and improved their efficacy.

(1) 4G10H1V(M): (115 aa, mutation positions underlined in the amino acid sequence based on 4G10H1V)

**QVQLVESGAELVKPGASMKISCKASGYSFTGYTMNWVKQAPGQCLEWIGLINPYNNITNYNQKFMGKATFTVDKSISTAYMELSRLTSDDSGVYFCARLDYRSYWGQGTLVTVSA (SEQ ID NO: 41)**

(2) 4G10L1V(M): (110 aa, mutation positions underlined in the amino acid sequence based on 4G10L1V)

**QAVVTQEPSLTVSPGGTVTLTCGSSTGAVTTSNFANWVQEKPGQAFRSLIGGTNNRASWVPARFSGSLLGGKAALTISGAQPEDEAEYFCALWYSNHWVFGCGTKLTVLR (SEQ ID NO: 42)**

(3) 4G10H3V(M): (115 aa, mutation positions underlined in the amino acid sequence based on 4G10H3V)

**QVQLVESGAEVKKPGASVKVSCKASGYSFTGYTMNWVRQAPGQCLEWIGLINPYNNITNYAQKFQGRVTFTVDTSISTAYMELSRLRSDDTGVYFCARLDYRSYWGQGTLVTVSA (SEQ ID NO: 43)**

(4) 4G10L3V(M): (110 aa, mutation positions underlined in the amino acid sequence based on 4G10L3V)

**QAVVTQEPSLTVSPGGTVTLTCGSSTGAVTTSNFPNWVQQKPGQAPRSLIGGTNNKASWTPARFSGSLLGGKAALTISGAQPEDEAEYYCALWYSNHWVFGCGTKLTVLR (SEQ ID NO: 44)**

For distinguishment from the mutated antibodies hereinafter, BiAb004(M) is also referred to as BiAb004(hG1WT) in the this example. BiAb004(M) described above is the "wild-type", comprising an Ig gamma-1 chain C region (ACCESSION: P01857) as the heavy chain constant region and an Ig kappa chain C region (ACCESSION: P01834) as the light chain constant region.

Preparation Example 4: Non-Variable Region Amino Acid Mutation Design Based on Humanized Bifunctional Antibody BiAb004

On the basis of BiAb004(hG1WT) obtained in Preparation Example 3, BiAb004(hG1TM) was obtained by introducing a leucine-to-alanine point mutation at position 234 (L234A), a leucine-to-alanine point mutation at position 235 (L235A), and a glycine-to-alanine point mutation at position 237 (G237A) in the heavy chain.

DNA sequence of heavy chain of the immunoglobulin moiety in BiAb004(hG1TM): (1344 bp, mutation positions underlined)

**GAAGTGCAGCTGGTCGAGTCTGGGGGAGGGCTGGTGCAGCCCGGCGGGTCACTGCGACTGAGCTGCGCAGCTTCCGGATTCGCCTTTAGCTCCTACGACATGTCCTGGGTGCGACAGGCACCAGGAAAGGGACTGGATTGGGTCGCTACTATCTCAGGAGGCGGGAGATACACCTACTATCCTGACAGCGTCAAGGGCCGGTTCACAATCTCTAGAGATAACAGTAAGAACAATCTGTATCTGCAGATGAACAGCCTGAGGGCTGAGGACACCGCACTGTACTATTGTGCCAACCGCTACGGGGAAGCATGGTTTGCCTATTGGGGGCAGGGAACCCTGGTGACAGTCTCTAGTGCCAGCACCAAAGGGCCCAGCGTGTTTCCTCTCGCCCCCTCCTCCAAAAGCACCAGCGGAGGAACCGCTGCTCTCGGATGTCTGGTGAAGGACTACTTCCCTGAACCCGTCACCGTGAGCTGGAATAGCGGCGCTCTGACAAGCGGAGTCCATACATTCCCTGCTGTGCTGCAAAGCAGCGGACTCTATTCCCTGTCCAGCGTCGTCACAGTGCCCAGCAGCAGCCTGGGCACCCAGACCTACATCTGTAACGTCAACCACAAGCCCTCCAACACCAAGGTGGACAAGAAAGTGGAGCCCAAATCCTGCGACAAGACACACACCTGTCCCCCCTGTCCTGCTCCCGAAGCTGCTGGAGCCCCTAGCGTCTTCCTCTTTCCTCCCAAACCCAAGGACACCCTCATGATCAGCAGAACCCCTGAAGTCACCTGTGTCGTCGTGGATGTCAGCCATGAGGACCCCGAGGTGAAATTCAACTGGTATGTCGATGGCGTCGAGGTGCACAACGCCAAAACCAAGCCCAGGGAGGAACAGTACAACTCCACCTACAGGGTGGTGTCCGTGCTGACAGTCCTCCACCAGGACTGGCTGAACGGCAAGGAGTACAAGTGCAAGGTGTCCAACAAGGCTCTCCCTGCCCCCATTGAGAAGACCATCAGCAAGGCCAAAGGCCAACCCAGGGAGCCCCAGGTCTATACACTGCCTCCCTCCAGGGACGAACTCACCAAGAACCAGGTGTCCCTGACCTGCCTGGTCAAGGGCTTTTATCCCAGCGACATCGCCGTCGAGTGGGAGTCCAACGGACAGCCCGAGAATAACTACAAGACCACCCCTCCTGTCCTCGACTCCGACGGCTCCTTCTTCCTGTACAGCAAGCTGACCGTGGACAAAAGCAGGTGGCAGCAGGGAAACGTGTTCTCCTGCAGCGTGATGCACGAAGCCCTCCACAACCACTACACCCAGAAAAGCCTGTCCCTGAGCCCCGGCAAA (SEQ ID NO: 39)**

Amino acid sequence of heavy chain of the immunoglobulin moiety in BiAb004(hG1TM): (448 aa, mutation positions underlined)

**EVQLVESGGGLVQPGGSLRLSCAASGFAFSSYDMSWVRQAPGKGLDWVATISGGGRYTYYPDSVKGRFTISRDNSKNNLYLQMNSLRAEDTALYYCANRYGEAWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGAPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO: 40)**

BiAb004(hG1TM)and BiAb004(hG1WT) share the same DNA sequence of light chain and the same encoded amino acid sequence. The specific sequence is shown in Preparation Example 3.

Experimental Example 1: Affinity Assay of BiAb004(hG1WT) and BiAb004(hG1TM) to Receptor FcyRI

The Fc receptor FcyRI, also known as CD64, can bind to the Fc fragment of IgG antibodies and is involved in antibody-dependent cell-mediated cytotoxicity (ADCC). The binding capacity of a therapeutic monoclonal antibody to Fc receptors will influence the safety and efficacy of the antibody.
The affinity constants of BiAb004(hG1WT) and BiAb004(hG1TM) to FcyRI were measured in this experiment using a Fortebio Octet system to evaluate the potential ADCC and ADCP activities of the antibodies.
The method for determining the affinity constant of the antibodies to FcyRI by the Fortebio Octet system is briefly described as follows: the sample dilution buffer was a solution of PBS, 0.02% Tween-20 and 0.1% BSA, pH 7.4. A 1 μg/mL FcyRI solution (from Sinobio) was added to the HIS1K sensor to immobilize the FcyRI on the sensor surface for 50 s. The association and dissociation constants of the antibodies to FcyRI were both determined in the buffer with the antibody concentrations being 3.12-50 nM (serial two-fold dilution). The sensor with immobilized antigen was equilibrated in the buffer for 60 s, and then the binding of the immobilized FcyRI on the sensor to the antibodies was determined for 120 s; the dissociation of FcyRI from the antibodies was determined in 120 s. The temperature was 30 °C and the frequency was 0.3 Hz. The data were fitted and analyzed with a 1:1 model to obtain the affinity constants to FcyRI for the antibodies.
The results of affinity constant assay of BiAb004(hG1TM) and BiAb004(hG1WT) to FcyRI are shown in Table 1 and FIGs. 1 and 2 below.

Table 1: Kinetics for binding of BiAb004(hG1WT) and BiAb004(hG1TM) to FcyRI

| Sample ID | $K_D$ (M) | kon (1/Ms) | S E (kon) | kdis (1/s) | S E (kdis) | Rmax (nm) |
|---|---|---|---|---|---|---|
| **BiAb004 (hG1TM)** | **N/A** | **N/A** | **N/A** | **N/A** | **N/A** | **N/A** |
| **BiAb004 (hG1WT)** | **5.92E-09** | **3.06E+05** | **8.35E+03** | **1.81E-03** | **5.75E-05** | **0.53-0.62** |

The results showed that BiAb004(hG1WT) bound to FcyRI with an affinity constant of 5.92E-09 M; while for BiAb004(hG1TM), no corresponding data was obtained as no binding signal or an extremely weak signal to FcyRI was detected, indicating no binding to FcyRI.
The results suggest that the binding activity of BiAb004(hG1TM) to FcyRI is effectively eliminated as compared to BiAb004(hG1WT).

Experimental Example 2: Affinity Constant Assay of BiAb004(hG1WT) and BiAb004(hG1TM) to FcγRIIIa V158

The Fc receptor FcγRIIIa_V158 (also known as CD16a_V158), can bind to the Fc fragment of IgG antibodies and mediate ADCC effects.
The affinity constants of BiAb004(hG1WT) and BiAb004(hG1TM) to FcγRIIIa_V158 were measured in this experiment using a Fortebio Octet system to evaluate the ADCC activity of the antibodies.
The method for determining the affinity constant of the antibodies to FcγRIIIa_V158 by the Fortebio Octet system is briefly described as follows: the sample dilution buffer was a solution of PBS, 0.02% Tween-20 and 0.1% BSA, pH 7.4. 5 μg/mL FcγRIIIa_V158 was immobilized on the HIS1K sensor for 120 s. The sensor was equilibrated in a buffer for 60 s, and the binding of the immobilized FcγRIIIa_V158 on the sensor to the antibodies at concentrations of 31.25-500 nM (serial two-fold dilution) was determined for 60 s. The antibody was dissociated in the buffer for 60 s. The sensor was refreshed 4 times in 10 mM glycine pH 1.5, each for 5 s. The temperature was 30 °C and the frequency was 0.3 Hz. The data were analyzed by 1:1 model fitting to obtain affinity constants.
The results of affinity constant assay of BiAb004(hG1TM) and BiAb004(hG1WT) to FcγRIIIa_V158 are shown in Table 2 and FIGs. 3 and 4 below.

Table 2: Kinetics for binding of BiAb004(hG1WT) and BiAb004(hG1TM) to FcγRIIIa_V158

| Sample ID | $K_D$ (M) | kon (1/Ms) | S E (kon) | kdis (1/s) | S E (kdis) | Rmax (nm) |
|---|---|---|---|---|---|---|
| **BiAb004 (hG1TM)** | **N/A** | **N/A** | **N/A** | **N/A** | **N/A** | **N/A** |
| **BiAb004 (hG1WT)** | **1.77E-07** | **1.21E+05** | **1.64E+04** | **2.14E-02** | **3.71E-03** | **1.56-1.61** |

The results showed that BiAb004(hG1WT) bound to FcγRIIIa_V158 with an affinity constant of 1.77E-07 M; while for BiAb004(hG1TM), no corresponding data was obtained as no binding signal or an extremely weak signal to FcγRIIIa_V158 was detected, indicating no binding to FcγRIIIa_V158.

The results suggest that the binding activity of BiAb004(hG1TM) to FcγRIIIa_V158 is effectively eliminated as compared to BiAb004(hG1WT).

Experimental Example 3: Affinity Constant Assay of BiAb004(hG1WT) and BiAb004(hG1TM) to FcγRIIIa F158

The Fc receptor FcγRIIIa_F158 (also known as CD16a_F158), can bind to the Fc fragment of IgG antibodies and mediate ADCC effects.

The affinity constants of BiAb004(hG1WT) and BiAb004(hG1TM) to FcγRIIIa_F158 were measured in this experiment using a Fortebio Octet system to evaluate the ADCC activity of the antibodies.

The method for determining the affinity constant of BiAb004(hG1WT) and BiAb004(hG1TM) to FcγRIIIa_F158 by the Fortebio Octet system is briefly described as follows: the sample dilution buffer was a solution of PBS, 0.02% Tween-20 and 0.1% BSA, pH 7.4. 5 μg/mL FcγRIIIa_F158 was immobilized on the HIS1K sensor for 120 s. The sensor was equilibrated in a buffer for 60 s, and the binding of the immobilized FcγRIIIa_F158 on the sensor to the antibodies at concentrations of 31.25-500 nM (serial two-fold dilution) was determined for 60 s. The antibody was dissociated in the buffer for 60 s. The sensor was refreshed 4 times in 10 mM glycine pH 1.5, each for 5 s. The temperature was 30 °C and the frequency was 0.3 Hz. The data were analyzed by 1:1 model fitting to obtain affinity constants.

The results of affinity constant assay of BiAb004(hG1TM) and BiAb004(hG1WT) to FcγRIIIa_F158 are shown in Table 3 and FIGs. 5 and 6 below.

Table 3: Kinetics for binding of BiAb004(hG1WT) and BiAb004(hG1TM) to FcγRIIIa_F158

| Sample ID | $K_D$ (M) | kon (1/Ms) | S E (kon) | kdis (1/s) | S E (kdis) | Rmax (nm) |
|---|---|---|---|---|---|---|
| BiAb004 (hG1TM) | N/A | N/A | N/A | N/A | N/A | N/A |
| BiAb004 (hG1WT) | 2.21E-07 | 1.12E+05 | 1.39E+04 | 2.47E-02 | 3.43E-03 | 0.39-0.64 |

The results showed that BiAb004(hG1WT) bound to FcγRIIIa_F158 with an affinity constant of 2.21E-07 M; while for BiAb004(hG1TM), no corresponding data was obtained as no binding signal or an extremely weak signal to FcγRIIIa_F158 was detected, indicating no binding to FcγRIIIa_F158.

The results suggest that the binding activity of BiAb004(hG1TM) to FcγRIIIa_F158 is effectively eliminated as compared to BiAb004(hG1WT).

Experimental Example 4: Affinity Constant Assay of BiAb004(hG1WT) and BiAb004(hG1TM) to FcγRIIa H131

The Fc receptor FcγRIIa_H131 (also known as CD32a_H131), can bind to the Fc fragment of IgG antibodies and mediate ADCC effects.

The affinity constants of BiAb004(hG1WT) and BiAb004(hG1TM) to FcγRIIa_H131 were measured in this experiment using a Fortebio Octet system to evaluate the ADCC activity of the antibodies.

The method for determining the affinity constant of BiAb004(hG1WT) and BiAb004(hG1TM) to FcyRIIa_H131 by the Fortebio Octet system is briefly described as follows: the immobilization dilution buffer was a solution of PBS, 0.02% Tween-20 and 0.1% BSA, pH 7.4, and the analyte dilution buffer was a solution of PBS, 0.02% Tween-20, 0.02% casein and 0.1% BSA, pH 7.4. 5 μg/mL FcγRIIa_H131 was immobilized on the NTA sensor at an immobilization height of about 1.0 nm. The sensor was equilibrated in a buffer of PBS, 0.02% Tween-20, 0.02% casein and 0.1% BSA, pH 7.4 for 300 s of blocking, and the binding of the immobilized FcγRIIa_H131 on the sensor to the antibodies at concentrations of 12.5-200 nM (serial two-fold dilution) was determined for 60 s. The antibody was dissociated in the buffer for 60 s. The sensor was refreshed in 10 mM glycine pH 1.7 and 10 nM nickel sulfate. The temperature was 30 °C and the frequency was 0.6 Hz. The data were analyzed by 1:1 model fitting to obtain affinity constants.

The results of affinity constant assay of BiAb004(hG1TM) and BiAb004(hG1WT) to FcγRIIa_H131 are shown in Table 4 and FIGs. 7 and 8 below.

Table 4: Kinetics for binding of BiAb004(hG1WT) and BiAb004(hG1TM) to FcγRIIa_H131

| Sample ID | $K_D$ (M) | kon (1/Ms) | S E (kon) | kdis (1/s) | S E (kdis) | Rmax (nm) |
|---|---|---|---|---|---|---|
| BiAb004 (hG1TM) | N/A | N/A | N/A | N/A | N/A | N/A |
| BiAb004 (hG1WT) | 2.22E-08 | 3.83E+05 | 4.03E+04 | 8.49E-03 | 7.44E-04 | 0.96-1.63 |

The results showed that BiAb004(hG1WT) bound to FcγRIIa_H131 with an affinity constant of 2.22E-08 M;

while for BiAb004(hG1TM), no corresponding data was obtained as no binding signal or an extremely weak signal to FcγRIIa_H131 was detected, indicating no binding to FcγRIIa_H131.

The results suggest that the binding activity of BiAb004(hG1TM) to FcγRIIa_H131 is effectively eliminated as compared to BiAb004(hG1WT).

Experimental Example 5: Affinity Constant Assay of BiAb004(hG1WT) and BiAb004(hG1TM) to FcγRIIa R131

The Fc receptor FcγIIa_R131 (also known as CD32a_R131), can bind to the Fc fragment of IgG antibodies and mediate ADCC effects.

The affinity constants of BiAb004(hG1WT) and BiAb004(hG1TM) to FcγIIa_R131 were measured in this experiment using a Fortebio Octet system to evaluate the ADCC activity of the antibodies.

The method for determining the affinity constant of BiAb004(hG1WT) and BiAb004(hG1TM) by the Fortebio Octet system is briefly described as follows: the immobilization dilution buffer was a solution of PBS, 0.02% Tween-20 and 0.1% BSA, pH 7.4, and the analyte dilution buffer was a solution of PBS, 0.02% Tween-20, 0.02% casein and 0.1% BSA, pH 7.4. 5 μg/mL FcγIIa_R131 was immobilized on the NTA sensor at an immobilization height of about 1.0 nm. The sensor was equilibrated in a buffer of PBS, 0.02% Tween-20, 0.02% casein and 0.1% BSA, pH 7.4 for 300 s of blocking, and the binding of the immobilized FcγIIa_R131 on the sensor to the antibodies at concentrations of 12.5-200 nM (serial two-fold dilution) was determined for 60 s. The antibody was dissociated in the buffer for 60 s. The sensor was refreshed in 10 mM glycine pH 1.7 and 10 nM nickel sulfate. The temperature was 30 °C and the frequency was 0.6 Hz. The data were analyzed by 1:1 model fitting to obtain affinity constants.

The results of affinity constant assay of BiAb004(hG1TM) and BiAb004(hG1WT) to FcγIIa_R131 are shown in Table 5 and FIGs. 9 and 10 below.

Table 5: Kinetics for binding of BiAb004(hG1WT) and BiAb004(hG1TM) to FcγIIa_R131

| Sample ID | $K_D$ (M) | kon (1/Ms) | S E (kon) | kdis (1/s) | S E (kdis) | Rmax (nm) |
|---|---|---|---|---|---|---|
| **BiAb004 (hG1TM)** | **4.20E-08** | **3.72E+05** | **4.19E+04** | **1.56E-02** | **8.99E-04** | **0.16-0.36** |
| **BiAb004 (hG1WT)** | **1.43E-08** | **3.58E+05** | **3.13E+04** | **5.10E-03** | **5.87E-04** | **0.66-1.34** |

The results showed that both BiAb004(hG1WT) and BiAb004(hG1TM) bound to FcγIIa_R131 with affinity constants of 1.43E-08 M and 4.20E-08 M, respectively.

The results suggest that both BiAb004(hG1WT) and BiAb004(hG1TM) have binding activity to FcγRIIa_R131. However, the results in FIGs. 9 and 10 showed that BiAb004(hG1WT) had a stronger binding signal and thus a higher affinity than BiAb004(hG1TM).

Experimental Example 6: Affinity Constant Assay of BiAb004(hG1WT) and BiAb004(hG1TM) to FcγRIIb

The Fc receptor FcyRIIb (also known as CD32b), can bind to the Fc fragment of IgG antibodies, down-regulate functions of immune cells, inhibit the activation and proliferation of immune cells and inhibit the secretion of cytokines.

The affinity constants of BiAb004(hG1WT) and BiAb004(hG1TM) to FcyRIIb were measured in this experiment using a Fortebio Octet system to evaluate binding capacity of BiAb004(hG1WT) and BiAb004(hG1TM) to the Fc receptor.

The method for determining the affinity constant of BiAb004(hG1WT) and BiAb004(hG1TM) to FcyRIIb by the Fortebio Octet system is briefly described as follows: the immobilization dilution buffer was a solution of PBS, 0.02% Tween-20 and 0.1% BSA, pH 7.4, and the analyte dilution buffer was a solution of PBS, 0.02% Tween-20, 0.02% casein and 0.1% BSA, pH 7.4. 5 μg/mL FcyRIIb was immobilized on the NTA sensor at an immobilization height of about 1.0 nm. The sensor was equilibrated in a buffer of PBS, 0.02% Tween-20, 0.02% casein and 0.1% BSA, pH 7.4 for 300 s of blocking, and the binding of the immobilized FcyRIIb on the sensor to the antibodies at concentrations of 12.5-200 nM (serial two-fold dilution) was determined for 60 s. The antibody was dissociated in the buffer for 60 s. The sensor was refreshed in 10 mM glycine pH 1.7 and 10 nM nickel sulfate. The temperature was 30 °C and the frequency was 0.6 Hz. The data were analyzed by 1:1 model fitting to obtain affinity constants.

The results of affinity constant assay of BiAb004(hG1TM) and BiAb004(hG1WT) to FcyRIIb are shown in Table 6 and FIGs. 11 and 12 below.

Table 6: Kinetics for binding of BiAb004(hG1WT) and BiAb004(hG1TM) to FcyRIIb

| Sample ID | $K_D$ (M) | kon (1/Ms) | S E (kon) | kdis (1/s) | S E (kdis) | Rmax (nm) |
|---|---|---|---|---|---|---|
| **BiAb004 (hG1TM)** | **N/A** | **N/A** | **N/A** | **N/A** | **N/A** | **N/A** |

(continued)

| Sample ID | $K_D$ (M) | kon (1/Ms) | S E (kon) | kdis (1/s) | S E (kdis) | Rmax (nm) |
|---|---|---|---|---|---|---|
| BiAb004 (hG1WT) | 5.61E-08 | 2.07E+05 | 1.54E+04 | 1.16E-02 | 4.33E-04 | 0.68-0.83 |

The results showed that BiAb004(hG1WT) bound to FcγRIIb with an affinity constant of 5.61E-08 M; while for BiAb004(hG1TM), no corresponding data was obtained as no binding signal or an extremely weak signal to FcγRIIb was detected, indicating no binding to FcγRIIb.

The results suggest that the binding activity of BiAb004(hG1TM) to FcγRIIb is effectively eliminated as compared to BiAb004(hG1WT).

Experimental Example 7: Affinity Assay of BiAb004(hG1WT) and BiAb004(hG1TM) to C1q

Serum complement C1q can bind to the Fc fragment of IgG antibodies and mediate CDC effects. The binding capacity of a therapeutic monoclonal antibody to C1q will influence the safety and efficacy of the antibody.

The affinity constants of BiAb004(hG1WT) and BiAb004(hG1TM) to C1q were measured in this experiment using a Fortebio Octet system to evaluate the CDC activity of the antibodies. In this experimental example, an anti-PDL1 antibody 5C10H2L2-IgG1mt was used as a control, of which the preparation is described in PCT Publication No. WO2017148424A1.

The method for determining the affinity constants of the antibodies to C1q by the Fortebio Octet system is briefly described as follows: the sample dilution buffer was a solution of PBS, 0.02% Tween-20 and 0.1% BSA, pH 7.4. 50 μg/mL antibody was immobilized on the FAB2G sensor at an immobilization height of about 2.0 nm. The sensor was equilibrated in a buffer for 60 s for blocking, and the binding of the immobilized antibody on the sensor to the antigen C1q at concentrations of 1.25-20 nM (serial two-fold dilution) was determined for 60 s. The antigen and antibody were dissociated in the buffer for 60 s. The sensor was refreshed 4 times in 10 mM glycine pH 1.7, each for 5 s. The shaking speed of the sample plate was 1000 rpm, the temperature was 30 °C and the frequency was 0.6 Hz. The data were analyzed by 1:1 model fitting to obtain affinity constants. The data acquisition software was Fortebio Data Acquisition 7.0, and the data analysis software was Fortebio Data Analysis 7.0.

The results of affinity constant assay of BiAb004(hG1TM), BiAb004(hG1WT) and 5C10H2L2-IgG1mt to C1q are shown in Table 7 and FIGs. 13, 14 and 15 below.

Table 7: Kinetics for binding of BiAb004(hG1WT), BiAb004(hG1TM) and 5C10H2L2-IgG1mt to C1q

| Sample ID | $K_D$ (M) | kon (1/Ms) | S E (kon) | kdis (1/s) | S E (kdis) | Rmax (nm) |
|---|---|---|---|---|---|---|
| BiAb004 (hG1TM) | N/A | N/A | N/A | N/A | N/A | N/A |
| BiAb004 (hG1WT) | 2.53E-09 | 2.05E+06 | 2.10E+05 | 5.17E-03 | 5.81E-04 | 0.05-0.23 |
| 5C10H2L2-IgG1mt | 4.43E-09 | 2.38E+06 | 4.21E+05 | 1.05E-02 | 1.10E-03 | 0.19-0.26 |

The results showed that BiAb004(hG1WT) bound to C1q with an affinity constant of 2.53E-09 M; while for BiAb004(hG1TM), no corresponding data was obtained as no binding signal or an extremely weak signal to C1q was detected, indicating no binding to C1q.

The results suggest that the binding activity of BiAb004(hG1TM) to C1q is effectively eliminated as compared to BiAb004(hG1WT).

Experimental Example 8: ADCC Activity Assay of BiAb004(hG1WT) and BiAb004(hG1TM) on 293T-CTLA4-PD1 Cells Expressing CTLA4 and PD-1 Antigens

The ADCC effect refers to that effector immune cells with killing activity recognize Fc fragments of antibodies bound to antigens of target cells through Fc receptors (FcR) expressed on their surfaces, and directly kill the target cells. To test the ADCC effect of the anti-CTLA4/anti-PD-1 bifunctional antibodies BiAb004(hG1WT) and BiAb004(hG1TM), the inventors constructed a co-culture system of 293T-CTLA4-PD1 cells expressing PD-1 and CTLA4 antigens and primary PBMCs to test the ADCC activity of the antibodies.

The method for ADCC activity assay of BiAb004(hG1WT) and BiAb004(hG1TM) on 293T-CTLA4-PD1 cells expressing CTLA4 and PD-1 antigens is as follows:

Normal human PBMCs were isolated according to the Ficoll peripheral blood mononuclear cell isolation instruction. The isolated PBMCs were resuspended in RPMI-1640 complete medium, stained with AO/PI, counted and frozen. One day before the experiment, PBMCs were thawed and counted, and the viability was determined. The cells were incubated overnight in a carbon dioxide incubator at 5% $CO_2$ and 37 °C. On the day of the experiment, 293T-CTLA4-PD1 cells

and PBMCs were collected, centrifuged at 800 rpm or 1200 rpm for 5 min, resuspended in RPMI-1640 (containing 1% FBS; hereinafter referred to as the medium) and washed twice. The cells were counted and viability was determined.

The cell density was adjusted to a proper range using the medium. 100 μL of 293T-CTLA4-PD1 cells was added to a 96-well plate at a density of 3.0E+04 cells/well according to the experimental design. 50 μL of antibody was added, and the cells were pre-incubated for 1 h at room temperature; after 1 h, 50 μL of PBMCs were added at 9.0E+05 cells/well and the mixture was mixed well, incubated at 37 °C for 4 h in a carbon dioxide incubator at 5% $CO_2$. The cells were centrifuged at 250x g for 5 min. 100 μL of supernatant was transferred to a new 96-well flat-bottom microplate (do not pipette the cell precipitate). 100 μL of freshly prepared reaction solution was added to each well according to the Cytotoxicity Detection Kit instruction. The cells were incubated for 30 min at room temperature in the dark. OD values at 490 nm and 650 nm were measured. ADCC percentage was calculated for each group according to the formula ADCC(%) = (treatment group - negative control group)/(maximum LDH release in target cell - spontaneous LDH release in target cell) ×100%.

The ADCC activity of BiAb004(hG1WT) and BiAb004(hG1TM) on 293T-CTLA4-PD1 cells expressing CTLA4 and PD-1 antigens was expressed as ADCC percentages, and the results are shown in FIG. 16.

The results showed that in the mixed culture system of PBMC and 293T-CTLA4-PD1, the ADCC percentage induced by BiAb004(hG1TM) was significantly lower than that induced by BiAb004(hG1WT) at the same level.

The results suggest that BiAb004(hG1TM) has no ADCC activity.

Experimental Example 9: Growth Inhibition of Colorectal Cancer MC38 Cell Graft in Mice by BiAb004(hG1TM) in Combination with Lenvatinib

The mouse MC38 cell line is a mouse colorectal cancer cell line. It has been demonstrated that the MC38 cells line is a useful model for studying human MSI-H/dMMR tumors (Efremova M et al., Nat Commun., 2018; 9(1):32).

MC-38 cells (purchased from Shanghai Ruilu Biotech Co., Ltd.) were collected, adjusted to a density of 5 million cells/mL, and subcutaneously grafted into PD-1 transgenic mice (purchased from Shanghai Model Organisms Center, Inc.) in a volume of 200 μL/mouse at the right flank. 12 mice were grafted, each bearing $1 \times 10^6$ cells. When the tumor volume reached about 60-150 $mm^3$, the mice were divided by tumor volume (with similar average volume of approximately 94 $mm^3$) into 2 groups, model group and BiAb004(hG1TM)/lenvatinib treatment group, each containing 6 mice. The day of grouping was set as D0. 6 doses of antibody were intraperitoneally administered on D0, D4, D7, D10, D13 and D17, respectively; lenvatinib was administered daily by oral gavage for 20 days. Tumor volume was measured by vernier caliper. Tumor dimensions were measured twice weekly after grouping using vernier caliper, and tumor volume was calculated according to the formula $TV = 0.5 \times ab^2$, wherein a is the longest diameter of the tumor, b is the shortest diameter of the tumor, and TV is the volume of the tumor. The specific protocol is shown in Table 8.

Table 8: Study design

| Group | Number of animals | Model establishment | Regimen |
|---|---|---|---|
| **Model group** | **6** | **MC-38 cells were grafted subcutaneously at a dose of 1 million cells/200 μL/mouse** | **Anti-HEL antibody (i.e., isotype control antibody) at 1 mg/kg was injected intraperitoneally twice weekly in a total of 6 doses** |
| **BiAb004 (hG1TM) + lenvatinib group** | **6** | | **BiAb004(hG1TM) at 1.33 mg/kg was injected intraperitoneally twice weekly in a total of 6 doses; Lenvatinib at 5 mg/kg was administered by oral gavage once daily in 20 doses** |

The sequence of the isotype control in this experiment, human anti-hen egg lysozyme IgG (anti-HEL antibody, or human IgG, abbreviated as hIgG), is derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1):130-46).

The experimental results are shown in FIG. 17.

The results showed that BiAb004(hG1TM) in combination with lenvatinib significantly inhibited the growth of MC38 tumors; BiAb004(hG1TM) in combination with lenvatinib demonstrated significantly efficacy against rectal and/or colon cancers of MSI-H/dMMR phenotype.

Experimental Example 10: Pharmacodynamic Evaluation of BiAb004(hG1TM) in C57BL/6-hPD1/hPDL1/hCD73 Mouse Colon Cancer MC38-hPDL1/hCD73 Subcutaneous Graft Tumor Model

Female C57BL/6-hPD1/hPDL1/hCD73 mice (purchased from Nanjing GemPharmatech Co., Ltd.) were divided into groups of 8 and grafted subcutaneously on the right forelimb with colon cancer MC38-hPDL1/hCD73 cells (purchased from Nanjing GemPharmatech Co., Ltd.) ($2 \times 10^6$ cells/100 μL/mouse). The day of grafting was defined as D0. The dosing volume was adjusted according to the body weight: 10 μL/g mouse body weight (g). The isotype control antibody (the preparation and the source are the same as those of the Experimental Example 9) or BiAb004(hG1TM) was administrated intraperitoneally twice weekly for 3 weeks, in a total of 6 doses. Tumors were measured continuously in the experiment, and the volume was calculated as the following formula: tumor volume ($mm^3$) = (tumor length $\times$ (tumor width)$^2$)/2.

The results are shown in FIG. 18 and in Table 9 below.

Table 9: Protocol and grouping

| Group | Dose (mg/kg) | Route | Frequency | Cycle |
|---|---|---|---|---|
| **Isotype Control** | **10** | **Intraperitoneal injection** | **Twice weekly, for a total of three weeks** | **6 doses in total** |
| **BiAb004 (hG1TM)** | **1** | | | |

The results showed that the tumor volume increase in the BiAb004(hG1TM) group was inhibited relative to the isotype control antibody group, indicating that BiAb004(hG1TM) significantly inhibits the proliferation of mouse MC38 cells, and can effectively treat colon cancer and/or rectal cancer.

Experimental Example 11: Antibody-Mediated Phagocytic Activity Study of BiAb004(hG1WT) and BiAb004(hG1TM) on CHO-K1-PD1

Zhang et al. (Zhang T et al, Cancer Immunol Immunother., 2018; 67(7):1079-1090.) and Dahan et al. (Dahan R et al., Cancer cell, 2015, 28(3):285-95.) reported that the binding of Fc fragments of antibodies targeting immune checkpoints such as PD-1 and CTLA-4 to Fc receptors negatively affects antibody-mediated anti-cancer activity, possibly due to Fc-dependent effector function-induced immune cell damage, where antibody-dependent cellular phagocytosis (ADCP) is an important mechanism leading to immune cell damage.

To test the ADCP activity of BiAb004(hG1TM), murine macrophages were used as effector cells and cell lines overexpressing the corresponding antigens were used as target cells. The ADCP effect mediated by the cells was tested. The femoral bone marrow of C57BL/6 mice (purchased from Guangdong Medical Laboratory Animal Center) was first aseptically collected and lysed by erythrocyte lysis buffer on ice for 5 min. The lysis was terminated with DMEM complete medium (containing 10% FBS), and the lysate was centrifuged at 1000 rpm and washed twice. The cell pellet was resuspended in 10 mL of DMEM complete medium and macrophage colony stimulating factor (M-CSF) were added at a working concentration of 100 ng/mL. The cells were cultured for 7 days at 37 °C and 5% $CO_2$ in a cell culture chamber for induction. Half of the medium was exchanged and M-CSF was added on Days 3 and 5. The induction of cells was completed on day 7. The cells were digested with 0.25% trypsin. Macrophages were collected, and centrifuged at 170x g for 5 min. The supernatant was discarded and the cells were suspended in DMEM complete medium and counted. The cell was adjusted to a proper density and filled into sterile EP tubes for further use.

CHO-K1-CTLA4-PD1 cells (a cell line based on CHO-K1 cells overexpressing both human CTLA4 and PD1 antigens, constructed by Akeso Biopharma, Inc.) at logarithmic phase were collected, centrifuged at 170x g for 5 min, washed with PBS, resuspended and counted, and the viability was determined. Carboxyfluorescein diacetate succinimidyl ester (CFSE) was diluted to 2.5 μM with PBS to resuspend the cells (staining density: 10 million cells/mL). The cells were incubated in a cell incubator for 20 min. 6 mL of DMEM complete medium was added to stop the staining. The cells were centrifuged at 170x g for 5 min, and the supernatant was discarded. 1 mL of DMEM complete medium was added for resuspension. The cells were incubated in an incubator for 10 min, and adjusted to the experiment density. The cells were coded as CHO-K1-PD1-CTLA4-CFSE. The test antibodies were diluted in DMEM complete medium. An isotype control antibody anti-HEL antibody and a medium were used as the isotype control group and a blank control group. According to the study design, the diluted antibody and CHO-K1-PD1-CTLA4-CFSE cells were added into 1.5 mL EP tubes containing macrophages (the final volume was 100 μL, the effector-to-target ratio was 50,000:150,000, the working concentrations of the antibody were 50, 5 and 0.5 nM). The mixture was resuspended, mixed evenly and incubated in an incubator at 37 °C for 2 h. 800 μL of PBS containing 1 % bovine serum albumin (BSA) was added at room temperature to each tube. The mixture was centrifuged at 1200x g for 5 min, and the supernatant was discarded.

The cells were washed once with 800 μL of 1 % PBSA. APC anti-mouse/human CD11b antibody (Biolegend, Cat. No.: 101212) was diluted 400-fold with 1 % PBSA and added to the corresponding samples at 100 μL/sample. The mixture was mixed well, incubated on ice for 30 min, washed once with 800 μL of 1% PBSA, and centrifuged at 1200x g for 5 min, and the supernatant was discarded. 200 μL of 1 % PBSA was added to each tube to resuspend the cells. The cells were transferred to loading tube and analyzed by BD FACSCalibur flow cytometer. Macrophages in the system were $APC^+$ positive, and macrophages involved in phagocytosis were APC and CFSE double positive. The phagocytosis rate was determined as the ratio of the number of double positive cells to the number of APC positive cells, and the antibody-mediated ADCP activity was evaluated.

The ADCP activity of each group, expressed as P%, was calculated as follows:

$$\mathbf{P}\% = \frac{\textbf{\textit{Number of macrophages involved in phagocytosis}}}{\textbf{\textit{Total number of macrophages}}} \times \mathbf{100}\%$$

The results are shown in FIG. 19.

The results showed that in the antibody validation system for mediating phagocytic activity, the phagocytic activity of CHO-K1-PD1-CTLA4 cells by macrophages mediated by BiAb004(hG1TM) had no significant difference from that of the isotype control antibody anti-HEL antibody in comparison with BiAb004(hG1WT), indicating that BiAb004(hG1TM) had no ADCP activity.

The results suggest that the amino acid mutation introduced by BiAb004(hG1TM) can effectively eliminate the ADCP effect, and a surprising technical effect is obtained.

Experimental Example 12: Significantly Enhanced Immune Response of Immune Cells to Human Gastric Cancer KATO III Cells by BiAb004(hG1TM)

PBMCs were isolated from healthy human peripheral blood according to the Ficoll-Paque™ Plus reagent instruction, and the isolated PBMCs were counted and frozen. Raji-PDL1 and KATO III cells (purchased from Chinese Academy of Sciences Shanghai Cell Bank) were cultured in DMEM + 10% FBS complete medium. PBMCs were thawed and activated with 0.5 μg/mL SEB for two days. On the day of the experiment, Raji-PDL1 cells were treated with 2 μg/mL mitomycin C (MMC) for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. KATO III cells in logarithmic growth phase were collected and seeded on the 96-well plate at $5 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 20.

The results showed that BiAb004(hG1TM) more significantly enhanced the immune response of immune cells to human gastric cancer cells KATO III characterized by significantly increased secretion level of IL-2 as compared to BiAb004(hG1WT), and thus BiAb004(hG1TM) is prospective for treating gastric cancer.

Experimental Example 13: Significantly Enhanced Immune Response of Immune Cells to Human Cervical Cancer Hela Cells by BiAb004(hG1TM)

PBMCs were isolated from healthy human peripheral blood according to the Ficoll-Paque™ Plus reagent instruction, and the isolated PBMCs were counted and frozen. Raji-PDL1 and Hela cells (purchased from Chinese Academy of Sciences Cell Bank) were cultured in RPMI 1640 + 10% FBS complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. Hela cells in logarithmic growth phase were collected and seeded on the 96-well plate at $5 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 21.

The results showed that BiAb004(hG1TM) more significantly enhanced the immune response of immune cells to human cervical cancer Hela cells characterized by significantly increased secretion level of IL-2 as compared to BiAb004(hG1WT), and thus BiAb004(hG1TM) is prospective for treating cervical cancer.

Experimental Example 14: Significantly Enhanced Immune Response of Immune Cells to Human T Cell Lymphomas Jurkat Cells by BiAb004(hG1TM)

PBMCs were isolated from healthy human peripheral blood according to the Ficoll-Paque™ Plus reagent instruction, and the isolated PBMCs were counted and frozen. Raji-PDL1 and human T-cell lymphoma Jurkat cells (purchased from Chinese Academy of Sciences Cell Bank) were cultured in RPMI 1640+10% FBS complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. Jurkat cells in logarithmic growth phase were collected and seeded on the 96-well plate at $1 \times 10^5$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 22.

BiAb004(hG1TM) significantly enhanced the immune response of immune cells to human T-cell lymphoma Jurkat cells characterized by significantly increased secretion level of IL-2 as compared to BiAb004(hG1WT), while eliminating the ADCC and CDC (i.e., ADCP); the antibody has an equivalent or higher pharmacological activity at doses of 1.34 nM and 20 nM as compared to BiAb004(hG1WT) and is prospective for treating cervical cancer.

Experimental Example 15: Significantly Enhanced Immune Response of Immune Cells to Human Nasopharyngeal Cancer CNE-2Z Cells by BiAb004(hG1TM)

PBMCs were isolated from healthy human peripheral blood according to the Ficoll-Paque™ Plus reagent instruction, and the isolated PBMCs were counted and frozen. Raji-PDL1 and CNE-2Z cells (purchased from GuangZhou Jennio Biotech Co., Ltd.) were cultured in RPMI 1640 + 10% FBS complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. CNE-2Z cells in logarithmic growth phase were collected and seeded on the 96-well plate at $3 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 23.

The results showed that BiAb004(hG1TM) equivalently and/or more significantly enhanced the immune response of immune cells to human nasopharyngeal cancer cells CNE-2Z characterized by significantly increased secretion level of IL-2 as compared to BiAb004(hG1WT), and that BiAb004(hG1TM) especially had significant superiority at 20 nM.

The above results showed that BiAb004(hG1TM) had better or equivalent pharmacological activity relative to BiAb004(hG1WT) on the basis of effectively eliminating ADCC, CDC and ADCP effects, indicating the potential for treating human nasopharyngeal cancer.

Experimental Example 16: Significantly Enhanced Immune Response of Immune Cells to Human Breast Cancer MDA-MB-231 Cells bv BiAb004(hG1TM)

PBMCs were isolated from healthy human peripheral blood according to the Ficoll-Paque™ Plus reagent instruction, and the isolated PBMCs were counted and frozen. Raji-PDL1 and MDA-MB-231 cells (purchased from GuangZhou Jennio Biotech Co., Ltd.) were cultured in RPMI 1640 + 10% FBS complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. MDA-MB-231 cells in logarithmic growth phase were collected and seeded on the 96-well plate at $3 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 24.

BiAb004(hG1TM) equivalently or more significantly enhanced the immune response of immune cells to human breast cancer MDA-MB-231 cells characterized by significantly increased secretion level of IL-2 as compared to BiAb004(hG1WT).

The above results showed that BiAb004(hG1TM) had better or equivalent pharmacological activity relative to

BiAb004(hG1WT) on the basis of effectively eliminating ADCC, CDC and ADCP effects, indicating the potential for treating human breast cancer.

Experimental Example 17: Significantly Enhanced Immune Response of Immune Cells to Human Mesothelioma NCI-H2452 Cells bv BiAb004(hG1TM)

PBMCs were isolated from healthy human peripheral blood according to the Ficoll-Paque™ Plus reagent instruction, and the isolated PBMCs were counted and frozen. Raji-PDL1 and NCI-H2452 cells (purchased from Chinese Academy of Sciences Shanghai Cell Bank) were cultured in RPMI 1640 + 10% FBS complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. NCI-H2452 cells in logarithmic growth phase were collected and seeded on the 96-well plate at $3 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 25.

The results showed that BiAb004(hG1TM) more significantly enhanced the immune response of immune cells to human mesothelioma cells NCI-H2452 characterized by significantly increased secretion level of IL-2 as compared to BiAb004(hG1WT), and thus BiAb004(hG1TM) is prospective for treating mesothelioma.

Experimental Example 18: Significantly Enhanced Immune Response of Immune Cells to Human Non-Small Cell Lung Cancer (Human Lung Adenocarcinoma) A549 Cells by BiAb004(hG1TM) in Combination with Anlotinib

PBMCs were isolated from healthy human peripheral blood according to the Ficoll-Paque™ Plus reagent instruction, and the isolated PBMCs were counted and frozen. Raji-PDL1 cells were cultured in RPMI 1640 + 10% FBS complete medium and A549 cells were cultured in DMEM + 10% FBS complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. A549 cells (purchased from Chinese Academy of Sciences Cell Bank) in logarithmic growth phase were collected and seeded on the 96-well plate at $5 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

As shown in Fig. 26, BiAb004(hG1TM) in combination with anlotinib hydrochloride more significantly enhanced the immune response of immune cells to human non-small cell lung cancer (human lung adenocarcinoma) A549 cells characterized by significantly increased secretion level of IL-2 as compared to BiAb004(hG1TM) monotherapy, BiAb004(hG1WT) monotherapy and BiAb004(hG1WT) in combination with anlotinib hydrochloride, and thus BiAb004(hG1TM) in combination with anlotinib hydrochloride is prospective for treating human non-small cell lung cancer or human lung adenocarcinoma.

Experimental Example 19: Significantly Enhanced Immune Response of Immune Cells to Human Small Cell Lung Cancer NCI-H446 Cells bv BiAb004(hG1TM) in Combination with Anlotinib

PBMCs were isolated from healthy human peripheral blood according to the Ficoll-Paque™ Plus reagent instruction, and the isolated PBMCs were counted and frozen. Raji-PDL1 and NCI-H446 cells (purchased from Chinese Academy of Sciences, Shanghai Institutes for Biological Sciences) were cultured in RPMI 1640 + 10% FBS complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. NCI-H446 cells (purchased from Chinese Academy of Sciences, Shanghai Institutes for Biological Sciences) in logarithmic growth phase were collected and seeded on the 96-well plate at $8 \times 10^4$ cells/well. The diluted antibody and anlotinib were added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 27.

BiAb004(hG1TM) in combination with anlotinib hydrochloride significantly enhanced the immune response of immune cells to human small cell lung cancer NCI-H446 cells characterized by significantly increased secretion level of IL-2 as compared to BiAb004(hG1MT) monotherapy, BiAb004(hG1WT) monotherapy and BiAb004(hG1WT) in combination with anlotinib hydrochloride, and thus BiAb004(hG1TM) in combination with anlotinib hydrochloride is prospective for treating human small cell lung cancer.

Experimental Example 20: Significantly Enhanced Immune Response of Immune Cells to Human Squamous Cell Lung Cancer NCI-H226 Cells bv BiAb004(hG1TM) in Combination with Anlotinib

PBMCs were isolated from healthy human peripheral blood according to the Ficoll-Paque™ Plus reagent instruction, and the isolated PBMCs were counted and frozen. Raji-PDL1 and NCI-H226 cells (purchased from Chinese Academy of Sciences, Shanghai Institutes for Biological Sciences) were cultured in RPMI 1640 + 10% FBS complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. NCI-H226 cells in logarithmic growth phase were collected and seeded on the 96-well plate at $5 \times 10^4$ cells/well. The diluted antibody and anlotinib were added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 28.

BiAb004(hG1TM) monotherapy more effectively and significantly enhanced the immune response of immune cells to human squamous cell lung cancer NCI-H226 cells characterized by significantly increased secretion level of IL-2 as compared to BiAb004(hG1WT) monotherapy.

BiAb004(hG1TM) in combination with anlotinib hydrochloride more significantly enhanced the immune response of immune cells to human squamous cell lung cancer NCI-H226 cells as compared to BiAb004(hG1WT) monotherapy and BiAb004(hG1TM) monotherapy, and demonstrated a comparable pharmacological activity with BiAb004(hG1WT) in combination with anlotinib.

The above results showed that BiAb004(hG1TM) had better or equivalent pharmacological activity relative to BiAb004(hG1WT) monotherapy or BiAb004(hG1WT) in combination with anlotinib on the basis of effectively eliminating ADCC, CDC and ADCP effects, indicating a better efficacy for treating human squamous cell lung cancer.

Experimental Example 21: Significantly Enhanced Immune Response of Immune Cells to Human Colorectal Cancer SW48 Cells of MSI-H/dMMR Phenotype bv BiAb004(hG1TM)

SW48 is a human colorectal cancer cell line and is identified with MSI-H/dMMR phenotype (Branch P et al., (1995), Cancer Res, 55(11): 2304-2309.). It was used for detecting the enhanced immune cell response to tumor of MSI-H/dMMR phenotype by BiAb004(hG1TM).

In the experiment, PBMCs were isolated from healthy human peripheral blood according to the Ficoll-Paque™ Plus reagent instruction, and the isolated PBMCs were counted and frozen. Raji-PDL1 cells were cultured in RPMI 1640 + 10% FBS complete medium, and SW48 cells (purchased from GuangZhou Jennio Biotech Co., Ltd.) were cultured in DMEM + 10% FBS complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. SW48 cells in logarithmic growth phase were collected and seeded on the 96-well plate at $2 \times 10^5$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 29.

Both BiAb004(hG1WT) and BiAb004(hG1TM) significantly enhanced the immune response of immune cells to human colorectal cancer cells SW48 cells of MSI-H/dMMR phenotype characterized by significantly increased secretion level of IL-2 as compared to anti-HEL antibody.

The above results showed that BiAb004(hG1TM) had better or equivalent pharmacological activity relative to BiAb004(hG1WT) on the basis of effectively eliminating ADCC, CDC and ADCP effects, indicating the potential for treating solid tumor of MSI-H/dMMR phenotype, particularly colon cancer and/or rectal cancer of MSI-H/dMMR phenotype.

Experimental Example 22: Significantly Enhanced Immune Response of Immune Cells to Human Colorectal Cancer SW837 Cells of MSI-H/dMMR Phenotype bv BiAb004(hG1TM)

SW837 is a human colorectal cancer cell line of non-MSI-H/dMMR (i.e., MSS) phenotype (Guo J et al., Cancer Res., 2011;71(8):2978-2987.), and was used for detecting the enhanced immune cell response to tumor of non-MSI-H/dMMR phenotype by BiAb004(hG1TM) in this example.

PBMCs were isolated from healthy human peripheral blood according to the Ficoll-Paque™ Plus reagent instruction, and the isolated PBMCs were counted and frozen. Raji-PDL1 cells were cultured in RPMI 1640 + 10% FBS complete medium, and SW837 cells (purchased from Shanghai Honsun Biological Technology Co., Ltd) were cultured in 10% FBS + Leibovitz's L-15 (purchased from Gibco) complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDL1 and PBMC cells were seeded on 96-well plates at $1 \times 10^5$ cells/well. SW837 cells in logarithmic growth phase were collected and seeded on the 96-well plate at $5 \times 10^4$ cells/well. The diluted antibody was added according to the study design. The mixture was mixed evenly and incubated in a 5% CO2 incubator at 37 °C for 3 days.After 3 days, the cell culture supernatant was collected and tested for IL-2 according to ELISA KIT instruction.

The media in this experiment were all 10% FBS + RPMI 1640.

The results are shown in FIG. 30.

Both BiAb004(hG1WT) and BiAb004(hG1TM) significantly enhanced the immune response of immune cells to human colorectal cancer cells SW837 cells of non-MSI-H/dMMR phenotype as compared to anti-HEL antibody. The pharmacological activity of BiAb004(hG1TM) in the high dose group was superior to that of BiAb004(hG1WT), characterized by significantly increased secretion level of IL-2.

The above results showed that BiAb004(hG1TM) had better or equivalent pharmacological activity relative to BiAb004(hG1WT) on the basis of effectively eliminating ADCC, CDC or ADCP effects, indicating the potential for treating solid tumor of non-MSI-H/dMMR phenotype, particularly colon cancer and/or rectal cancer of non-MSI-H/dMMR phenotype. Although specific embodiments of the present invention have been described in detail, those skilled in the art will understand. Various modifications and substitutions can be made to those details according to all the teachings that have been disclosed, and these changes are all within the protection scope of the present invention. The full scope of the present invention is given by the appended claims and any equivalent thereof.

## Claims

**1.** A bispecific antibody, comprising:

a first protein functional region targeting PD-1, and
a second protein functional region targeting CTLA4;
wherein the first protein functional region is an immunoglobulin, and the second protein functional region is a single chain antibody; or, the first protein functional region is a single chain antibody, and the second protein functional region is an immunoglobulin;
wherein,
for the immunoglobulin, the heavy chain variable region comprises HCDR1-HCDR3 of amino acid sequences set forth in SEQ ID NOs: 27-29 respectively, and the light chain variable region comprises LCDR1-LCDR3 of amino acid sequences set forth in SEQ ID NOs: 30-32 respectively; for the single chain antibody, the heavy chain variable region comprises HCDR1-HCDR3 of amino acid sequences set forth in SEQ ID NOs: 33-35 respectively, and the light chain variable region comprises LCDR1-LCDR3 of amino acid sequences set forth in SEQ ID NOs: 36-38 respectively;
or,
for the immunoglobulin, the heavy chain variable region comprises HCDR1-HCDR3 of amino acid sequences set forth in SEQ ID NOs: 33-35 respectively, and the light chain variable region comprises LCDR1-LCDR3 of amino acid sequences set forth in SEQ ID NOs: 36-38 respectively; for the single chain antibody, the heavy chain variable region comprises HCDR1-HCDR3 of amino acid sequences set forth in SEQ ID NOs: 27-29 respectively, and the light chain variable region comprises LCDR1-LCDR3 of amino acid sequences set forth in SEQ ID NOs: 30-32 respectively;
the immunoglobulin is of human IgG1 subtype;
wherein, according to the EU numbering system, the heavy chain constant region of the immunoglobulin has mutations at any 2 or 3 of positions 234, 235 and 237, and the affinity constant of the bispecific antibody to

FcyRIIIa and/or C1q is reduced after the mutation as compared to that before the mutation; preferably, the affinity constant is measured by a Fortebio Octet system.

**2.** The bispecific antibody according to claim 1, wherein, according to the EU numbering system, the heavy chain constant region of the immunoglobulin has the following mutations:

L234A and L235A;
L234A and G237A;
L235A and G237A;
or
L234A, L235A and G237A.

**3.** A bispecific antibody, comprising:

a first protein functional region targeting PD-1, and
a second protein functional region targeting CTLA4;
wherein the first protein functional region is an immunoglobulin, and the second protein functional region is a single chain antibody; or, the first protein functional region is a single chain antibody, and the second protein functional region is an immunoglobulin;
wherein,
for the immunoglobulin, the heavy chain variable region comprises HCDR1-HCDR3 of amino acid sequences set forth in SEQ ID NOs: 27-29 respectively, and the light chain variable region comprises LCDR1-LCDR3 of amino acid sequences set forth in SEQ ID NOs: 30-32 respectively; for the single chain antibody, the heavy chain variable region comprises HCDR1-HCDR3 of amino acid sequences set forth in SEQ ID NOs: 33-35 respectively, and the light chain variable region comprises LCDR1-LCDR3 of amino acid sequences set forth in SEQ ID NOs: 36-38 respectively;
or,
for the immunoglobulin, the heavy chain variable region comprises HCDR1-HCDR3 of amino acid sequences set forth in SEQ ID NOs: 33-35 respectively, and the light chain variable region comprises LCDR1-LCDR3 of amino acid sequences set forth in SEQ ID NOs: 36-38 respectively; for the single chain antibody, the heavy chain variable region comprises HCDR1-HCDR3 of amino acid sequences set forth in SEQ ID NOs: 27-29 respectively, and the light chain variable region comprises LCDR1-LCDR3 of amino acid sequences set forth in SEQ ID NOs: 30-32 respectively;
the immunoglobulin is of human IgG1 subtype;
wherein, according to the EU numbering system, the heavy chain constant region of the immunoglobulin has the following mutations:

L234A and L235A;
L234A and G237A;
L235A and G237A; or
L234A, L235A and G237A.

**4.** The bispecific antibody according to any of claims 1-3, wherein, according to the EU numbering system, the heavy chain constant region of the immunoglobulin has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

**5.** The bispecific antibody according to any of claims 1-4, wherein,

the amino acid sequence of the heavy chain variable region of the immunoglobulin is selected from SEQ ID NO: 14 and SEQ ID NO: 18; the amino acid sequence of the light chain variable region of the immunoglobulin is selected from SEQ ID NO: 16 and SEQ ID NO: 20; the amino acid sequence of the heavy chain variable region of the single chain antibody is selected from SEQ ID NO: 2, SEQ ID NO: 6, SEQ ID NO: 10, SEQ ID NO: 41 and SEQ ID NO: 43; the amino acid sequence of the light chain variable region of the single chain antibody is selected from SEQ ID NO: 4, SEQ ID NO: 8, SEQ ID NO: 12, SEQ ID NO: 42 and SEQ ID NO: 44;
or,
the amino acid sequence of the heavy chain variable region of the immunoglobulin is selected from SEQ ID

NO: 2, SEQ ID NO: 6, SEQ ID NO: 10, SEQ ID NO: 41 and SEQ ID NO: 43; the amino acid sequence of the light chain variable region of the immunoglobulin is selected from SEQ ID NO: 4, SEQ ID NO: 8, SEQ ID NO: 12, SEQ ID NO: 42 and SEQ ID NO: 44; the amino acid sequence of the heavy chain variable region of the single chain antibody is selected from SEQ ID NO: 14 and SEQ ID NO: 18; the amino acid sequence of the light chain variable region of the single chain antibody is selected from SEQ ID NO: 16 and SEQ ID NO: 20.

**6.** The bispecific antibody according to any of claims 1-5, wherein the bispecific antibody is selected from any one of the following (1)-(20):

(1)
the amino acid sequence of the heavy chain variable region of the immunoglobulin is set forth in SEQ ID NO: 14, and the amino acid sequence of the light chain variable region of the immunoglobulin is set forth in SEQ ID NO: 16; the amino acid sequence of the heavy chain variable region of the single chain antibody is set forth in SEQ ID NO: 2, and the amino acid sequence of the light chain variable region of the single chain antibody is set forth in SEQ ID NO: 4;
(2)
the amino acid sequence of the heavy chain variable region of the immunoglobulin is set forth in SEQ ID NO: 14, and the amino acid sequence of the light chain variable region of the immunoglobulin is set forth in SEQ ID NO: 16; the amino acid sequence of the heavy chain variable region of the single chain antibody is set forth in SEQ ID NO: 6, and the amino acid sequence of the light chain variable region of the single chain antibody is set forth in SEQ ID NO: 8;
(3)
the amino acid sequence of the heavy chain variable region of the immunoglobulin is set forth in SEQ ID NO: 14, and the amino acid sequence of the light chain variable region of the immunoglobulin is set forth in SEQ ID NO: 16; the amino acid sequence of the heavy chain variable region of the single chain antibody is set forth in SEQ ID NO: 10, and the amino acid sequence of the light chain variable region of the single chain antibody is set forth in SEQ ID NO: 12;
(4)
the amino acid sequence of the heavy chain variable region of the immunoglobulin is set forth in SEQ ID NO: 18, and the amino acid sequence of the light chain variable region of the immunoglobulin is set forth in SEQ ID NO: 20; the amino acid sequence of the heavy chain variable region of the single chain antibody is set forth in SEQ ID NO: 2, and the amino acid sequence of the light chain variable region of the single chain antibody is set forth in SEQ ID NO: 4;
(5)
the amino acid sequence of the heavy chain variable region of the immunoglobulin is set forth in SEQ ID NO: 18, and the amino acid sequence of the light chain variable region of the immunoglobulin is set forth in SEQ ID NO: 20; the amino acid sequence of the heavy chain variable region of the single chain antibody is set forth in SEQ ID NO: 6, and the amino acid sequence of the light chain variable region of the single chain antibody is set forth in SEQ ID NO: 8;
(6)
the amino acid sequence of the heavy chain variable region of the immunoglobulin is set forth in SEQ ID NO: 18, and the amino acid sequence of the light chain variable region of the immunoglobulin is set forth in SEQ ID NO: 20; the amino acid sequence of the heavy chain variable region of the single chain antibody is set forth in SEQ ID NO: 10, and the amino acid sequence of the light chain variable region of the single chain antibody is set forth in SEQ ID NO: 12;
(7)
the amino acid sequence of the heavy chain variable region of the immunoglobulin is set forth in SEQ ID NO: 2, and the amino acid sequence of the light chain variable region of the immunoglobulin is set forth in SEQ ID NO: 4; the amino acid sequence of the heavy chain variable region of the single chain antibody is set forth in SEQ ID NO: 14, and the amino acid sequence of the light chain variable region of the single chain antibody is set forth in SEQ ID NO: 16;
(8)
the amino acid sequence of the heavy chain variable region of the immunoglobulin is set forth in SEQ ID NO: 2, and the amino acid sequence of the light chain variable region of the immunoglobulin is set forth in SEQ ID NO: 4; the amino acid sequence of the heavy chain variable region of the single chain antibody is set forth in SEQ ID NO: 18, and the amino acid sequence of the light chain variable region of the single chain antibody is set forth in SEQ ID NO: 20;
(9)

the amino acid sequence of the heavy chain variable region of the immunoglobulin is set forth in SEQ ID NO: 6, and the amino acid sequence of the light chain variable region of the immunoglobulin is set forth in SEQ ID NO: 8; the amino acid sequence of the heavy chain variable region of the single chain antibody is set forth in SEQ ID NO: 14, and the amino acid sequence of the light chain variable region of the single chain antibody is set forth in SEQ ID NO: 16;

(10)

the amino acid sequence of the heavy chain variable region of the immunoglobulin is set forth in SEQ ID NO: 6, and the amino acid sequence of the light chain variable region of the immunoglobulin is set forth in SEQ ID NO: 8; the amino acid sequence of the heavy chain variable region of the single chain antibody is set forth in SEQ ID NO: 18, and the amino acid sequence of the light chain variable region of the single chain antibody is set forth in SEQ ID NO: 20;

(11)

the amino acid sequence of the heavy chain variable region of the immunoglobulin is set forth in SEQ ID NO: 10, and the amino acid sequence of the light chain variable region of the immunoglobulin is set forth in SEQ ID NO: 12; the amino acid sequence of the heavy chain variable region of the single chain antibody is set forth in SEQ ID NO: 14, and the amino acid sequence of the light chain variable region of the single chain antibody is set forth in SEQ ID NO: 16;

(12)

the amino acid sequence of the heavy chain variable region of the immunoglobulin is set forth in SEQ ID NO: 10, and the amino acid sequence of the light chain variable region of the immunoglobulin is set forth in SEQ ID NO: 12; the amino acid sequence of the heavy chain variable region of the single chain antibody is set forth in SEQ ID NO: 18, and the amino acid sequence of the light chain variable region of the single chain antibody is set forth in SEQ ID NO: 20;

(13)

the amino acid sequence of the heavy chain variable region of the immunoglobulin is set forth in SEQ ID NO: 14, and the amino acid sequence of the light chain variable region of the immunoglobulin is set forth in SEQ ID NO: 16; the amino acid sequence of the heavy chain variable region of the single chain antibody is set forth in SEQ ID NO: 41, and the amino acid sequence of the light chain variable region of the single chain antibody is set forth in SEQ ID NO: 42;

(14)

the amino acid sequence of the heavy chain variable region of the immunoglobulin is set forth in SEQ ID NO: 14, and the amino acid sequence of the light chain variable region of the immunoglobulin is set forth in SEQ ID NO: 16; the amino acid sequence of the heavy chain variable region of the single chain antibody is set forth in SEQ ID NO: 43, and the amino acid sequence of the light chain variable region of the single chain antibody is set forth in SEQ ID NO: 44;

(15)

the amino acid sequence of the heavy chain variable region of the immunoglobulin is set forth in SEQ ID NO: 18, and the amino acid sequence of the light chain variable region of the immunoglobulin is set forth in SEQ ID NO: 20; the amino acid sequence of the heavy chain variable region of the single chain antibody is set forth in SEQ ID NO: 41, and the amino acid sequence of the light chain variable region of the single chain antibody is set forth in SEQ ID NO: 42;

(16)

the amino acid sequence of the heavy chain variable region of the immunoglobulin is set forth in SEQ ID NO: 18, and the amino acid sequence of the light chain variable region of the immunoglobulin is set forth in SEQ ID NO: 20; the amino acid sequence of the heavy chain variable region of the single chain antibody is set forth in SEQ ID NO: 43, and the amino acid sequence of the light chain variable region of the single chain antibody is set forth in SEQ ID NO: 44;

(17)

the amino acid sequence of the heavy chain variable region of the immunoglobulin is set forth in SEQ ID NO: 41, and the amino acid sequence of the light chain variable region of the immunoglobulin is set forth in SEQ ID NO: 42; the amino acid sequence of the heavy chain variable region of the single chain antibody is set forth in SEQ ID NO: 14, and the amino acid sequence of the light chain variable region of the single chain antibody is set forth in SEQ ID NO: 16;

(18)

the amino acid sequence of the heavy chain variable region of the immunoglobulin is set forth in SEQ ID NO: 43, and the amino acid sequence of the light chain variable region of the immunoglobulin is set forth in SEQ ID NO: 44; the amino acid sequence of the heavy chain variable region of the single chain antibody is set forth in SEQ ID NO: 14, and the amino acid sequence of the light chain variable region of the single chain antibody is

set forth in SEQ ID NO: 16;
(19)
the amino acid sequence of the heavy chain variable region of the immunoglobulin is set forth in SEQ ID NO: 41, and the amino acid sequence of the light chain variable region of the immunoglobulin is set forth in SEQ ID NO: 42; the amino acid sequence of the heavy chain variable region of the single chain antibody is set forth in SEQ ID NO: 18, and the amino acid sequence of the light chain variable region of the single chain antibody is set forth in SEQ ID NO: 20;
and,
(20)
the amino acid sequence of the heavy chain variable region of the immunoglobulin is set forth in SEQ ID NO: 43, and the amino acid sequence of the light chain variable region of the immunoglobulin is set forth in SEQ ID NO: 44; the amino acid sequence of the heavy chain variable region of the single chain antibody is set forth in SEQ ID NO: 18, and the amino acid sequence of the light chain variable region of the single chain antibody is set forth in SEQ ID NO: 20.

**7.** The bispecific antibody according to any of claims 1-6, wherein,
the amino acid sequence of the heavy chain of the immunoglobulin is set forth in SEQ ID NO: 40, and the amino acid sequence of the light chain of the immunoglobulin is set forth in SEQ ID NO: 24.

**8.** The bispecific antibody according to any of claims 1-7, wherein the immunoglobulin or the antigen-binding fragment thereof binds to FcγRIIIa_F158, FcyRI, FcγRIIa_H131, FcγRIIIa_V158 and/or FcyRIIb with an affinity constant greater than about $10^{-7}$ M, for example, greater than about $10^{-6}$ M, $10^{-5}$ M, $10^{-4}$ M, or $10^{-3}$ M or greater; preferably, the affinity constant is measured by a Fortebio Octet system;
preferably, the immunoglobulin or the antigen binding fragment thereof has no binding signal or a binding signal of less than 0.1 nm to FcγRIIIa_F158, FcyRI, FcγRIIa_H131, FcγRIIIa_V158 and/or FcyRIIb; preferably, the binding signal refers to a response measured by a Fortebio Octet system.

**9.** The bispecific antibody according to any of claims 1-8, wherein the immunoglobulin or the antigen-binding fragment thereof binds to C1q with an affinity constant greater than about $10^{-9}$ M, for example, greater than about $10^{-8}$ M, $10^{-7}$ M, $10^{-6}$ M, or $10^{-5}$ M or greater; preferably, the affinity constant is measured by a Fortebio Octet system;
preferably, the immunoglobulin or the antigen binding fragment thereof has no binding signal or a binding signal of less than 0.1 nm to C1q; preferably, the binding signal refers to a response measured by a Fortebio Octet system.

**10.** The bispecific antibody of any of claims 1-9, wherein the first protein functional region is linked to the second protein functional region either directly or via a linker fragment; and/or the heavy chain variable region of the single chain antibody is linked to the light chain variable region of the single chain antibody either directly or via a linker fragment.

**11.** The bispecific antibody of claim 10, wherein the linker fragment is (GGGGS)n, n being a positive integer; preferably, n is 1, 2, 3, 4, 5 or 6.

**12.** The bispecific antibody according to any of claims 1-11, wherein the numbers of the first protein functional region and second protein functional region are each independently 1, 2 or more.

**13.** The bispecific antibody according to any of claims 1-12, wherein the single chain antibody is linked to the C terminus of the heavy chain of the immunoglobulin.

**14.** A bispecific antibody, comprising:

a first protein functional region targeting PD-1, and
a second protein functional region targeting CTLA4;
the number of the first protein functional region is 1, and the number of the second protein functional region is 2; wherein the first protein functional region is an immunoglobulin, and the second protein functional region is a single chain antibody;
the amino acid sequence of the heavy chain of the immunoglobulin is set forth in SEQ ID NO: 40, and the amino acid sequence of the light chain of the immunoglobulin is set forth in SEQ ID NO: 24;
the amino acid sequence of the heavy chain variable region of the single chain antibody is set forth in SEQ ID NO: 43, and the amino acid sequence of the light chain variable region of the single chain antibody is set forth in SEQ ID NO: 44;

the single chain antibody is linked to the C terminus of the heavy chain of the immunoglobulin;
the first protein functional region is linked to the second protein functional region via a first linker fragment; and the heavy chain variable region of the single chain antibody is linked to the light chain variable region of the single chain antibody via a second linker fragment;
the first linker fragment and the second linker fragment are the same or different;
preferably, the amino acid sequences of the first linker fragment and second linker fragment are independently selected from SEQ ID NO: 25 and SEQ ID NO: 26;
preferably, the amino acid sequences of the first linker fragment and second linker fragments are set forth in SEQ ID NO: 26.

**15.** An isolated nucleic acid molecule, encoding the bispecific antibody according to any of claims 1-14.

**16.** A vector, comprising the isolated nucleic acid molecule according to claim 15.

**17.** A host cell, comprising the isolated nucleic acid molecule according to claim 15 or the vector according to claim 16.

**18.** A conjugate comprising an antibody or antigen-binding fragment thereof, and a conjugated moiety, wherein the immunoglobulin is the bispecific antibody according to any of claims 1-14, and the conjugated moiety is a detectable label; preferably, the conjugated moiety is a radioisotope, a fluorescent substance, a luminescent substance, a colored substance, or an enzyme.

**19.** A kit, comprising the bispecific antibody according to any of claims 1-14 or the conjugate according to claim 18; wherein preferably, the kit further comprises a second antibody capable of specifically recognizing the immunoglobulin or the antigen binding fragment thereof; optionally, the second antibody further comprises a detectable label, for example, a radioisotope, a fluorescent substance, a luminescent substance, a colored substance, or an enzyme.

**20.** Use of the bispecific antibody according to any of claims 1-14 or the conjugate according to claim 18 in preparing a kit for detecting the presence or level of PD-1 and/or CTLA4 in a sample.

**21.** A pharmaceutical composition, comprising the bispecific antibody according to any of claims 1-14 or the conjugate according to claim 18, wherein, optionally, the pharmaceutical composition further comprises a pharmaceutically acceptable carrier and/or excipient.

**22.** The pharmaceutical composition according to claim 21, further comprising one or more anti-tumor chemotherapeutics;
preferably, the anti-tumor chemotherapeutic is a tyrosine kinase inhibitor; more preferably, the anti-tumor chemotherapeutic is anlotinib or a pharmaceutically acceptable salt thereof (e.g., hydrochloride salt), or lenvatinib or a pharmaceutically acceptable salt thereof (e.g., mesylate salt).

**23.** The pharmaceutical composition according to claim 21 or 22, wherein the unit dose of the pharmaceutical composition is 100-1000 mg, 200-800 mg, 200-500 mg, 300-600 mg, 400-500 mg, or 450 mg, based on the mass of the bispecific antibody.

**24.** A combination product comprising a first product and a second product in separate packages,

wherein,
the first product comprises the bispecific antibody according to any of claims 1-14, the conjugate according to claim 18, or the pharmaceutical composition according to any of claims 21-23;
the second product comprises one or more anti-tumor chemotherapeutics; preferably, the anti-tumor chemotherapeutic is a tyrosine kinase inhibitor; more preferably, the anti-tumor chemotherapeutic is anlotinib or a pharmaceutically acceptable salt thereof (e.g., hydrochloride salt), or lenvatinib or a pharmaceutically acceptable salt thereof (e.g., mesylate salt);
preferably, the first product and the second product further independently comprise one or more pharmaceutically acceptable excipients;
preferably, the combination product further comprises a package insert.

**25.** The combination product according to claim 24, wherein the unit dose of the first product is 100-1000 mg, 200-800 mg, 200-500 mg, 300-600 mg, 400-500 mg, or 450 mg, based on the mass of the bispecific antibody.

**26.** The combination product according to claim 24 or 25, wherein the unit dose of the second product is 0.1-100 mg, 0.5-50 mg, 1-20 mg, 2-15 mg, 4-12 mg, or 8-12 mg, based on the mass of the active ingredient.

**27.** Use of the bispecific antibody according to any of claims 1-14 or the conjugate according to claim 18 in preparing a medicament for treating and/or preventing a tumor or anemia, or in preparing a medicament for diagnosing a tumor or anemia;

preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

**28.** Use of the bispecific antibody according to any of claims 1-14 or the conjugate according to claim 18 in preparing:

a medicament for blocking the binding of PD-1 to PD-L1,
a medicament for down-regulating the activity or level of PD-1,
a medicament for relieving the immunosuppression of PD-1 in an organism, or
a medicament for elevating IFN-$\gamma$ and/or IL-2 expression in T lymphocytes;
and/or
a medicament for blocking the binding of CTLA4 to B7,
a medicament for down-regulating the activity or level of CTLA4,
a medicament for relieving the immunosuppression of CTLA4 in an organism, or
a medicament for elevating IL-2 expression in T lymphocytes.

**29.** The bispecific antibody according to any of claims 1-14, the conjugate according to claim 18, the pharmaceutical composition according to any of claims 21-23 or the combination product according to any of claims 24-26 for use in treating and/or preventing a tumor or anemia, or for use in diagnosing a tumor or anemia;

preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

**30.** A method for preventing and/or treating a tumor, comprising: administering to a subject in need an effective amount of the bispecific antibody according to any of claims 1-14, the conjugate according to claim 18, the pharmaceutical composition according to any of claims 21-23 or the combination product according to any one of claims 24-26;

preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma and nasopharyngeal cancer;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

**31.** The method according to claim 30, wherein the administration is before or after a surgical treatment and/or before or after a radiotherapy.

**32.** The method according to claim 30 or 31, wherein the unit dose of the bispecific antibody is 0.1-100 mg per kg body weight, preferably 1-10 mg per kg body weight; alternatively, the unit dose of the bispecific antibody is 10-1000 mg, preferably 50-500 mg in each subject;

preferably, the dose is given once every 3 days, 4 days, 5 days, 6 days, 10 days, 1 week, 2 weeks or 3 weeks; preferably, the route of administration is intravenous drip infusion or intravenous injection.

**33.** The method according to any of claims 30-32, wherein the administration of the bispecific antibody is performed in cycles of 2 or 3 weeks, and preferably, the bispecific antibody is administered intravenously on the first day of each cycle; preferably, the bispecific antibody is administered once every two or three weeks.

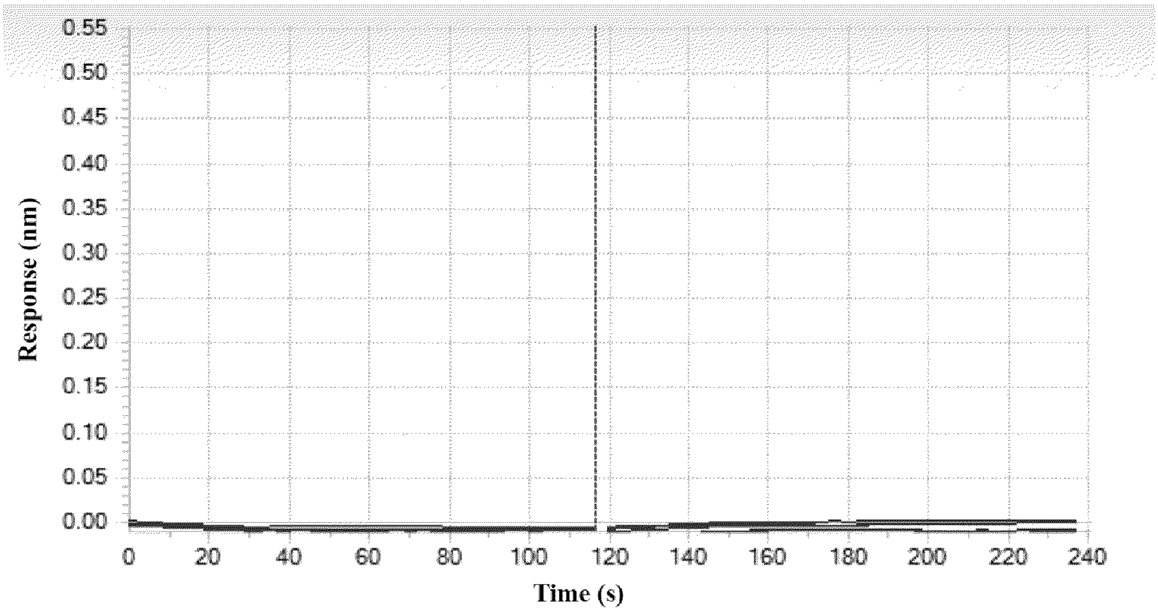
0.55
0.50
0.45
0.40
0.35
0.30
0.25
0.20
0.15
0.10
0.05
0.00
Response (nm)
0
20
40
60
80
100
120
140
160
180
200
220
240
Time (s)

FIG. 1

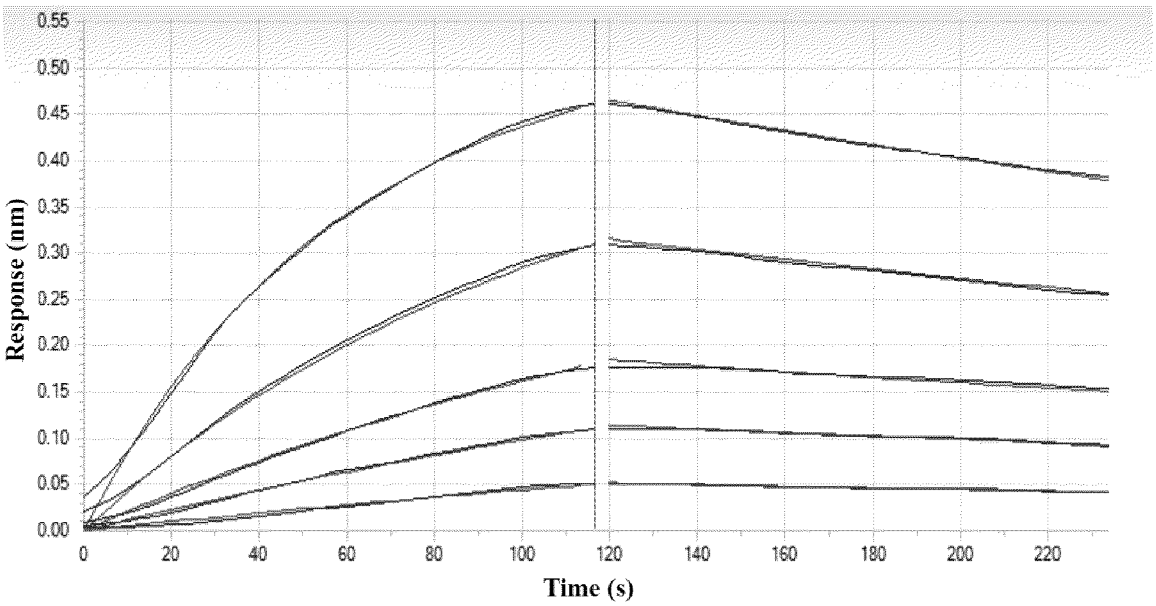
0.55
0.50
0.45
0.40
0.35
0.30
0.25
0.20
0.15
0.10
0.05
0.00
Response (nm)
0
20
40
60
80
100
120
140
160
180
200
220
Time (s)

FIG. 2

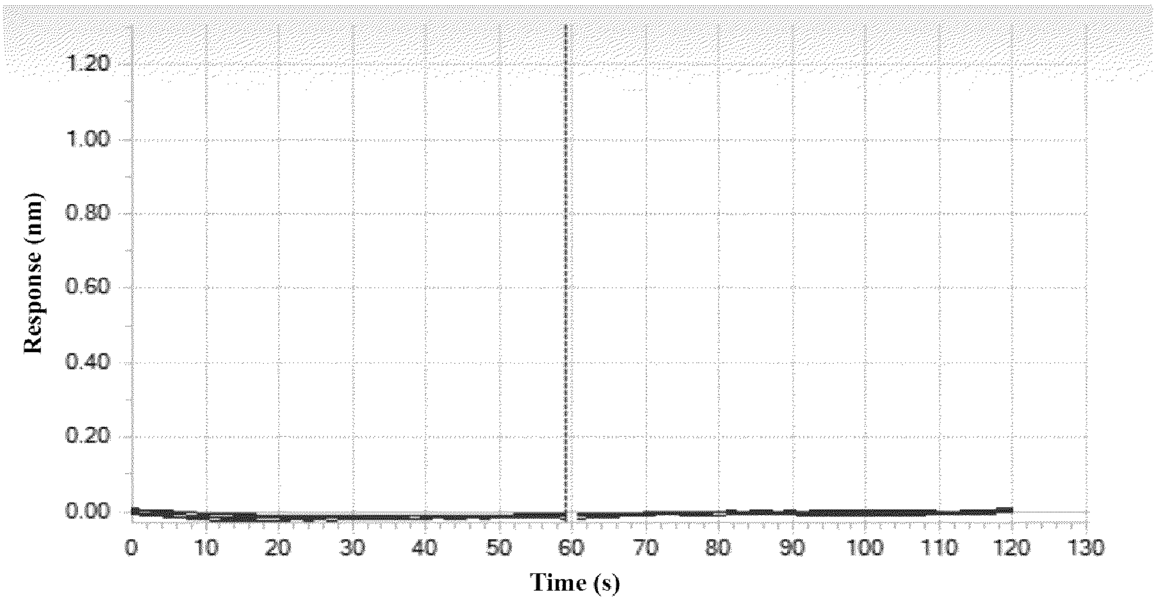
Response (nm)
1.20
1.00
0.80
0.60
0.40
0.20
0.00
0 10 20 30 40 50 60 70 80 90 100 110 120 130
Time (s)

FIG. 3

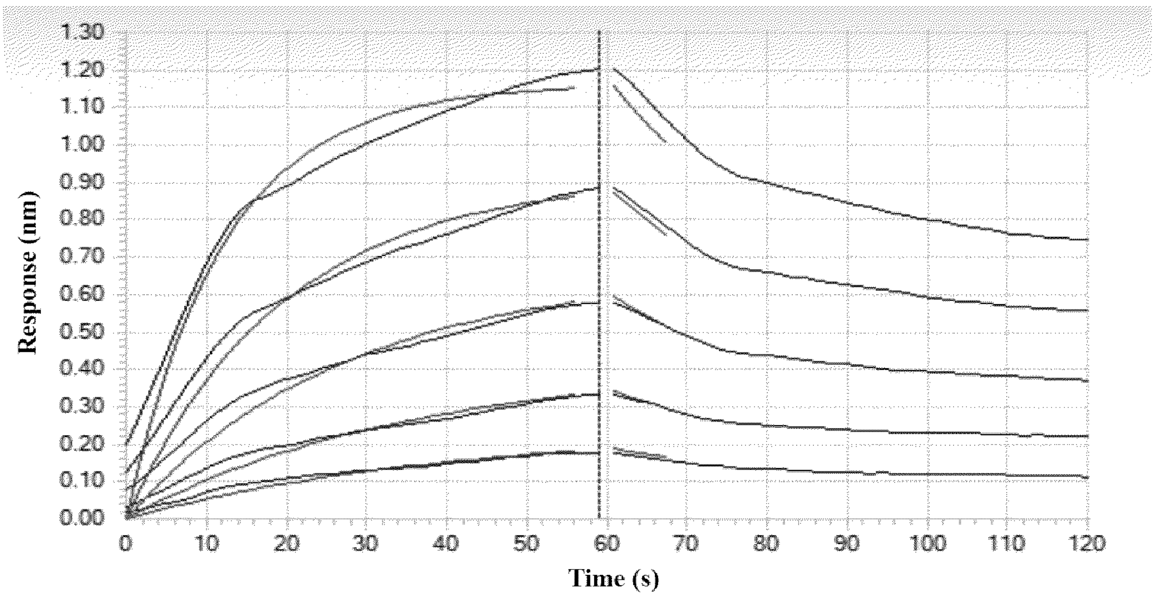
Response (nm)
1.30
1.20
1.10
1.00
0.90
0.80
0.70
0.60
0.50
0.40
0.30
0.20
0.10
0.00
0 10 20 30 40 50 60 70 80 90 100 110 120
Time (s)

FIG. 4

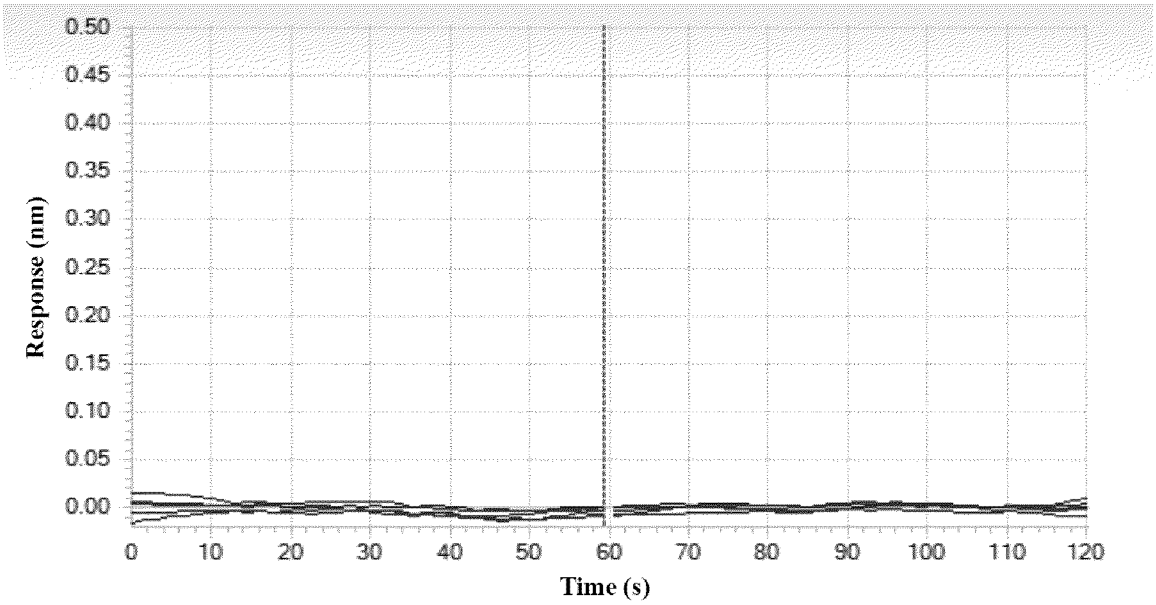
Response (nm)
0.50
0.45
0.40
0.35
0.30
0.25
0.20
0.15
0.10
0.05
0.00
0
10
20
30
40
50
60
70
80
90
100
110
120
Time (s)

FIG. 5

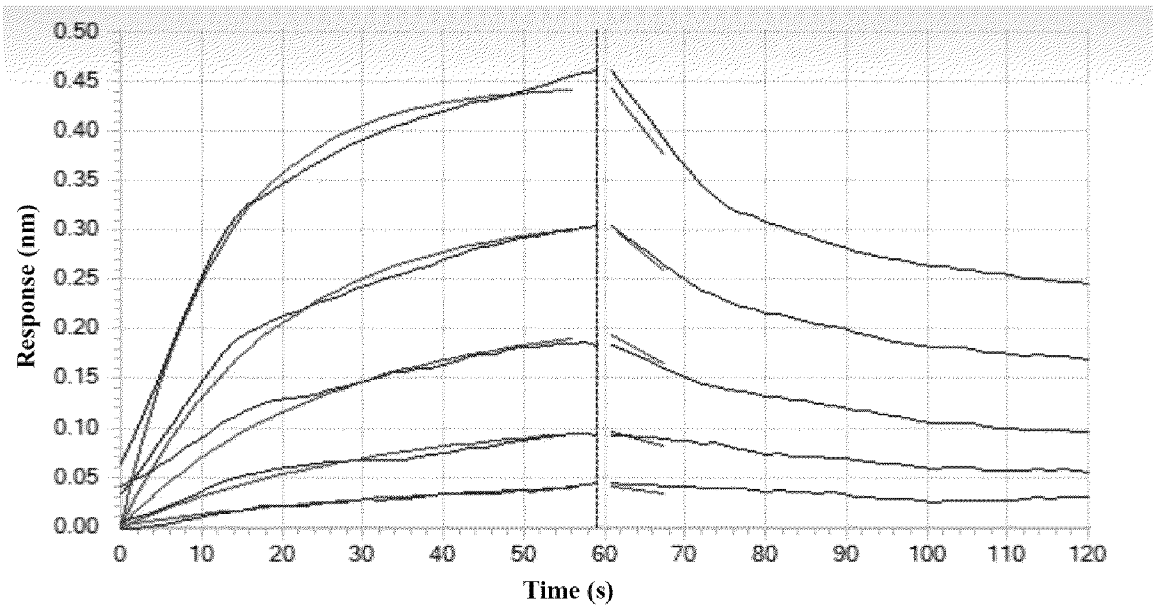
Response (nm)
0.50
0.45
0.40
0.35
0.30
0.25
0.20
0.15
0.10
0.05
0.00
0
10
20
30
40
50
60
70
80
90
100
110
120
Time (s)

FIG. 6

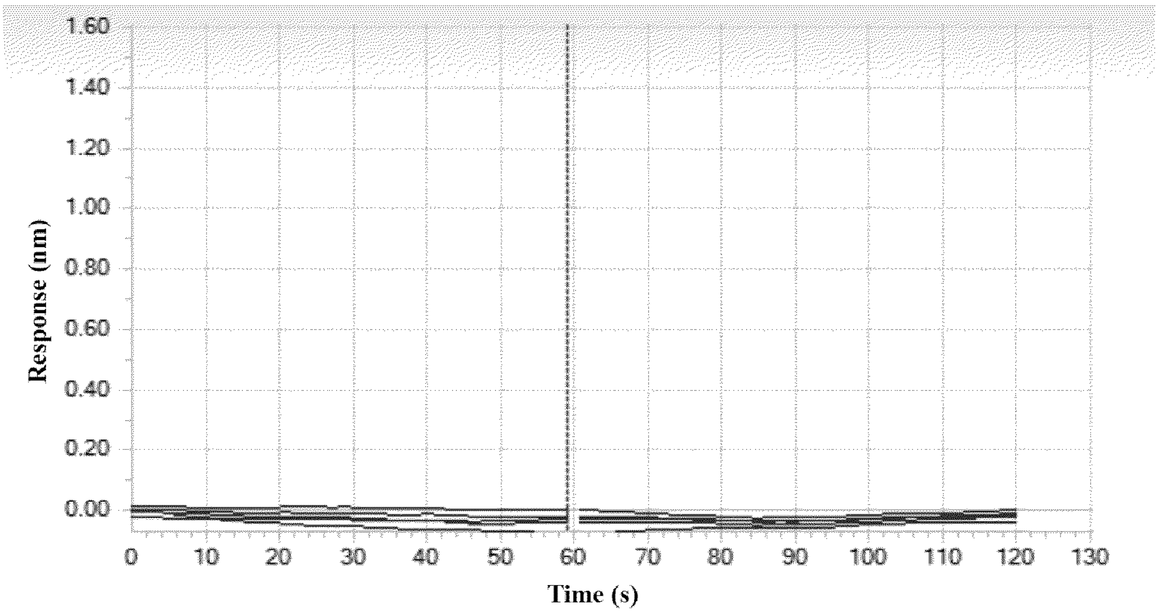
1.60
1.40
1.20
1.00
0.80
0.60
0.40
0.20
0.00
Response (nm)
0
10
20
30
40
50
60
70
80
90
100
110
120
130
Time (s)

FIG. 7

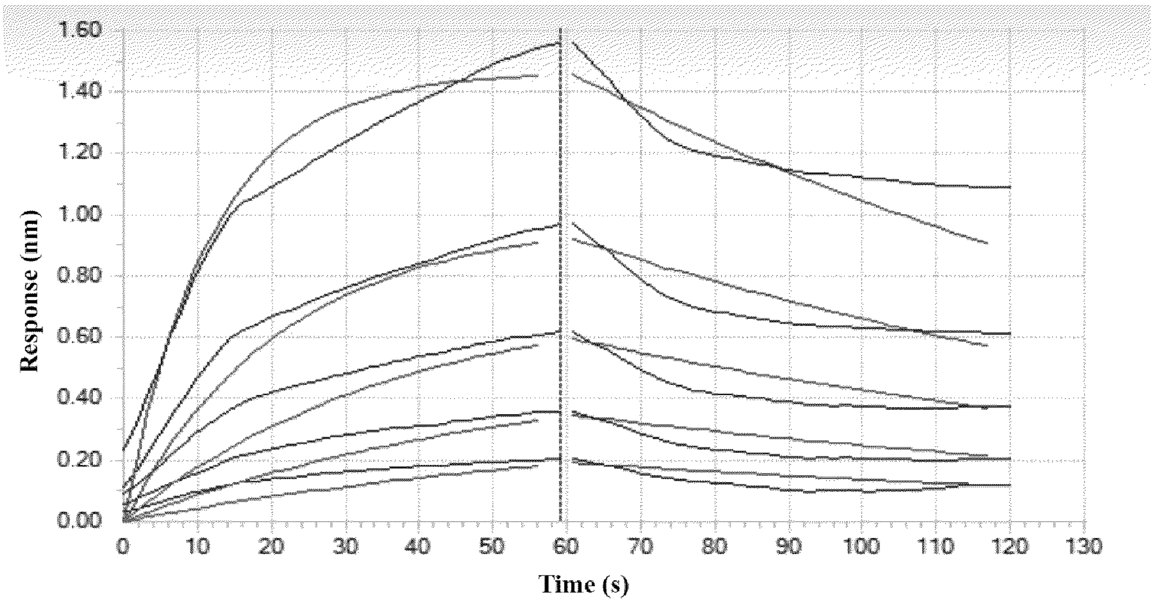
1.60
1.40
1.20
1.00
0.80
0.60
0.40
0.20
0.00
Response (nm)
0
10
20
30
40
50
60
70
80
90
100
110
120
130
Time (s)

FIG. 8

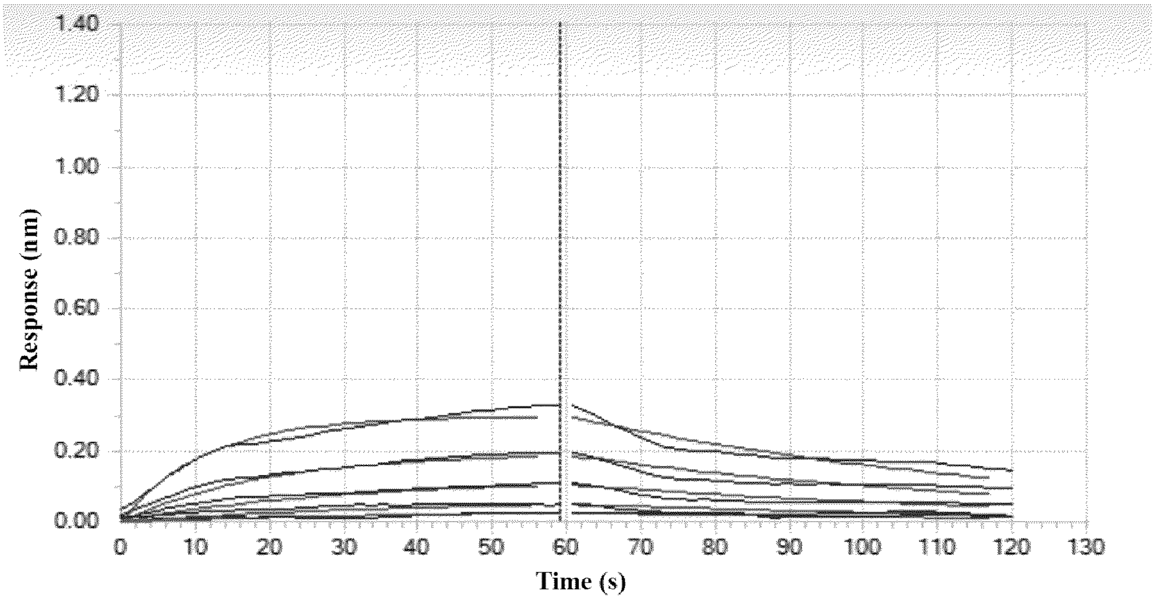
1.40
1.20
1.00
0.80
0.60
0.40
0.20
0.00
Response (nm)
0
10
20
30
40
50
60
70
80
90
100
110
120
130
Time (s)

FIG. 9

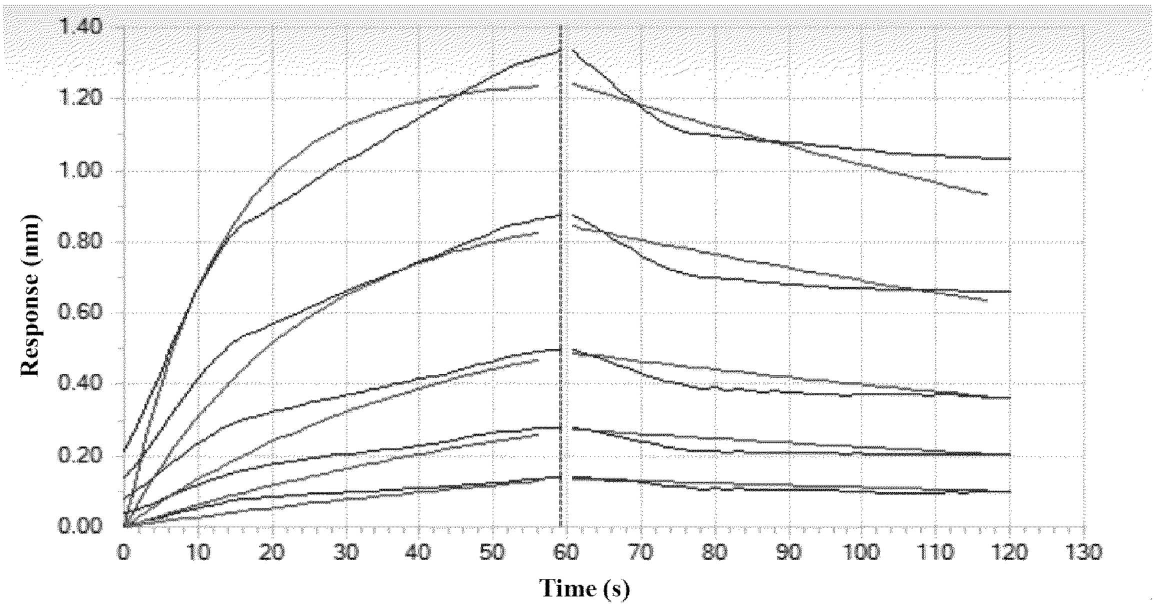
1.40
1.20
1.00
0.80
0.60
0.40
0.20
0.00
Response (nm)
0
10
20
30
40
50
60
70
80
90
100
110
120
130
Time (s)

FIG. 10

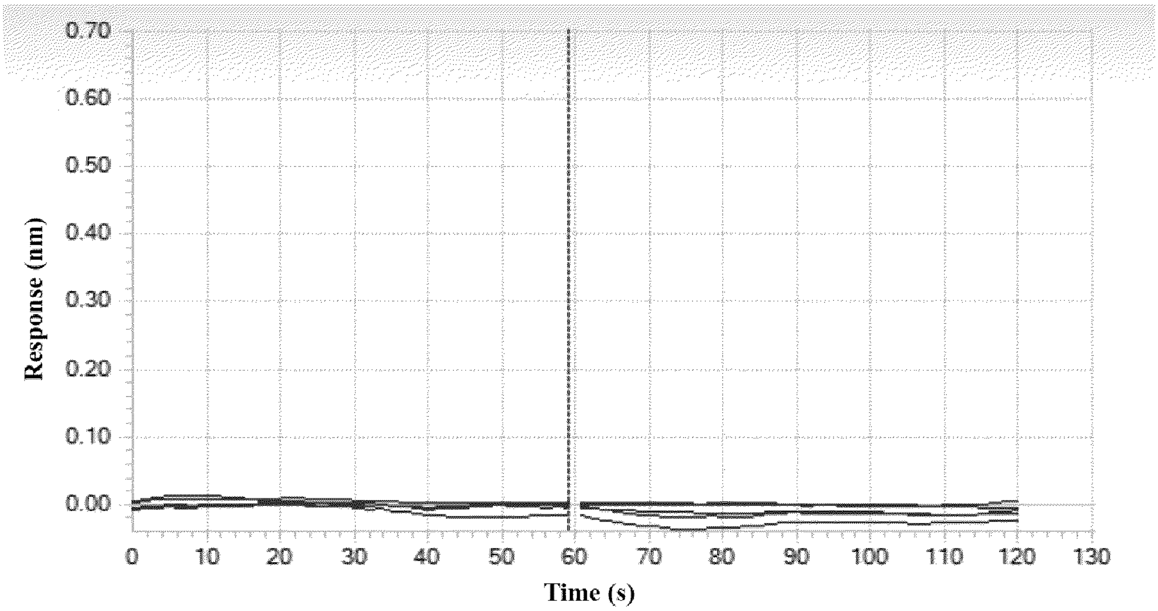
0.70
0.60
0.50
0.40
0.30
0.20
0.10
0.00
Response (nm)
0
10
20
30
40
50
60
70
80
90
100
110
120
130
Time (s)

FIG. 11

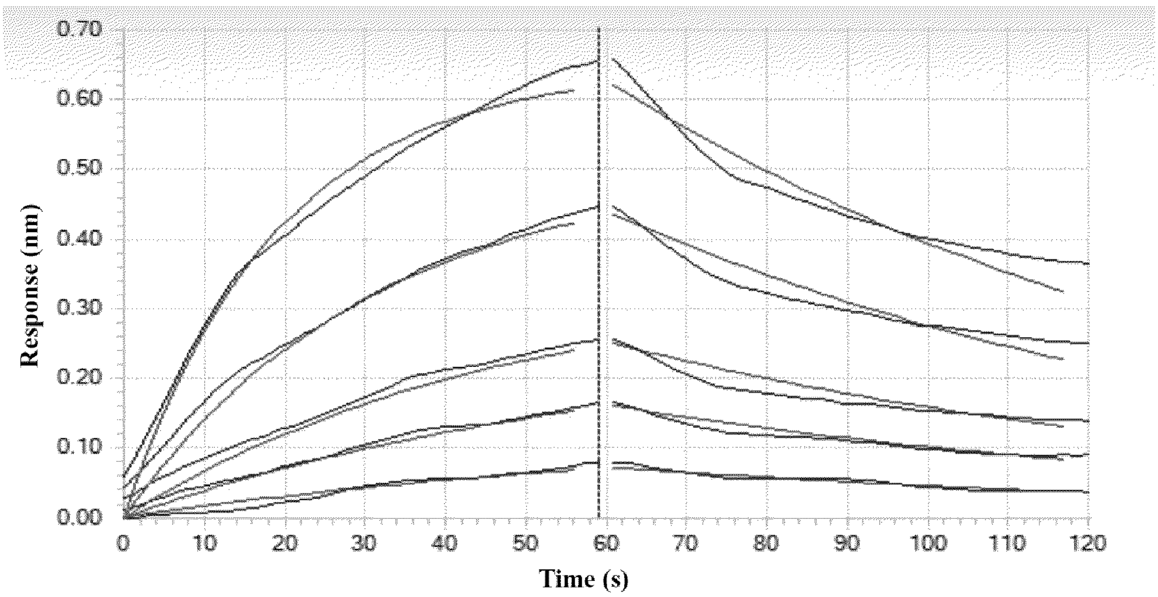
0.70
0.60
0.50
0.40
0.30
0.20
0.10
0.00
Response (nm)
0
10
20
30
40
50
60
70
80
90
100
110
120
Time (s)

FIG. 12

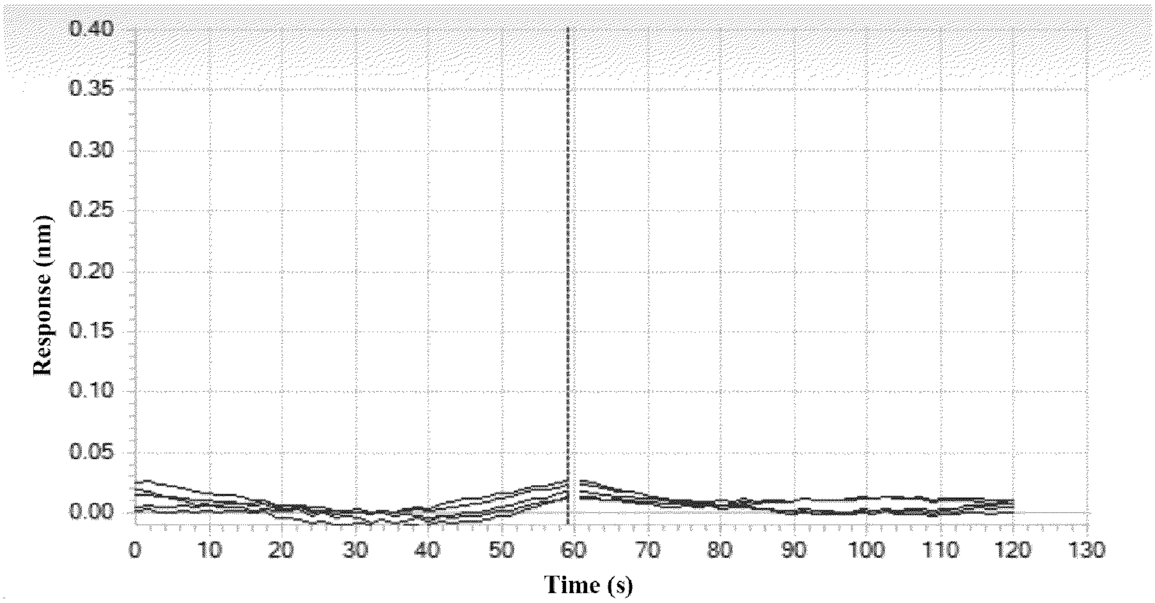
0.40
0.35
0.30
0.25
0.20
0.15
0.10
0.05
0.00
Response (nm)
0
10
20
30
40
50
60
70
80
90
100
110
120
130
Time (s)

FIG. 13

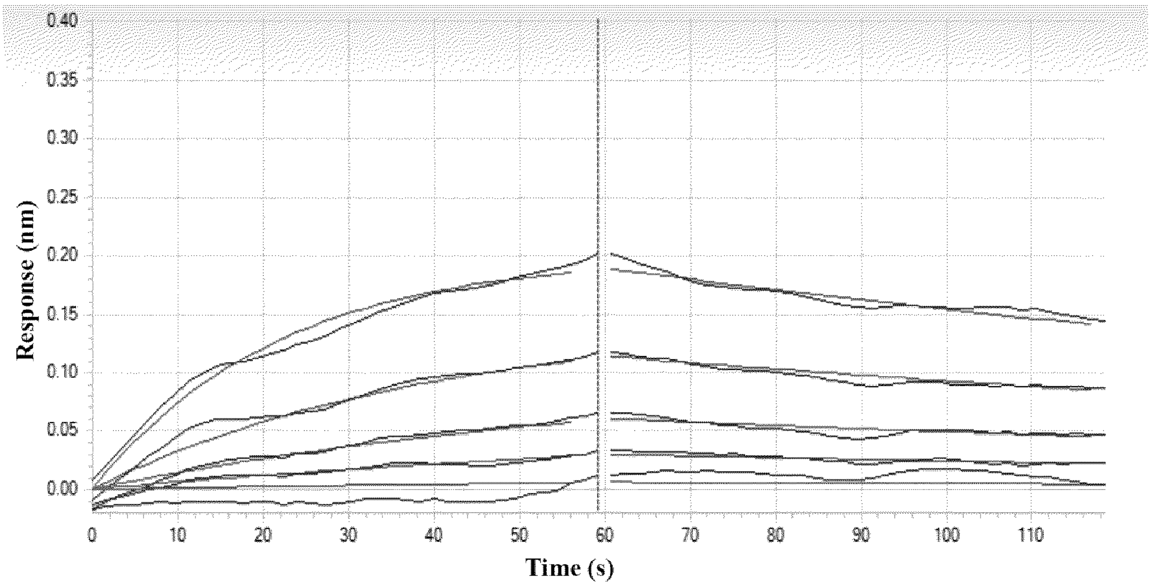
0.40
0.35
0.30
0.25
0.20
0.15
0.10
0.05
0.00
Response (nm)
0
10
20
30
40
50
60
70
80
90
100
110
Time (s)

FIG. 14

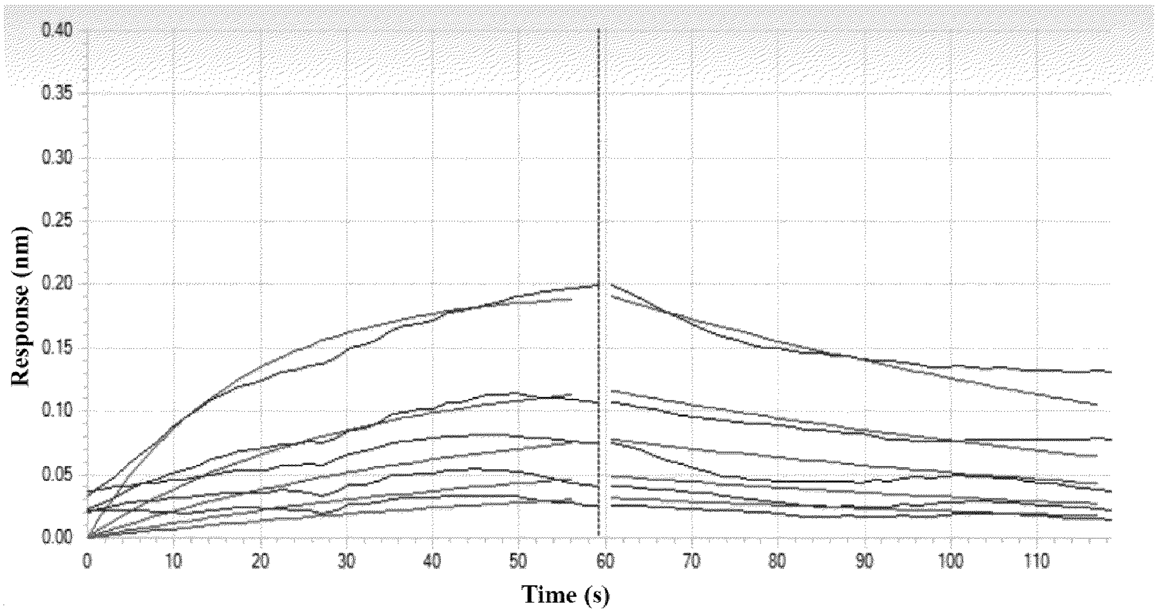
Response (nm)
0.40
0.35
0.30
0.25
0.20
0.15
0.10
0.05
0.00
0
10
20
30
40
50
60
70
80
90
100
110
Time (s)

FIG. 15

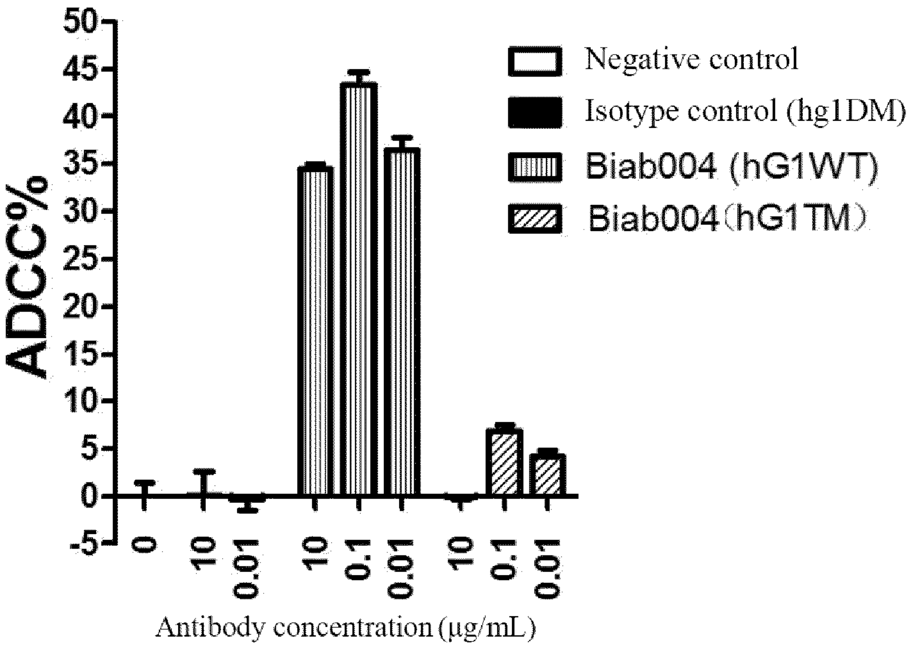
ADCC%
50
45
40
35
30
25
20
15
10
5
0
-5
Negative control
Isotype control (hg1DM)
Biab004 (hG1WT)
Biab004(hG1TM)
0
10
0.01
10
0.1
0.01
10
0.1
0.01
Antibody concentration (μg/mL)

FIG. 16

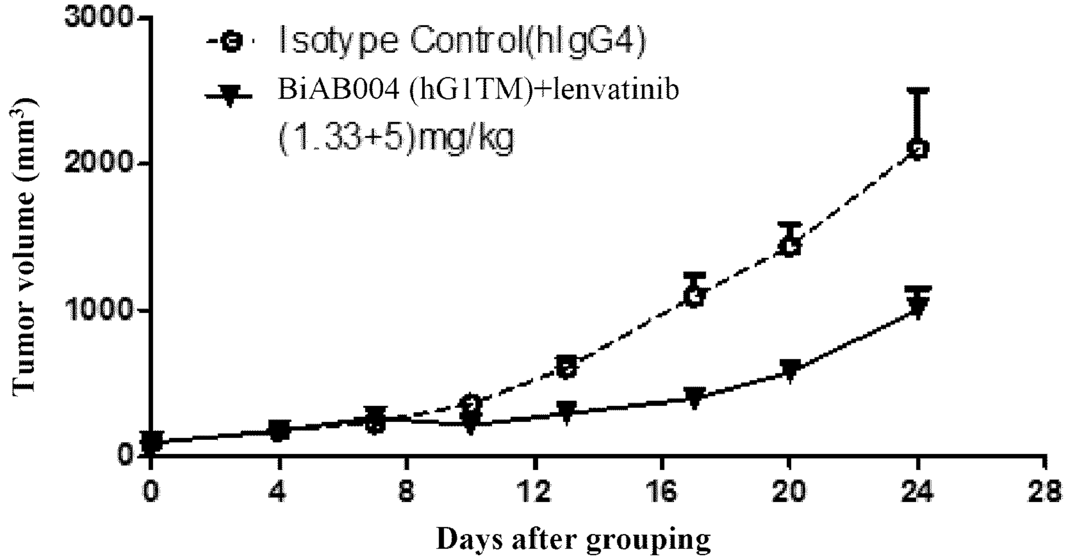
Isotype Control(hIgG4)
BiAB004 (hG1TM)+lenvatinib
(1.33+5)mg/kg
Tumor volume (mm³)
3000
2000
1000
0
0
4
8
12
16
20
24
28
Days after grouping

FIG. 17

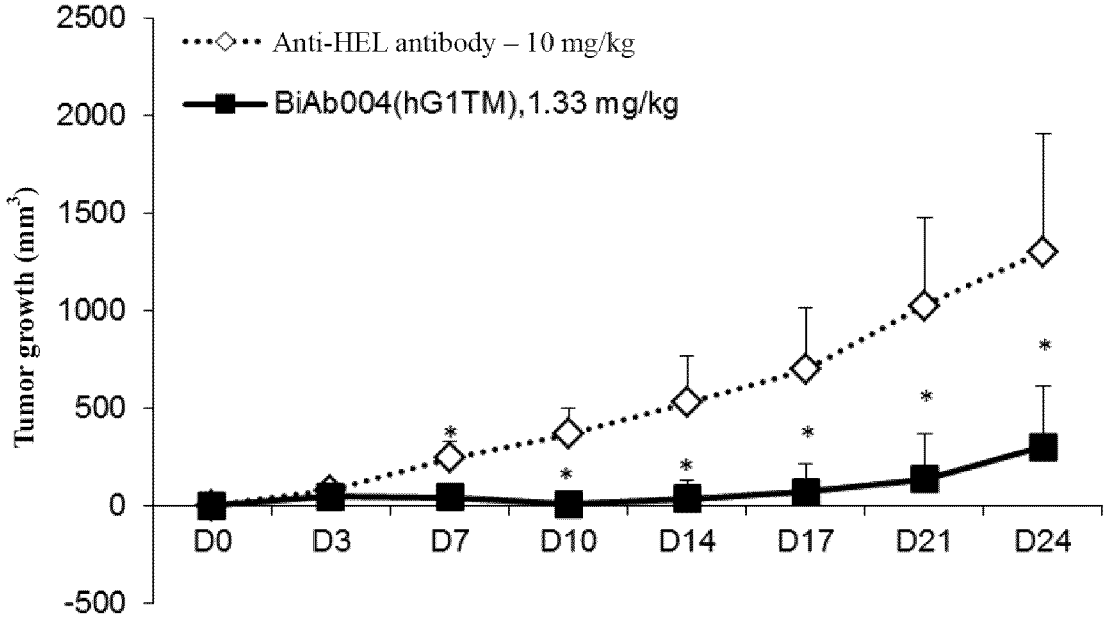
Anti-HEL antibody – 10 mg/kg
BiAb004(hG1TM),1.33 mg/kg
Tumor growth (mm³)
2500
2000
1500
1000
500
0
-500
D0
D3
D7
D10
D14
D17
D21
D24

FIG. 18

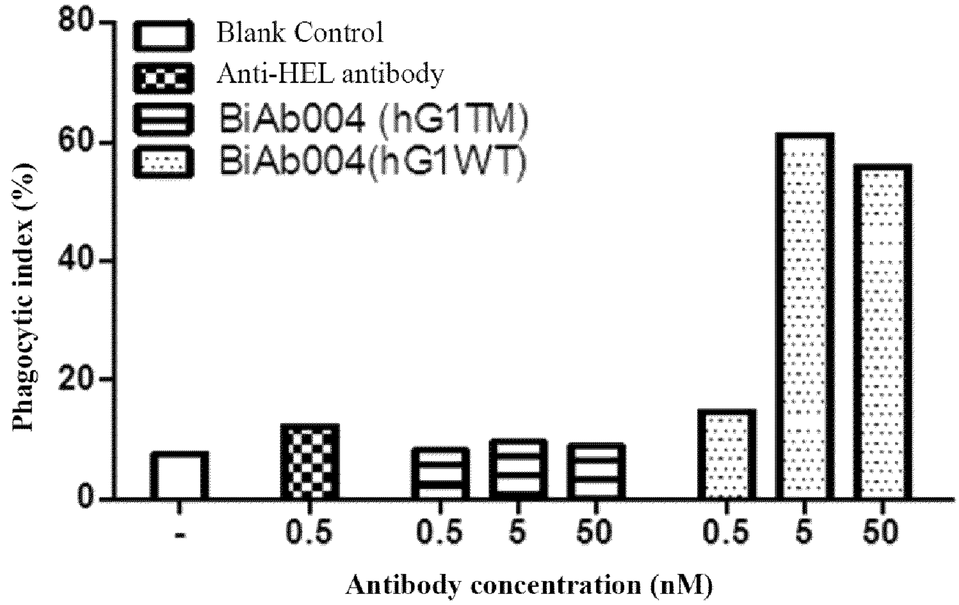
Phagocytic index (%)
80
60
40
20
0
Blank Control
Anti-HEL antibody
BiAb004 (hG1TM)
BiAb004(hG1WT)
-
0.5
0.5
5
50
0.5
5
50
Antibody concentration (nM)

FIG. 19

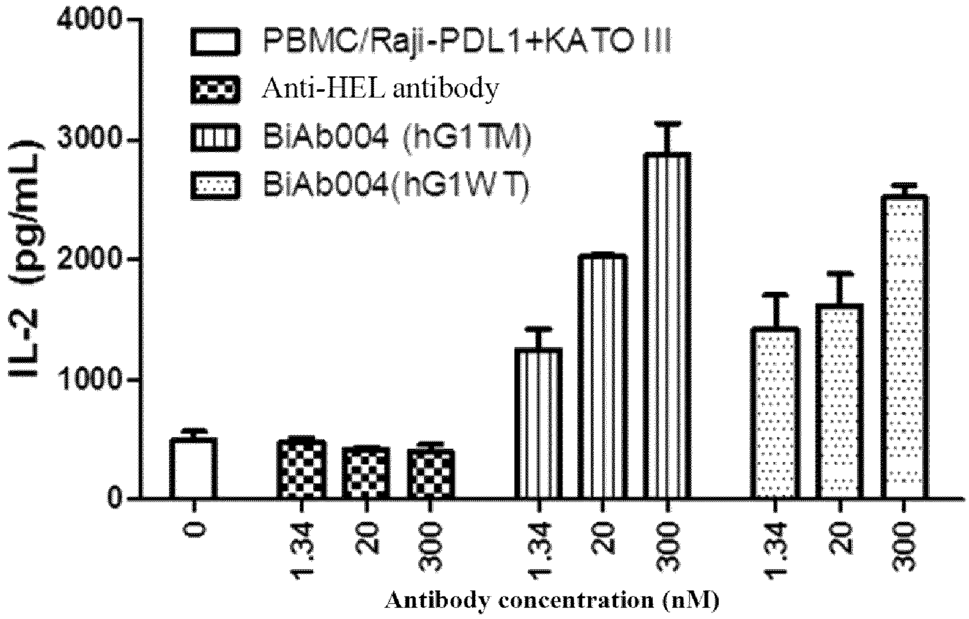
IL-2 (pg/mL)
4000
3000
2000
1000
0
PBMC/Raji-PDL1+KATO III
Anti-HEL antibody
BiAb004 (hG1TM)
BiAb004(hG1WT)
0
1.34
20
300
1.34
20
300
1.34
20
300
Antibody concentration (nM)

FIG. 20

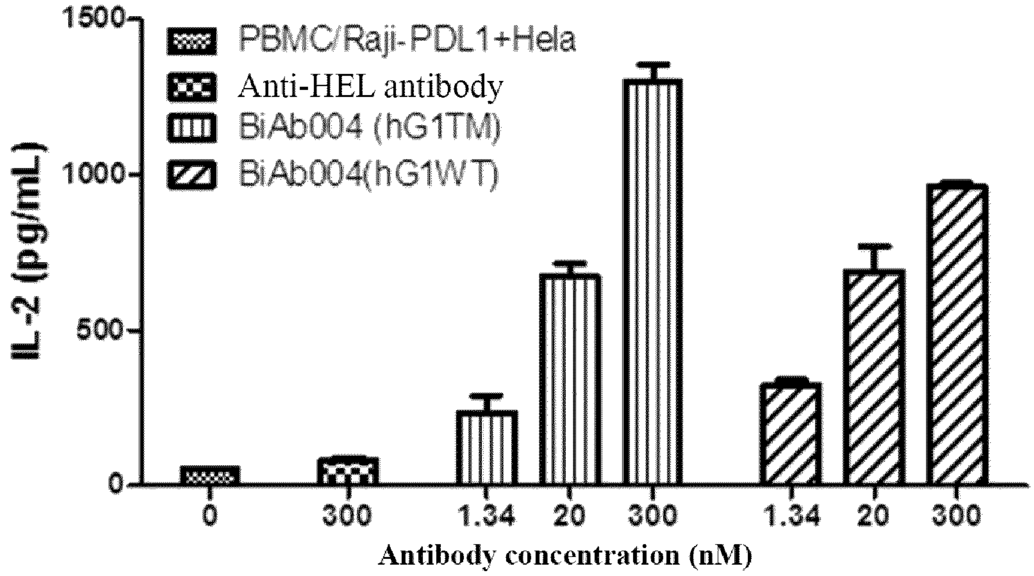
PBMC/Raji-PDL1+Hela
Anti-HEL antibody
BiAb004 (hG1TM)
BiAb004(hG1WT)
IL-2 (pg/mL)
1500
1000
500
0
0
300
1.34
20
300
1.34
20
300
Antibody concentration (nM)

FIG. 21

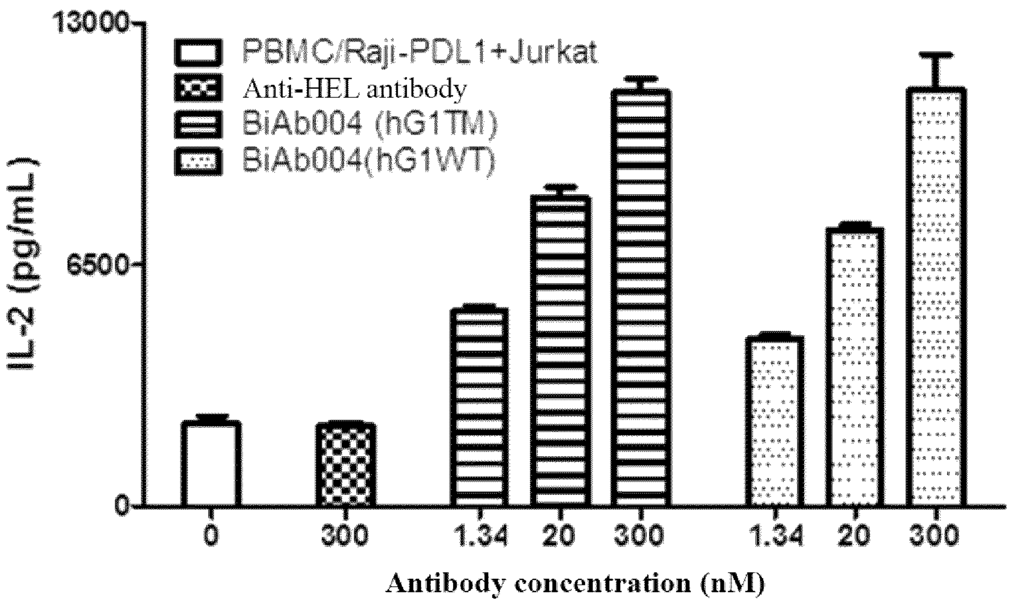
PBMC/Raji-PDL1+Jurkat
Anti-HEL antibody
BiAb004 (hG1TM)
BiAb004(hG1WT)
IL-2 (pg/mL)
13000
6500
0
0
300
1.34
20
300
1.34
20
300
Antibody concentration (nM)

FIG. 22

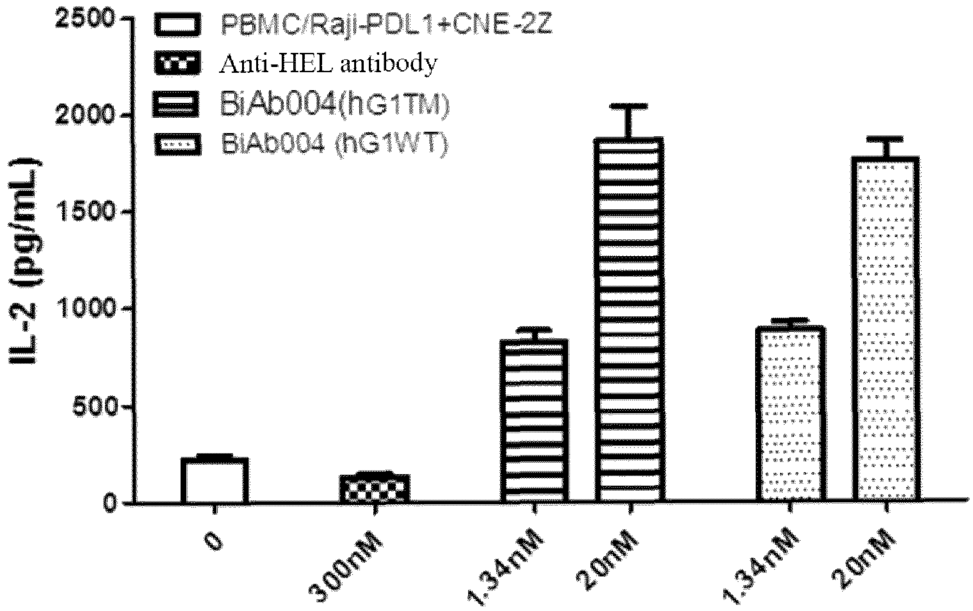
2500
2000
1500
1000
500
0
IL-2 (pg/mL)
PBMC/Raji-PDL1+CNE-2Z
Anti-HEL antibody
BiAb004(hG1TM)
BiAb004 (hG1WT)
0
300nM
1.34nM
20nM
1.34nM
20nM

FIG. 23

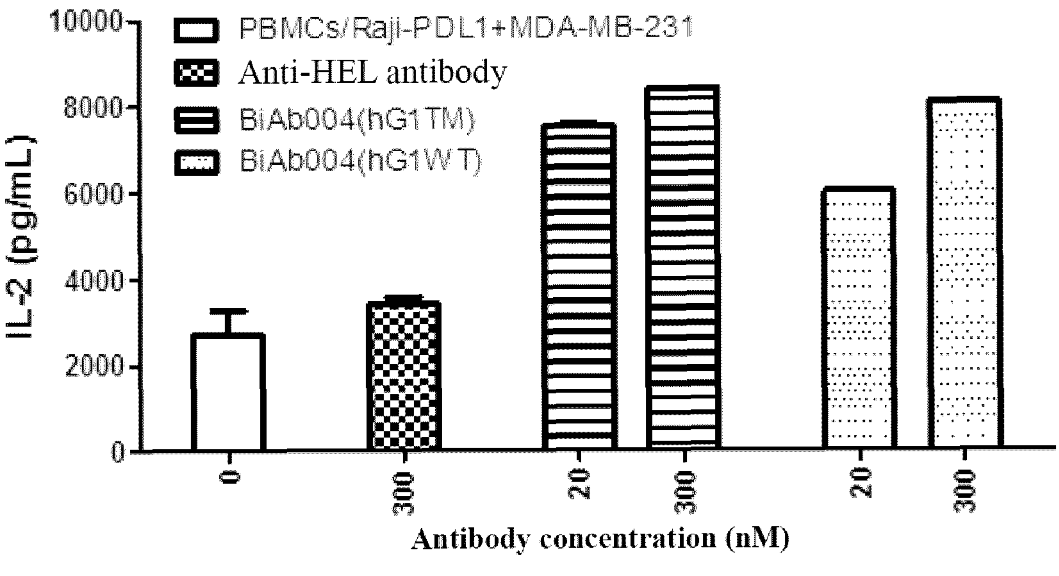
10000
8000
6000
4000
2000
0
IL-2 (pg/mL)
PBMCs/Raji-PDL1+MDA-MB-231
Anti-HEL antibody
BiAb004(hG1TM)
BiAb004(hG1WT)
0
300
20
300
20
300
Antibody concentration (nM)

FIG. 24

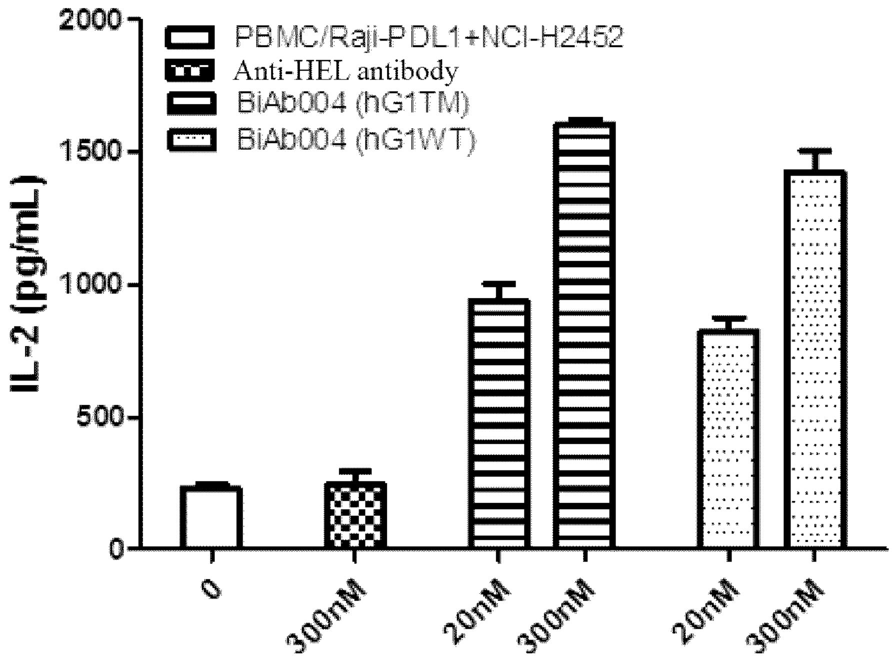

PBMC/Raji-PDL1+NCI-H2452
Anti-HEL antibody
BiAb004 (hG1TM)
BiAb004 (hG1WT)
IL-2 (pg/mL)
2000
1500
1000
500
0
0
300nM
20nM
300nM
20nM
300nM


FIG. 25

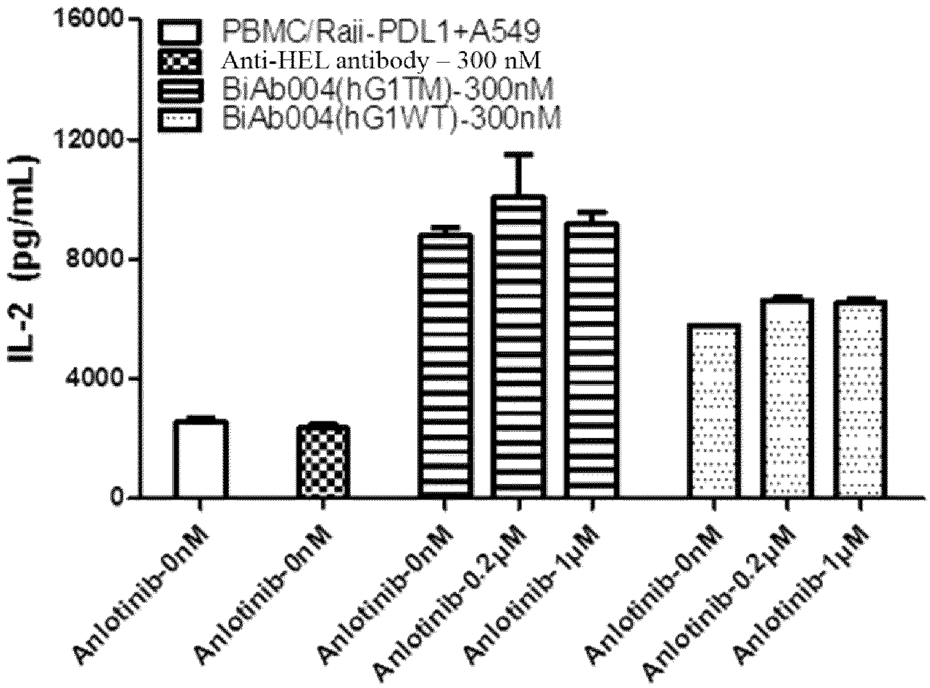

PBMC/Raji-PDL1+A549
Anti-HEL antibody – 300 nM
BiAb004(hG1TM)-300nM
BiAb004(hG1WT)-300nM
IL-2 (pg/mL)
16000
12000
8000
4000
0
Anlotinib-0nM
Anlotinib-0nM
Anlotinib-0nM
Anlotinib-0.2μM
Anlotinib-1μM
Anlotinib-0nM
Anlotinib-0.2μM
Anlotinib-1μM


FIG. 26

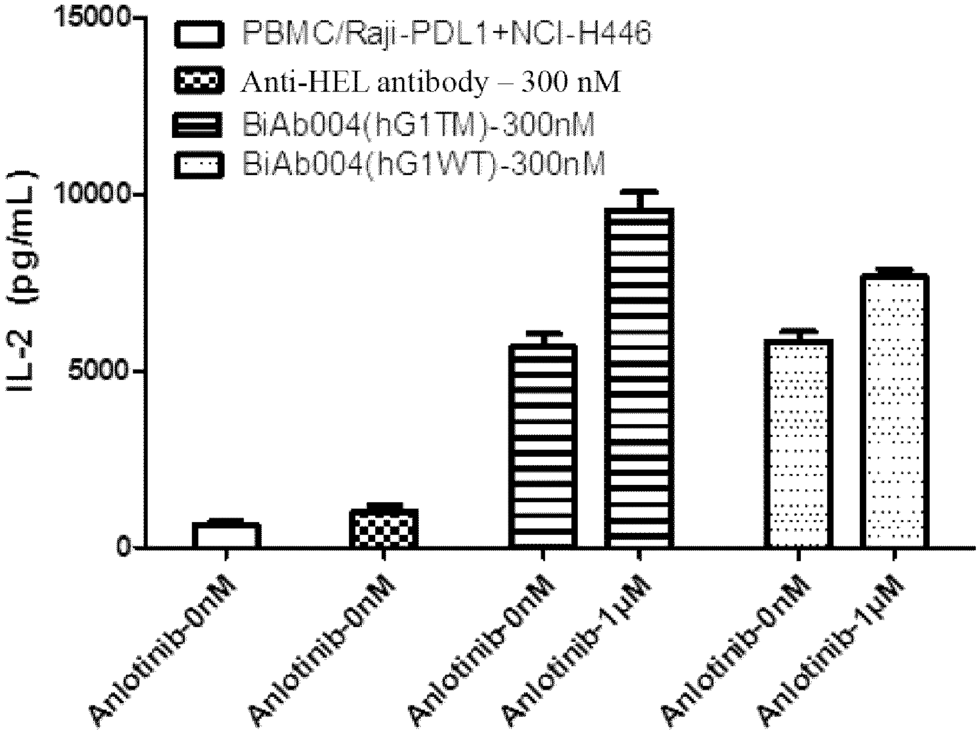
15000
10000
5000
0
IL-2 (pg/mL)
PBMC/Raji-PDL1+NCI-H446
Anti-HEL antibody – 300 nM
BiAb004(hG1TM)-300nM
BiAb004(hG1WT)-300nM
Anlotinib-0nM
Anlotinib-0nM
Anlotinib-0nM
Anlotinib-1μM
Anlotinib-0nM
Anlotinib-1μM

**FIG. 27**

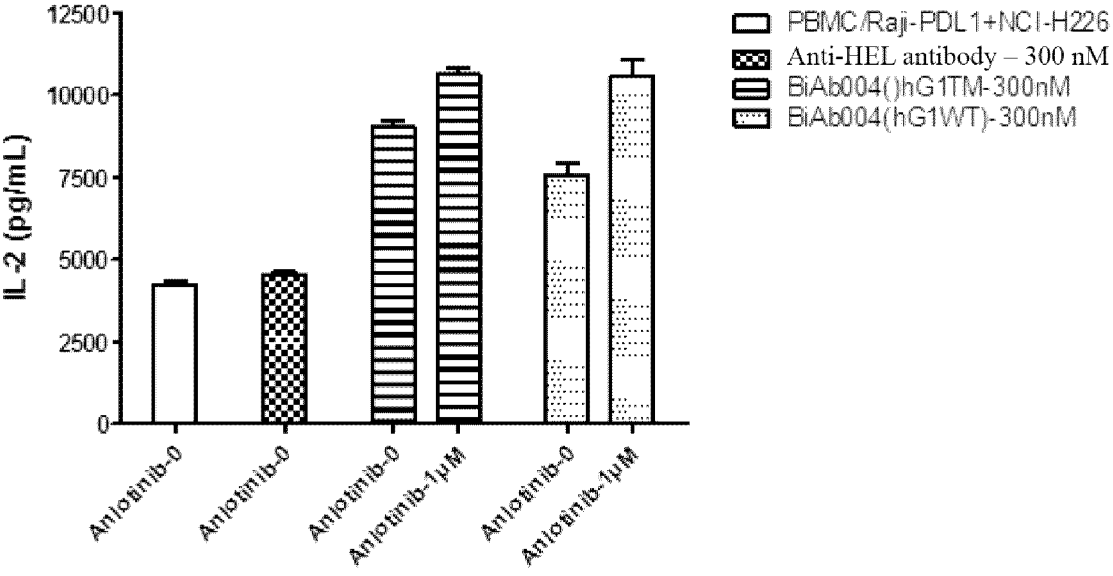
12500
10000
7500
5000
2500
0
IL-2 (pg/mL)
PBMC/Raji-PDL1+NCI-H226
Anti-HEL antibody – 300 nM
BiAb004()hG1TM-300nM
BiAb004(hG1WT)-300nM
Anlotinib-0
Anlotinib-0
Anlotinib-0
Anlotinib-1μM
Anlotinib-0
Anlotinib-1μM

**FIG. 28**

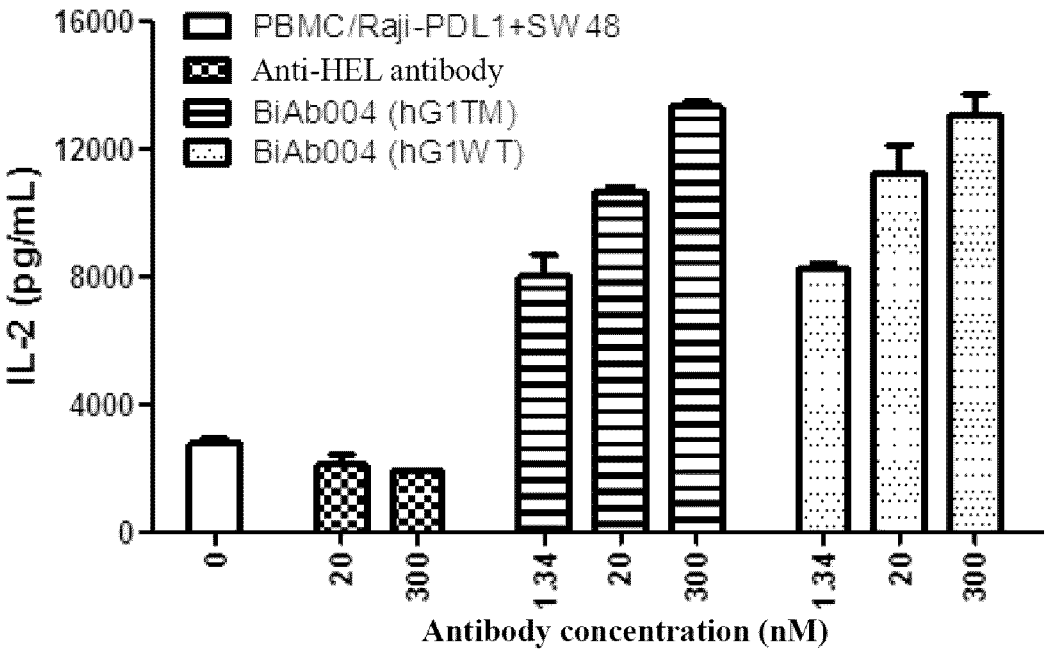

16000
12000
8000
4000
0
IL-2 (pg/mL)
PBMC/Raji-PDL1+SW48
Anti-HEL antibody
BiAb004 (hG1TM)
BiAb004 (hG1WT)
0
20
300
1.34
20
300
1.34
20
300
Antibody concentration (nM)


FIG. 29

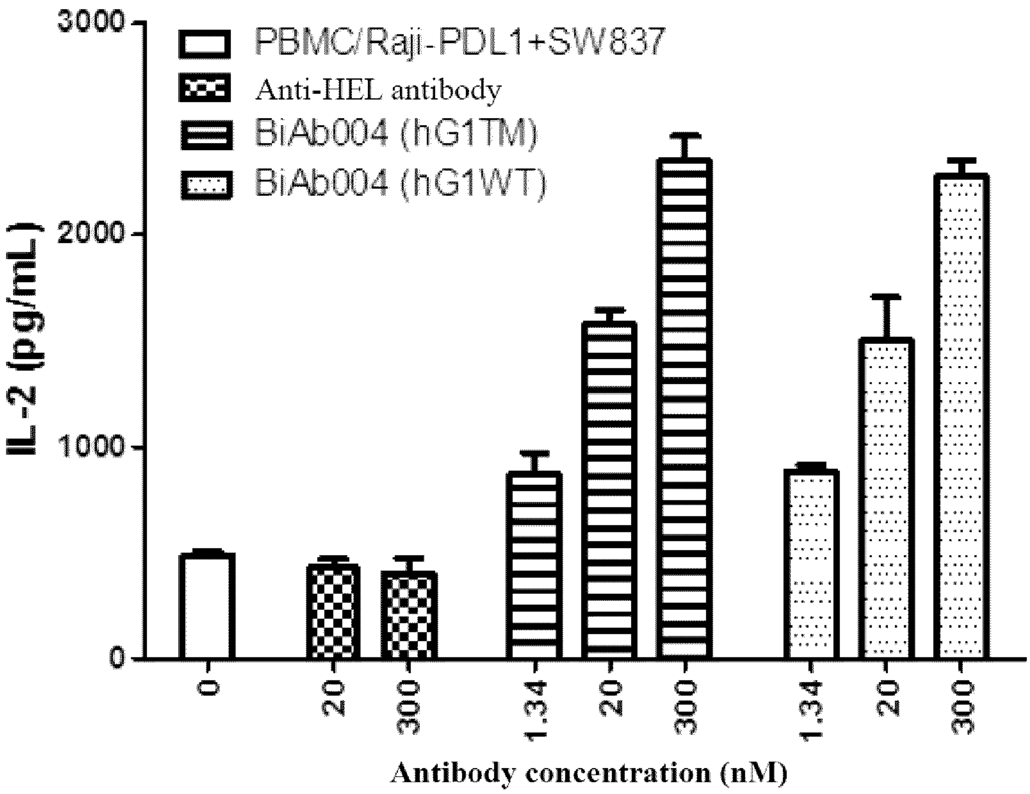

3000
2000
1000
0
IL-2 (pg/mL)
PBMC/Raji-PDL1+SW837
Anti-HEL antibody
BiAb004 (hG1TM)
BiAb004 (hG1WT)
0
20
300
1.34
20
300
1.34
20
300
Antibody concentration (nM)


FIG. 30

## INTERNATIONAL SEARCH REPORT

International application No.
**PCT/CN2020/106309**

**A. CLASSIFICATION OF SUBJECT MATTER**

C07K 16/46(2006.01)i; C07K 16/28(2006.01)i; C12N 15/13(2006.01)i; C12N 15/62(2006.01)i; C12N 15/63(2006.01)i; A61K 39/395(2006.01)i; G01N 33/577(2006.01)i; G01N 33/68(2006.01)i; A61P 7/06(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

C07K; C12N; A61K; G01N; A61P

Documentation searched other than minimum documentation to the extent that such documents are included in the fields searched

Electronic data base consulted during the international search (name of data base and, where practicable, search terms used)

CNMED; CNABS; CPEA; TWMED; DWPI; SIPOABS; EPOQUE; CNKI; ISI; ELESEVER; NCBI; PUBMED; GOOGLE; GenBank; EMBL; STN; 万方; 中国专利生物序列检索系统数据库: 细胞毒性T淋巴细胞先关抗原-4, 程序性死亡受体-1, CTLA4, PD-1, 双特异性抗体, Fc, 变体, 效应子, 铰链区, programmed cell death protein 1, cytotoxic T lymphocyte sociated antigen 4, bispecific antibody, mutant, effector, hinge region

**C. DOCUMENTS CONSIDERED TO BE RELEVANT**

| Category* | Citation of document, with indication, where appropriate, of the relevant passages | Relevant to claim No. |
|---|---|---|
| Y | CN 106967172 A (AKESO BIOPHARMA INC.; ZHONGSHAN AKESOBIO INNOVATION MEDICAMENT RESEARCH INSTITUTE CO., LTD.) 21 July 2017 (2017-07-21) see description, paragraphs 0013-0213, embodiments, claims | 1-29 |
| Y | WO 2018107058 A1 (ALECTOR LLC) 14 June 2018 (2018-06-14) see description paragraphs 0014, 0155-0176, claims 45-54 | 1-29 |
| Y | WO 2018227018 A1 (SILVERBACK THERAPEUTICS INC) 13 December 2018 (2018-12-13) see description paragraphs 0010, 0137-0151, claims 45-50 | 1-29 |
| Y | CN 102711810 A (JANSSEN BIOTECHNOLOGY, INC.) 03 October 2012 (2012-10-03) see description, paragraphs 0006, 0072, claims | 1-29 |
| Y | CN 103351434 A (XENCOR, INC.) 16 October 2013 (2013-10-16) see description paragraphs 0021-0156, 0223, 0274-0275, claims | 1-29 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 September 2020** | **14 October 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/106309**

**C. DOCUMENTS CONSIDERED TO BE RELEVANT**

| Category* | Citation of document, with indication, where appropriate, of the relevant passages | Relevant to claim No. |
|---|---|---|
| A | CN 109762068 A (Y-CLONE (SUZHOU) MEDICAL SCIENCES CO., LTD.) 17 May 2019 (2019-05-17)<br>see entire document | 1-29 |

Form PCT/ISA/210 (second sheet) (January 2015)

**INTERNATIONAL SEARCH REPORT**

International application No.
**PCT/CN2020/106309**

**Box No. I** **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2020/106309**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **30-33**
because they relate to subject matter not required to be searched by this Authority, namely:

[1] Disease treatment methods (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/106309**

| Patent document cited in search report | Publication date (day/month/year) | Patent family member(s) | Publication date (day/month/year) |
|---|---|---|---|
| CN 106967172 A | 21 July 2017 | EP 3511346 A4 | 29 April 2020 |
| | | WO 2018036473 A1 | 01 March 2018 |
| | | AU 2017317124 A1 | 11 April 2019 |
| | | EA 201990571 A1 | 30 August 2019 |
| | | JP 2019533475 A | 21 November 2019 |
| | | EP 3511346 A1 | 17 July 2019 |
| | | CN 106967172 B | 08 January 2019 |
| | | CA 3034850 A1 | 01 March 2018 |
| | | SG 11201901583W A | 28 March 2019 |
| | | BR 112019003695 A2 | 04 June 2019 |
| | | US 2019185569 A1 | 20 June 2019 |
| | | MX 2019002254 A | 28 November 2019 |
| | | KR 20190052677 A | 16 May 2019 |
| WO 2018107058 A1 | 14 June 2018 | AU 2017371070 A1 | 13 June 2019 |
| | | US 2019275150 A1 | 12 September 2019 |
| | | CN 110325549 A | 11 October 2019 |
| | | EP 3551661 A1 | 16 October 2019 |
| | | JP 2020500540 A | 16 January 2020 |
| | | CA 3044684 A1 | 14 June 2018 |
| | | SG 10201912879 Y A | 27 February 2020 |
| | | KR 20190091330 A | 05 August 2019 |
| WO 2018227018 A1 | 13 December 2018 | CN 110891605 A | 17 March 2020 |
| | | AU 2018279105 A1 | 19 December 2019 |
| | | CA 3065852 A1 | 13 December 2018 |
| | | EP 3634485 A1 | 15 April 2020 |
| | | US 2020199247 A1 | 25 June 2020 |
| CN 102711810 A | 03 October 2012 | US 8961967 B2 | 24 February 2015 |
| | | KR 101791430 B1 | 30 October 2017 |
| | | SI 2506871 T1 | 30 December 2016 |
| | | SM T201600387 B | 10 January 2017 |
| | | PT 2506871 T | 07 November 2016 |
| | | EP 2506871 B1 | 19 October 2016 |
| | | EA 030792 B1 | 28 September 2018 |
| | | AU 2010324684 A1 | 14 June 2012 |
| | | JP 2015232009 A | 24 December 2015 |
| | | IL 250508 D0 | 30 March 2017 |
| | | MX 2012006243 A | 15 October 2012 |
| | | HR P20161542 T1 | 30 December 2016 |
| | | EA 201890907 A1 | 30 April 2020 |
| | | WO 2011066501 A1 | 03 June 2011 |
| | | JP 2013512258 A | 11 April 2013 |
| | | RS 55460 B1 | 28 April 2017 |
| | | LT 2506871 T | 12 December 2016 |
| | | BR 112012013038 A2 | 25 October 2016 |
| | | US 2015118227 A1 | 30 April 2015 |
| | | CY 1118447 T1 | 28 June 2017 |
| | | JP 2017095479 A | 01 June 2017 |
| | | AU 2010324684 B2 | 03 September 2015 |
| | | CN 102711810 B | 22 April 2015 |
| | | EP 2506871 A1 | 10 October 2012 |

Form PCT/ISA/210 (patent family annex) (January 2015)

**INTERNATIONAL SEARCH REPORT**
**Information on patent family members**

International application No.
**PCT/CN2020/106309**

| Patent document cited in search report | Publication date (day/month/year) | Patent family member(s) | Publication date (day/month/year) |
|---|---|---|---|
| | | EP 3141260 A1 | 15 March 2017 |
| | | KR 20120116947 A | 23 October 2012 |
| | | SI EP2506871 T1 | 30 December 2016 |
| | | US 2011212087 A1 | 01 September 2011 |
| | | CA 2782218 A1 | 03 June 2011 |
| | | JP 5784624 B2 | 24 September 2015 |
| | | PL 2506871 T3 | 31 March 2017 |
| | | EA 201290413 A1 | 30 January 2013 |
| | | CA 2782218 C | 31 July 2018 |
| | | US 2017204193 A1 | 20 July 2017 |
| | | ES 2609043 T3 | 18 April 2017 |
| | | EP 2506871 A4 | 29 May 2013 |
| | | ES 2751549 T3 | 01 April 2020 |
| | | HU E032872 T2 | 28 November 2017 |
| | | ME 02568 B | 20 February 2017 |
| | | IL 219864 D0 | 31 July 2012 |
| | | EP 3141260 B1 | 14 August 2019 |
| | | DK 2506871 T3 | 02 January 2017 |
| | | US 9637549 B2 | 02 May 2017 |
| CN 103351434 A | 16 October 2013 | KR 20070029190 A | 13 March 2007 |
| | | IL 179048 D0 | 08 March 2007 |
| | | JP 5301611 B2 | 25 September 2013 |
| | | AU 2005272993 A1 | 23 February 2006 |
| | | WO 2006019447 A1 | 23 February 2006 |
| | | JP 2008505174 A | 21 February 2008 |
| | | EP 1919950 A1 | 14 May 2008 |
| | | SI EP2471813 T1 | 31 March 2015 |
| | | CN 101014619 A | 08 August 2007 |
| | | PL 2471813 T3 | 30 September 2015 |
| | | ES 2530340 T3 | 02 March 2015 |
| | | EP 3342782 A1 | 04 July 2018 |
| | | CN 101014619 B | 03 November 2010 |
| | | EP 2471813 B1 | 31 December 2014 |
| | | DK 2471813 T3 | 02 March 2015 |
| | | KR 100863776 B1 | 16 October 2008 |
| | | EP 2940043 A1 | 04 November 2015 |
| | | CN 103172731 A | 26 June 2013 |
| | | JP 2011188869 A | 29 September 2011 |
| | | SI 2471813 T1 | 31 March 2015 |
| | | CA 2565961 A1 | 23 February 2006 |
| | | AU 2005272993 B2 | 11 February 2010 |
| | | CN 103351434 B | 30 September 2015 |
| | | CN 101987870 B | 03 July 2013 |
| | | BR PI0510674 A | 26 December 2007 |
| | | EP 2471813 A1 | 04 July 2012 |
| | | CN 101987870 A | 23 March 2011 |
| CN 109762068 A | 17 May 2019 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008112407 A
- US 7612208 B
- US 7253286 B
- US 4816567 A
- CN 106967172 A
- WO 2017148424 A1


### Non-patent literature cited in the description


- **JULIE R et al.** *N Engl J Med.,* 2012, vol. 366, 2455-2465
- **HAMANISHI et al.** *Proc. Natl. Acad. Sci. USA,* 2007, vol. 104, 3360-5
- **HOMET M. B. ; PARISI G. et al.** *Semin Oncol.,* 2015, vol. 42 (3), 466-473
- **HELD SA ; HEINE A et al.** *Curr Cancer Drug Targets.,* 2013, vol. 13 (7), 768-74
- **GROSSO JF. ; JURE-KUNKEL MN.** *Cancer Immun.,* 2013, vol. 13, 5
- **CHAVEZ, A.R. et al.** *Ann. N. Y. Acad. Sci.,* 2009, vol. 1182, 14-27
- **DAFNE MULLER ; KONTERMANN R E.** *BioDrugs,* 2010, vol. 24 (2), 89-98
- **COLOMA MJ ; MORRISON SL.** *Nat Biotechnol.,* 1997, vol. 15, 159-163
- **MILLER BR ; DEMAREST SJ et al.** *Protein Eng Des Sel,* 2010, vol. 23, 549-57
- **FITZGERALD J ; LUGOVSKOY A.** *MAbs,* 2011, vol. 3, 299-309
- **HOGARTH PM ; PIETERSZ GA.** *NATURE REVIEWS DRUG DISCOVERY,* 2012, vol. 11 (4), 311-331
- **HE JIE et al.** Clinical Oncology. People's Medical Publishing House, vol. 2016, 230-237
- **HAN B et al.** *Br J Cancer,* 2018, vol. 118 (5), 654-661
- **HAN B et al.** *JAMA Oncol.,* November 2018, vol. 4 (11), 1569-1575
- **BARETTI M et al.** *Pharmacol Ther.,* 2018, vol. 189, 45-62
- **KABAT et al.** Sequences of Proteins of Immunological Interest. NIH Publication, 1991, vol. 1-3, 91-3242
- Kabat Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883
- **KOHLER et al.** *Nature,* 1975, vol. 256, 495
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **CLARK.** *Immunol. Today,* 2000, vol. 21, 397-402
- *Epitope Mapping Protocols in Methods in Molecular Biology,* 1996, vol. 66
- Remington's Pharmaceutical Sciences. Mack Publishing Company, 1995
- **J. SAMBROOK ; HUANG PEITANG et al.** Molecular Cloning: A Laboratory Manual. Science Press
- **ACIERNO et al.** Affinity maturation increases the stability and plasticity of the Fv domain of anti-protein antibodies. *J Mol Biol.,* 2007, vol. 374 (1), 130-146
- **EFREMOVA M et al.** *Nat Commun.,* 2018, vol. 9 (1), 32
- **ACIERNO et al.** *J Mol Biol.,* 2007, vol. 374 (1), 130-46
- **ZHANG T et al.** *Cancer Immunol Immunother.,* 2018, vol. 67 (7), 1079-1090
- **DAHAN R et al.** *Cancer cell,* 2015, vol. 28 (3), 285-95
- **BRANCH P et al.** *Cancer Res,* 1995, vol. 55 (11), 2304-2309
- **GUO J et al.** *Cancer Res.,* 2011, vol. 71 (8), 2978-2987